# DEMANDE DE BREVET EUROPEEN

(11) **EP 1 952 800 A2**
(43) Date de publication de la demande: **06.08.2008**
(21) Numéro de dépôt: 07123120.3
(22) Date de dépôt: 13.12.2007
(51) Int. Cl.: A61K 8/891, A61K 8/892, A61K 8/893, A61K 8/895, A61Q 5/06

(54) **Composition tinctoriale contenant une silicone réactive et un colorant fluorescent , procédé de coloration utilisant la composition**

(30) Priorité: 20.12.2006 FR 0655719
(71) Demandeur: L'Oréal, 75008 Paris (FR)
(72) Inventeur: Brun, Gaëlle, 75015, PARIS (FR); Gourlaouen, Luc, 92600, ASNIERES (FR)
(74) Mandataire: Fevrier, Murielle Françoise E.

(57) **Abrégé**

La présente invention a pour objet un procédé de coloration de fibres kératiniques qui consiste à appliquer sur lesdites fibres une composition tinctoriale comprenant : i) au moins un composé X ; ii) au moins un composé Y, les composés X et Y étant susceptibles de réagir ensemble par réaction d'hydrosilylation éventuellement en présence d'un catalyseur d'hydrosilylation, de condensation ou de réticulation en présence d'un peroxyde, lorsqu'ils sont mis en contact l'un de l'autre ; et iii) au moins un colorant fluorescent

La coloration obtenue après application sur cheveux de la composition, présente une bonne résistance aux diverses agressions. Par ailleurs les cheveux restent parfaitement individualisés et peuvent être coiffés facilement.

## Description

La présente invention a pour objet une composition tinctoriale contenant une silicone réactive et un colorant fluorescent utile pour la coloration des fibres kératiniques.

Depuis longtemps, on cherche à modifier la couleur des cheveux et en particulier à masquer les cheveux blancs. Pour ce faire, plusieurs technologies ont été développées.

Il est connu de teindre les fibres kératiniques, et en particulier des fibres kératiniques humaines, telles les cheveux, avec des compositions de teinture contenant des colorants directs. Les colorants classiques qui sont utilisés sont en particulier des colorants du type nitrés benzèniques, anthraquinoniques, nitropyridiniques, azoïques, xanthéniques, acridiniques, aziniques, triarylméthane ou des colorants naturels. Ces colorants peuvent être non ioniques, anioniques, cationiques ou amphotères.

Ces colorants qui sont des molécules colorées et colorantes ayant une affinité pour les fibres kératiniques sont appliqués pendant le temps nécessaire à l'obtention de la coloration désirée, puis rincés.

Les colorations qui en résultent sont des colorations particulièrement chromatiques qui sont cependant temporaires ou semi-permanentes car la nature des interactions qui lient les colorants directs à la fibre kératinique, et leur désorption de la surface et / ou du coeur de la fibre sont responsables de leur faible puissance tinctoriale et de leur mauvaise tenue aux lavages ou à la transpiration.

Par ailleurs, il est connu de teindre les fibres kératiniques de façon permanente par la coloration d'oxydation. Cette technique de coloration consiste à appliquer sur les fibres kératiniques une composition contenant des précurseurs de colorant tels que des bases d'oxydation et des coupleurs. Ces précurseurs sous l'action d'un agent oxydant vont former dans le cheveu une ou plusieurs espèces colorées.

La variété des molécules mises en jeu au niveau des bases d'oxydation et des coupleurs, permet l'obtention d'une riche palette de couleurs. Les colorations qui en résultent sont permanentes, puissantes, et résistantes aux agents extérieurs, notamment à la lumière, aux intempéries, aux lavages, à la transpiration et aux frottements. Cependant, ce type de coloration entraîne une dégradation de la fibre due à l'utilisation d'un agent oxydant.

Il existe toujours un besoin de développer de nouvelles compositions de teinture directe pour obtenir des nuances variées, en particulier des nuances pastels et qui présentent une bonne ténacité, notamment aux agents extérieurs tels que la lumière, le shampooing, la sueur tout en préservant la qualité des fibres kératiniques. En particulier, il existe un besoin de développer des compositions de coloration permettant d'obtenir des colorations présentant une ténacité proche de la coloration par oxydation sans les inconvénients liés à la présence d'un agent oxydant.

Par ailleurs, la coloration des fibres kératiniques à partir de colorants directs classiques ne permet pas d'éclaircir de façon notable les fibres kératiniques.

L'éclaircissement de la couleur de fibres kératiniques, plus particulièrement foncées vers des nuances plus claires, en modifiant éventuellement la nuance de celles-ci, constitue une demande importante.

Classiquement, pour obtenir une coloration plus claire on met en oeuvre un procédé de décoloration chimique. Ce procédé consiste à traiter les fibres kératiniques par un système oxydant fort, généralement constitué par du peroxyde d'hydrogène associé ou non à des persels, le plus souvent en milieu alcalin.

Comme vu précédemment, le système de décoloration présente l'inconvénient de dégrader des matières kératiniques, en particulier les fibres kératiniques, notamment humaines telles que les cheveux et d'altérer leurs propriétés cosmétiques. Les fibres ont en effet tendance à devenir rêches, plus difficilement démêlables et plus fragiles. Enfin, l'éclaircissement ou la décoloration de fibres kératiniques par des agents oxydants est incompatible avec les traitements de modification de la forme desdites fibres particulièrement dans les traitements de défrisage.

Une autre technique d'éclaircissement consiste à appliquer sur les cheveux foncés des colorants directs fluorescents. Cette technique décrite notamment dans les documents FR 2830189 et WO 2004/091473 permet de respecter la qualité de la fibre kératinique lors du traitement mais les colorants fluorescents employés ne présentent pas une résistance aux shampoings satisfaisante.

Tous les systèmes décrits faisant appel aux pigments ou colorant fluorescent ne présentent donc pas une rémanence satisfaisante vis-à-vis des agressions extérieures, sébum, transpiration, intempéries et shampooing successifs.

De façon inattendue avec la présente invention, il est possible de résoudre le problème technique de rémanence sans altérer les qualités cosmétiques des fibres, l'intégrité des fibres, et sans diminuer les propriétés colorantes ou d'éclaircissement provenant des pigments ou des colorants fluorescents.

Ainsi l'invention a pour objet un procédé de coloration de fibres kératiniques qui consiste à appliquer sur lesdites fibres une composition tinctoriale comprenant : i) au moins un composé X ; ii) au moins un composé Y, l'un au moins des composés X ou Y étant un composé siliconé, lesdits composés X et Y étant susceptibles de réagir ensemble par réaction d'hydrosilylation éventuellement en présence d'un catalyseur d'hydrosylilation ; de condensation, ou de réticulation en présence d'un peroxyde, lorsqu'ils sont mis en contact l'un de l'autre ; et iii) au moins un colorant fluorescent.

L'invention a également pour objet la composition telle que définie précédemment.

Un autre objet de l'invention est l'utilisation du mélange de X, Y éventuellement en présence d'un catalyseur d'hydrosylilation ou d'un peroxyde ; et au moins un colorant fluorescent ; pour la coloration de fibres kératiniques, l'éclaircissement des fibres kératiniques foncées.

L'invention concerne également un Kit ou dispositif de coloration comprenant au moins deux compositions (A) et (B) conditionnées séparément, le kit comprenant i) au moins un composé X, ii) au moins un composé Y, étant entendu que la composition (A) et/ou (B) comprenant au moins un colorant fluorescent ; et iii) éventuellement au moins un catalyseur ou un peroxyde ; le catalyseur lorsqu'il est présent ou le peroxyde ne sont pas présents simultanément dans la même composition ; lesdits composés X et Y étant susceptibles de réagir ensemble par une réaction d'hydrosilylation, ou par une réaction de condensation, ou par une réaction de réticulation en présence d'un peroxyde, lorsqu'ils sont mis en contact les uns avec les autres.

La composition conforme à la présente invention permet d'améliorer la visibilité de la coloration notamment sur cheveux foncés. En particulier, on obtient dans le cas des fibres kératiniques foncées une coloration très visible sans qu'il soit nécessaire d'éclaircir ou de décolorer les fibres kératiniques et par conséquent sans dégradation physique des fibres kératiniques. Cette composition permet en particulier d'obtenir des effets d'éclaircissement optique sur cheveux foncés, notamment des cheveux qui présentent une hauteur de ton inférieure ou égale à 6, plus préférentiellement inférieure ou égale à 4.

Elle permet aussi par le choix du composé fluorescent d'obtenir des colorations avec des effets spéciaux sous l'effet de lumière riche en rayonnement UV comme les éclairages utilisés dans les boites de nuit par exemple notamment la lumière noire.

De plus, cette coloration présente une bonne résistance aux diverses agressions que peuvent subir les cheveux telles que les shampooings, les frottements, la lumière, les intempéries, la sueur et les déformations permanentes. Ces propriétés sont particulièrement remarquables en ce qui concerne la résistance de la coloration vis à vis des shampooings. Les cheveux présentent par ailleurs de bonnes propriétés cosmétiques, ils restent en particulier parfaitement individualisés et peuvent être coiffés facilement.

### I) Composés X et Y

Par composé siliconé, on entend un composé comprenant au moins deux unités organosiloxanes. Selon un mode de réalisation particulier, les composés X et les composés Y sont siliconés. Les composés X et Y peuvent être aminés ou non aminés. Ils peuvent comprendre des groupes polaires pouvant être choisis parmi les groupes suivants -COOH, -COO⁻ , -COO- , -OH , -NH₂ , -NH-,-NR-, -SO₃H, -SO₃⁻, -OCH₂CH₂-, -O-CH₂CH₂CH₂-, -O-CH₂CH(CH₃)-, -NR₃⁺, -SH, -NO₂, I, Cl, Br, -CN, -PO₄³⁻, -CONH-, -CONR-, -CONH₂, -CSNH-, -SO₂- , -SO-, -SO₂NH-, -NHCO- , -NHSO₂- , -NHCOO-, -OCONH-, -NHCSO- et -OCSNH- avec R représentant un groupe alkyle.

Selon un autre mode de réalisation, au moins un des composés X et Y est un polymère dont la chaîne principale est formée majoritairement d'unités organosiloxanes.

Parmi les composés siliconés cités ci-après, certains peuvent présenter à la fois des propriétés filmogènes et adhésives, selon par exemple leur proportion en silicone ou selon qu'on les utilise en mélange avec un additif particulier. Il est par conséquent possible de moduler les propriétés filmogènes ou les propriétés adhésives de tels composés selon l'utilisation envisagée, c'est en particulier le cas pour les silicones élastomères réactives dites "room temperature vulcanization".

Les composés X et Y peuvent réagir ensemble à une température variant entre la température ambiante et 180 °C. Avantageusement, les composés X et Y sont susceptibles de réagir ensemble à température ambiante (20 ± 5 °C) et pression atmosphérique, avantageusement en présence d'un catalyseur, par une réaction d'hydrosilylation ou une réaction de condensation, ou une réaction de réticulation en présence d'un peroxyde.

### 1- Composés X et Y susceptibles de réagir par hydrosilylation

Selon un mode de réalisation, les composés X et Y sont susceptibles de réagir par hydrosilylation, cette réaction pouvant être de manière simplifiée schématisée comme suit : 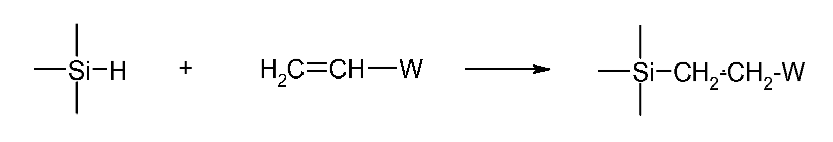 avec W représentant une chaîne carbonée et/ou siliconée contenant un ou plusieurs groupements aliphatiques insaturés.

Dans ce cas, le composé X peut être choisi parmi les composés siliconés comprenant au moins deux groupements aliphatiques insaturés. A titre d'exemple, le composé X peut comprendre une chaîne principale siliconée dont les groupements aliphatiques insaturés sont pendants à la chaîne principale (groupe latéral) ou situés aux extrémités de la chaîne principale du composé (groupe terminal). On appellera, dans la suite de la description, ces composés particuliers des polyorganosiloxanes à groupements aliphatiques insaturés.

Selon un mode de réalisation, le composé X est choisi parmi les polyorganosiloxanes comprenant au moins deux groupements aliphatique insaturés, par exemple deux ou trois groupements vinyliques ou allyliques, liés chacun à un atome de silicium.

Selon un mode de réalisation avantageux, le composé X est choisi parmi les polyorganosiloxanes comprenant des unités siloxanes de formule : 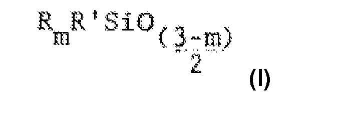 dans laquelle :
- R représente un groupe hydrocarboné monovalent, linéaire ou cyclique, comprenant de 1 à 30 atomes de carbone, de préférence de 1 à 20, et mieux de 1 à 10 atomes de carbone, comme par exemple un radical alkyle à chaîne courte, comprenant par exemple de 1 à 10 atomes de carbone, en particulier un radical méthyle ou encore un groupement phényle, de préférence un radical méthyle,
- m est égal à 1 ou 2 et
- R' représente :
   o un groupement hydrocarboné aliphatique insaturé comprenant de 2 à 10, de préférence de 2 à 5 atomes de carbone comme par exemple un groupe vinyle ou un groupe -R"-CH=CHR"' dans lequel R" est une chaîne hydrocarbonée aliphatique divalente, comprenant de 1 à 8 atomes de carbone, liée à l'atome de silicium et R"' est un atome d'hydrogène ou un radical alkyle comprenant de 1 à 4 atomes de carbone, de préférence un atome d'hydrogène ; on peut citer comme groupement R' les groupements vinyle, allyle et leurs mélanges ; ou
   o un groupement hydrocarboné cyclique insaturé comprenant de 5 à 8 atomes de carbone comme par exemple un groupe cyclohexenyle.

De préférence R' est un groupement hydrocarboné aliphatique insaturé, de préférence un groupe vinyle.

Selon un mode de réalisation particulier, le polyorganosiloxane comprend également des unités de formule 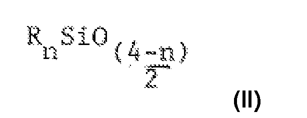 dans laquelle R est un groupe tel que défini plus haut, et n est égal à 1, 2 ou 3.

Selon une variante, le composé X peut être une résine de silicone comprenant au moins deux insaturations éthyléniques, ladite résine étant apte à réagir avec le composé Y par hydrosilylation. On peut citer par exemple les résines de type MQ ou MT portant elle-même des extrémités réactives insaturées -CH=CH₂.

Ces résines sont des polymères d'organosiloxanes réticulés.

La nomenclature des résines de silicone est connue sous le nom de "MDTQ", la résine étant décrite en fonction des différentes unités monomèriques siloxane qu'elle comprend, chacune des lettres "MDTQ" caractérisant un type d'unité.

La lettre M représente l'unité monofonctionelle de formule (CH₃)₃SiO_{1/2}, l'atome de silicium étant relié à un seul atome d'oxygène dans le polymère comprenant cette unité.

La lettre D signifie une unité difonctionnelle (CH₃)₂SiO_{2/2} dans laquelle l'atome de silicium est relié à deux atomes d'oxygène.

La lettre T représente une unité trifonctionnelle de formule (CH₃)₁SiO_{3/2}.

Dans les motifs M, D, T définis précédemment, au moins un des groupes méthyles peut être substitués par un groupe R différent du groupe méthyle tel qu'un radical hydrocarboné (notamment alkyle) ayant de 2 à 10 atomes de carbone ou un groupe phényl ou bien encore un groupe hydroxyle.

Enfin, la lettre Q signifie une unité tétrafonctionnelle SiO_{4/2} dans laquelle l'atome de silicium est lié à quatre atomes d'hydrogène eux mêmes liés au reste du polymère. Comme exemples de telles résine, on peut citer les résines de silicone MT telles que les poly(phenyl-vinylsilsesquioxane) comme celle commercialisées sous la référence SST-3PV1 par la société Gelest.

De préférence, les composés X comprennent de 0,01 à 1 % en poids de groupes aliphatiques insaturés.

Avantageusement, le composé X est choisi parmi les polyorganopolysiloxanes, notamment ceux comprenant les unités siloxanes (I) et éventuellement (II) décrites précédemment.

Le composé Y comprend alors au moins deux groupes Si-H (groupes hydrogénosilanes) libres.

Le composé Y peut être avantageusement choisi parmi les organosiloxanes comprenant au moins une unité alkylhydrogènosiloxane de formule suivante : 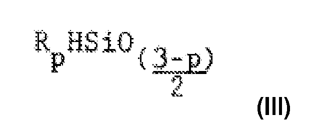 dans laquelle :
R représente un groupe hydrocarboné monovalent, linéaire ou cyclique, comprenant de 1 à 30 atomes de carbone, comme par exemple un radical alkyle ayant de 1 à 30 atomes de carbone, de préférence de 1 à 20 et mieux de 1 à 10 atomes de carbone, en particulier un radical méthyle, ou encore un groupement phényle et p est égal à 1 ou 2. De préférence R est un groupement hydrocarboné, de préférence le méthyle.

Ces composés Y organosiloxanes à unités alkylhydrogènosiloxanes peuvent comprendre en outre des unités de formule : 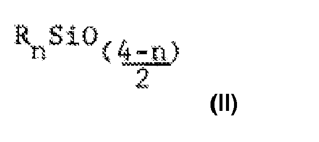 telles que définies plus haut.

Le composé Y peut être une résine de silicone comprenant au moins un motif choisis parmi les motifs M, D, T, Q tels que définis ci-dessus et comprenant au moins un groupe Si-H telles que les poly(methyl-hydridosilsesquioxane) commercialisées sous la référence SST-3MH1.1 par la société Gelest

De préférence, ces composés Y organosiloxanes comprennent de 0,5 à 2,5% en poids de groupes Si-H.

Avantageusement, les radicaux R représentent un groupement méthyle dans les formules (I), (II), (III) ci-dessus.

De préférence, ces organosiloxanes Y comprennent des groupes terminaux de formule (CH₃)₃SiO_{1/2}.

Avantageusement, les organosiloxanes Y comprennent au moins deux unités alkylhydrogènosiloxane de formule (H₃C)(H)SiO et comprennent éventuellement des unités (H₃C)₂SiO.

De tels composés Y organosiloxanes à groupements hydrogénosilane sont décrits par exemple dans le document EP 0465744.

Selon une variante, le composé X est choisi parmi les oligomères ou polymères organiques (par organique, on entend des composés dont la chaîne principale est non siliconée, de préférence des composés ne comprenant pas d'atomes de silicium) ou parmi les polymères ou oligomères hybrides organique/silicone, lesdits oligomères ou polymères portant au moins 2 groupements aliphatiques insaturés réactifs, le composé Y étant choisi parmi les hydrogénosiloxanes cités ci-dessus.

Le composé X, de nature organique, peut alors être choisi parmi les polymères ou oligomères vinyliques, (méth)acryliques, les polyesters, les polyuréthanes et/ou les polyurées, les polyéthers, les perfluoropolyéthers, les polyoléfines telles que le polybutène, le polyisobutylène, les dendrimères ou les polymères hyper-ramifiés organiques, ou leurs mélanges.

En particulier, le polymère organique ou la partie organique du polymère hybride peut être choisi parmi les polymères suivants :
a) les polyesters à insaturation(s) éthylénique(s) :
   II s'agit d'un groupe de polymères de type polyester présentant au moins 2 doubles liaisons éthyléniques, réparties de manière aléatoire dans la chaîne principale du polymère. Ces polyesters insaturés sont obtenus par polycondensation d'un mélange :
      - de diacides carboxyliques aliphatiques linéaires ou ramifiés ou cycloaliphatiques comportant notamment de 3 à 50 atomes de carbone, de préférence de 3 à 20 et mieux de 3 à 10 atomes de carbone, tels que l'acide adipique ou l'acide sébacique, de diacides carboxyliques aromatiques ayant notamment de 8 à 50 atomes de carbone, de préférence de 8 à 20 et mieux de 8 à 14 atomes de carbone, tels que les acides phtaliques, notamment l'acide téréphtalique, et/ou de diacides carboxyliques issus de dimères d'acides gras à insaturations éthyléniques tels que les dimères des acides oléique ou linoléique décrits dans la demande EP-A-959 066 (paragraphe [0021]) commercialisés sous les dénominations Pripol^{®} par la société Unichema ou Empol^{®} par la société Henkel, tous ces diacides devant être exempts de doubles liaisons éthyléniques polymérisables,
      - de diols aliphatiques linéaires ou ramifiés ou cycloaliphatiques comportant notamment de 2 à 50 atomes de carbone, de préférence de 2 à 20 et mieux de 2 à 10 atomes de carbone, tels que l'éthylèneglycol, le diéthylèneglycol, le propylèneglycol, le 1,4-butanediol ou le cyclohexanediméthanol, de diols aromatiques ayant de 6 à 50 atomes de carbone, de préférence de 6 à 20 et mieux de 6 à 15 atomes de carbone tel que le le bisphénol A et le bisphénol B, et/ou de dimères diols issus de la réduction des dimères d'acides gras tels que définis précédemment, et
      - d'un ou de plusieurs diacides carboxyliques ou leurs anhydrides comportant au moins une double liaison éthylénique polymérisable et ayant de 3 à 50 atomes de carbone, de préférence de 3 à 20 et mieux de 3 à 10 atomes de carbone, tels que l'acide maléique, l'acide fumarique ou l'acide itaconique.
b) les polyesters à groupes (meth)acrylate latéraux et/ou terminaux :
   II s'agit d'un groupe de polymères de type polyester obtenus par polycondensation d'un mélange :
      - de diacides carboxyliques aliphatiques linéaires ou ramifiés ou cycloaliphatiques comportant notamment de 3 à 50 atomes de carbone, de préférence de 3 à 20 et mieux de 3 à 10 atomes de carbone, tels que l'acide adipique ou l'acide sébacique, de diacides carboxyliques aromatiques ayant notamment de 8 à 50 atomes de carbone, de préférence de 8 à 20 et mieux de 8 à 14 atomes de carbone, tels que les acides phtaliques, notamment l'acide téréphtalique, et/ou de diacides carboxyliques issus de dimères d'acides gras à insaturation éthyléniques tels que les dimères des acides oléique ou linoléique décrits dans la demande EP-A-959 066 (paragraphe [0021]) commercialisés sous les dénominations Pripol^{®} par la société Unichema ou Empol^{®} par la société Henkel, tous ces diacides devant être exempts de doubles liaisons éthyléniques polymérisables,
      - de diols aliphatiques linéaires ou ramifiés ou cycloaliphatiques comportant notamment de 2 à 50 atomes de carbone, de préférence de 2 à 20 et mieux de 2 à 10 atomes de carbone, tels que l'éthylèneglycol, le diéthylèneglycol, le propylèneglycol, le 1,4-butanediol ou le cyclohexanediméthanol, de diols aromatiques ayant de 6 à 50 atomes de carbone, de préférence de 6 à 20 et mieux de 6 à 15 atomes de carbone tel que le bisphénol A et le bisphénol B, et
      - d'au moins un ester d'acide (méth)acrylique et d'un diol ou polyol ayant de 2 à 20 atomes de carbone, de préférence de 2 à 6 atomes de carbone, tels que le (méth)acrylate de 2-hydroxyéthyle, le (méth)acrylate de 2-hydroxypropyle et le méthacrylate de glycérol.
      Ces polyesters diffèrent de ceux décrits ci-dessus sous le point a) par le fait que les doubles liaisons éthyléniques ne sont pas situées dans la chaîne principale mais sur des groupes latéraux ou à l'extrémité des chaînes. Ces doubles liaisons éthyléniques sont celles des groupes (méth)acrylate présents dans le polymère.
      De tels polyesters sont commercialisés par exemple par la société UCB sous les dénominations EBECRYL^{®} (EBECRYL^{®} 450 : masse molaire 1600, en moyenne 6 fonctions acrylate par molécule, EBECRYL^{®} 652 : masse molaire 1500, en moyenne 6 fonctions acrylate par molécule, EBECRYL^{®} 800 : masse molaire 780, en moyenne 4 fonctions acrylate par molécule, EBECRYL^{®} 810 : masse molaire 1000, en moyenne 4 fonctions acrylate par molécule, EBECRYL^{®} 50 000 : masse molaire 1500, en moyenne 6 fonctions acrylate par molécule)
c) les polyuréthannes et/ou polyurées à groupes (méth)acrylate, obtenus par polycondensation
   - de diisocyanates, triisocyanates et/ou polyisocyanates aliphatiques cycloaliphatiques et/ou aromatiques ayant notamment de 4 à 50, de préférence de 4 à 30 atomes de carbone, tels que l'hexaméthylènediisocyanate, l'isophoronediisocyanate, le toluènediisocyanate, le diphénylméthanediisocyanate ou les isocyanurates de formule 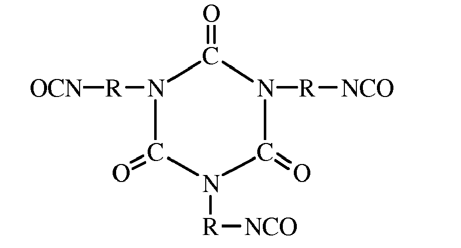 résultant de la trimérisation de 3 molécules de diisocyanates OCN-R-CNO, où R est un radical hydrocarboné linéaire, ramifié ou cyclique comportant de 2 à 30 atomes de carbone ;
   - de polyols, notamment de diols, exempts d'insaturations éthyléniques polymérisables, tels que le 1,4-butanediol, l'éthylèneglycol ou le triméthylolpropane, et/ou de polyamines, notamment de diamines, aliphatiques, cycloaliphatiques et/ou aromatiques ayant notamment de 3 à 50 atomes de carbone, telles que l'éthylènediamine ou l'hexaméthylènediamine, et
   - d'au moins un ester d'acide (méth)acrylique et d'un diol ou polyol ayant de 2 à 20 atomes de carbone, de préférence de 2 à 6 atomes de carbone, tels que le (méth)acrylate de 2-hydroxyéthyle, le (méth)acrylate de 2-hydroxypropyle et le méthacrylate de glycérol.
   De tels polyuréthannes/polyurées à groupes acrylates sont commercialisés par exemple sous la dénomination SR 368 (tris(2-hydroxyéthyl)isocyanurate-triacrylate) ou CRAYNOR^{®} 435 par la société CRAY VALLEY, ou sous la dénomination EBECRYL^{®} par la société UCB (EBECRYL^{®} 210 : masse molaire 1500, 2 fonctions acrylate par molécule, EBECRYL^{®} 230 : masse molaire 5000, 2 fonctions acrylate par molécule, EBECRYL^{®} 270 : masse molaire 1500, 2 fonctions acrylate par molécule, EBECRYL^{®} 8402 : masse molaire 1000, 2 fonctions acrylate par molécule, EBECRYL^{®} 8804 : masse molaire 1300, 2 fonctions acrylate par molécule, EBECRYL^{®} 220 : masse molaire 1000, 6 fonctions acrylate par molécule, EBECRYL^{®} 2220 : masse molaire 1200, 6 fonctions acrylate par molécule, EBECRYL^{®} 1290 : masse molaire 1000, 6 fonctions acrylate par molécule, EBECRYL^{®} 800 : masse molaire 800, 6 fonctions acrylate par molécule).
   On peut également citer les polyuréthannes aliphatiques diacrylate hydrosolubles commercialisés sous les dénominations EBECRYL^{®} 2000, EBECRYL^{®} 2001 et EBECRYL^{®} 2002, et les polyuréthannes diacrylate en dispersion aqueuse commercialisés sous les dénominations commerciales IRR^{®} 390, IRR^{®} 400, IRR^{®} 422 IRR^{®} 424 par la société UCB.
d) les polyéthers à groupes (meth)acrylate obtenus par estérification, par l'acide (méth)acrylique, des groupes hydroxyle terminaux d'homopolymères ou de copolymères d'alkylèneglycols en C₁₋₄, tels que le polyéthylèneglycol, le polypropylèneglycol, les copolymères d'oxyde d'éthylène et d'oxyde de propylène ayant de préférence une masse moléculaire moyenne en poids inférieure à 10 000, le triméthylolpropane polyéthoxylé ou polypropoxylé. Des polyoxyéthylènes-di(méth)acrylate de masse molaire appropriée sont commercialisés par exemple sous les dénominations SR 259, SR 344, SR 610, SR 210, SR 603 et SR 252 par la société CRAY VALLEY ou sous la dénomination EBECRYL^{®} 11 par UCB. Des triacrylates de triméthylolpropane polyéthoxylé sont commercialisés par exemple sous les dénominations SR 454, SR 498, SR 502, SR 9035, SR 415 par la société CRAY VALLEY ou sous la dénomination EBECRYL^{®} 160 par la société UCB. Des triacrylates de triméthylolpropane polypropoxylé sont commercialisés par exemple sous les dénominations SR 492 et SR 501 par la société CRAY VALLEY.
e) les époxyacrylates obtenus par réaction entre
   - au moins un diépoxyde choisi par exemple parmi :
      (i) l'éther diglycidylique de bisphénol A
      (ii) une résine diépoxy résultant de la réaction entre l'éther diglycidylique de bisphénol A et l'épichlorhydrine,
      (iii) une résine époxyester à extrémités α,ω-diépoxy résultant de la condensation d'un diacide carboxylique ayant de 3 à 50 atomes de carbone avec un excès stoechiométrique de (i) et/ou (ii),
      (iv) une résine époxyéther à extrémités α,ω-diépoxy résultant de la condensation d'un diol ayant de 3 à 50 atomes de carbone avec un excès stoechiométrique de (i) et/ou (ii),
      (v) les huiles naturelles ou synthétiques portant au moins 2 groupes époxyde, telles que l'huile de soja époxydée, l'huile de lin époxydée et l'huile de vernonia époxydée,
      (vi) un polycondensat phénol-formaldéhyde (résine Novolac^{®}), dont les extrémités et/ou les groupes latéraux ont été époxydés,
      et
   - un ou plusieurs acides carboxyliques ou polyacides carboxyliques comportant au moins une double liaison éthylénique en α,β du groupe carboxylique comme l'acide (méth)acrylique ou l'acide crotonique ou les esters d'acide (méth)acrylique et d'un diol ou polyol ayant de 2 à 20 atomes de carbone, de préférence de 2 à 6 atomes de carbone tels que le (méth)acrylate de 2-hydroxyéthyle.
   De tels polymères sont commercialisés par exemple sous les dénominations SR 349, SR 601, CD 541, SR 602, SR 9036, SR 348, CD 540, SR 480, CD 9038 par le société CRAY VALLEY, sous les dénominations EBECRYL^{®} 600 et EBECRYL^{®} 609, EBECRYL^{®} 150, EBECRYL^{®} 860, EBECRYL^{®} 3702 par la société UCB et sous les dénominations PHOTOMER^{®} 3005 et PHOTOMER^{®} 3082 par la société HENKEL.
f) les poly(méth)acrylates d'(alkyle en C₁₋₅₀), ledit alkyle étant linéaire, ramifié ou cyclique, comportant au moins deux fonctions à double liaison éthylénique portées par les chaînes hydrocarbonées latérales et/ou terminales.
   De tels copolymères sont commercialisés par exemple sous les dénominations IRR^{®} 375, OTA^{®} 480 et EBECRYL^{®}2047 par la société UCB.
g) les polyoléfines telles que le polybutène, le polyisobutylène,
h) les perfluoropolyéthers à groupes acrylate obtenus par estérification, par exemple par l'acide (méth)acrylique, de perfluoropolyéthers portant des groupes hydroxyle latéraux et/ou terminaux.
   De tels perfluoropolyéthers ⌀.∅-diols sont décrits notamment dans EP-A 1057849 et sont commercialisés par la société AUSIMONT sous la dénomination FOMBLIN^{®} Z DIOL.
i) les dendrimères et polymères hyperramifiés portant des groupes terminaux (méth)acrylate ou (méth)acrylamide obtenus respectivement par estérification ou amidification de dendrimères et de polymères hyperramifiés à fonctions terminales hydroxyle ou amino, par de l'acide (méth)acrylique.
Les dendrimères (du grec dendron = arbre) sont des molécules polymères "arborescentes", c'est-à-dire très ramifiées inventées par D. A. Tomalia et son équipe au début des années 90 (Donald A. Tomalia et al. Angewandte Chemie, Int. Engl. Ed., vol. 29, n° 2, pages 138 - 175). Il s'agit de structures construites autour d'un motif central généralement polyvalent. Autour de ce motif central, sont enchaînés, selon une structure parfaitement déterminée, des motifs ramifiés d'allongement de chaîne donnant ainsi naissance à des macromolécules symétriques, monodispersées ayant une structure chimique et stéréochimique bien définie. Des dendrimères de type polyamidoamine sont commercialisés par exemple sous la dénomination STARBUST^{®} par la société DENDRITECH.
Les polymères hyperramifiés sont des polycondensats, généralement de type polyester, polyamide ou polyéthylèneamine, obtenus à partir de monomères multifonctionnels, qui ont une structure arborescente similaire à celle des dendrimères mais beaucoup moins régulière que celle-ci (voir par exemple WO-A-93/17060 et WO 96/12754).
La société PERSTORP commercialise sous la dénomination BOLTORN^{®} des polyesters hyperramifiés. On trouvera sous la dénomination COMBURST^{®} de la société DENDRITECH des polyéthylèneamines hyperramifiées. Des poly(esteramide) hyperramifiés à extrémités hydroxyle sont commercialisés par la société DSM sous la dénomination HYBRANE^{®}.
Ces dendrimères et polymères hyperramifiés estérifiés ou amidifiés par l'acide acrylique et/ou méthacrylique se distinguent des polymères décrits sous les points a) à h) ci-dessus par le très grand nombre de doubles liaisons éthyléniques présentes. Cette fonctionnalité élevée, le plus souvent supérieure à 5, les rend particulièrement utiles en leur permettant de jouer un rôle de "noeud de réticulation", c'est-à-dire de site de réticulation multiple.
On peut donc utiliser ces polymères dendritiques et hyperramifiés en association avec un ou plusieurs des polymères et/ou oligomères a) à h) ci-dessus.

### 1a-Composés réactifs additionnels

Selon un mode de réalisation, les compositions comprenant le composé X et/ou Y peut comprendre en outre un composé réactif additionnel tels que
- les particules organiques ou minérales comprenant à leur surface au moins 2 groupements aliphatiques insaturés, on peut citer par exemple les silices traitées en surface par exemple par des composés siliconés à groupements vinyliques tels que par exemple la silice traitée cyclotetramethyl-tetravinylsiloxane,
- des composés silazanes tels que l'hexaméthyldisilazane.

### 1b-Catalyseur

La réaction d'hydrosilylation se fait avantageusement en présence d'un catalyseur qui peut être présent dans l'une ou l'autre des compositions comprenant le composé X et/ou le composé Y ou dans une composition séparée, le catalyseur étant de préférence à base de platine ou d'étain.

On peut citer par exemple les catalyseurs à base de platine déposé sur un support de gel de silice ou de poudre de charcoal (charbon), le chlorure de platine, les sels de platine et d'acides chloroplatiniques.

On utilise de préférence les acides chloroplatiniques sous forme hexahydrate ou anhydre, facilement dispersible dans les milieux organosiliconés.

On peut également citer les complexes de platine tels que ceux à base d'acide chloroplatinique hexahydrate et de divinyl tetramethyldisiloxane.

La catalyseur peut être présent dans l'une ou l'autre des compositions utiles dans la présente invention en une teneur allant 0,0001% à 20% en poids par rapport au poids total de la composition le comprenant.

On peut également introduire dans les compositions de l'invention des inhibiteurs ou retardateurs de polymérisation, et plus particulièrement des inhibiteurs du catalyseur, ceci afin d'accroître la stabilité de la composition dans le temps ou de retarder la polymérisation. De façon non limitative, on peut citer les polymethylvinylsiloxanes cycliques, et en particulier le tetravinyl tetramethyl cyclotetrasiloxane, les alcools acétyléniques, de préférence volatiles, tels que le methylisobutynol .

La présence de sels ioniques, tels que l'acétate de sodium, dans l'une et/ou l'autre des première et seconde compositions peut avoir une influence dans la vitesse de polymérisation des composés.

De façon avantageuse, les composés X et Y sont choisis parmi les composés siliconés susceptibles de réagir par hydrosilylation ; en particulier le composé X est choisi parmi les polyorganosiloxanes comprenant des unités de formule (I) décrits ci-dessus et le composé Y est choisi parmi les organosiloxanes comprenant des unités alkylhydrogénosiloxanes de formule **(III)** décrits ci-dessus. Selon un mode de réalisation particulier, le composé X est un polydimethylsiloxane à groupements vinyliques terminaux, et le composé Y est le méthylhydrogénosiloxane.

A titre d'exemple d'une combinaison de composés X et Y réagissant par hydrosilylation, on peut citer mélanges suivants proposée par la société Dow Corning, mélanges A et B suivants préparés par Dow Corning :

### Mélange A :

| Ingrédient (Nom INCI) | N°CAS | Teneurs (%) | Fonction |
|---|---|---|---|
| Dimethyl Siloxane, Dimethylvinylsiloxy-terminated | 68083-19-2 | 55-95 | Polymère |
| Silica Silylate | 68909-20-6 | 10-40 | Charge |
| 1,3-Diethenyl-1,1,3,3-Tetramethyldisiloxane complexes | 68478-92-2 | Trace | Catalyseur |
| Tetramethyldivinyldisiloxane | 2627-95-4 | 0.1-1 | Polymère |

### Mélange B :

| Ingrédient (Nom INCl) | N°CAS | Teneurs (%) | Fonction |
|---|---|---|---|
| Dimethyl Siloxane, Dimethylvinylsiloxy-terminated | 68083-19-2 | 55-95 | Polymère |
| Silica Silylate | 68909-20-6 | 10-40 | Charge |
| Dimethyl, Methylhydrogen Siloxane, trimethylsiloxy-terminated | 68037-59-2 | 1-10 | Polymère |

### 2-Composés X et Y susceptibles de réagir par condensation

Selon ce mode de réalisation, les composés X et Y sont susceptibles de réagir par condensation, soit en présence d'eau (hydrolyse) par réaction de 2 composés porteurs de groupements alcoxysilanes, soit par condensation dite « directe » par réaction d'un composé porteur de groupement(s) alcoxysilane(s) et d'un composé porteur de groupement(s) silanol(s) ou par réaction de 2 composés porteurs de groupement(s) silanol(s).

Lorsque la condensation se fait en présence d'eau, celle-ci peut être en particulier l'humidité ambiante, la sueur ou l'eau apportée par une source extérieure, par exemple par humidification préalable des fibres kératiniques (par exemple par un brumisateur).

Préférentiellement, lorsque la composition selon l'invention comprend des composés X et Y susceptibles de réagir par condensation, alors le colorant fluorescent est différent de la Rhodamine 101 :

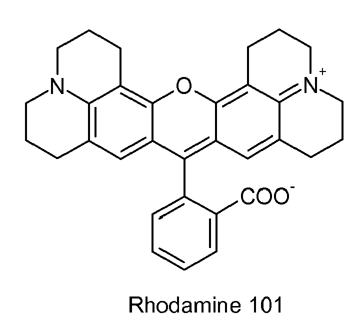

Dans ce mode de réaction par condensation, les composés X et Y, identiques ou différents, peuvent donc être choisis parmi les composés siliconés dont la chaîne principale comprend au moins deux groupes alcoxysilane et/ou au moins deux groupes silanol (Si-OH), latéraux et/ou en bout de chaîne.

Selon un mode de réalisation avantageux, les composés X et/ou Y sont choisis parmi les polyorganosiloxanes comprenant au moins deux groupes alcoxysilane. Par groupe « alcoxysilane », on entend un groupe comprenant au moins une partie -Si-OR, R étant un groupe alkyle comprenant de 1 à 6 atomes de carbone.

Les composés X et Y sont notamment choisis parmi les polyorganosiloxanes comprenant des groupes terminaux alcoxysilanes, plus spécifiquement ceux qui comprennent au moins 2 groupes alcoxysilanes terminaux, de préférence trialcoxysilanes terminaux.

Ces composés X et/ou Y comprennent de préférence de façon majoritaire des unités de formule :

R⁹ₛSiO_{(4-s)/2}, (IV)

dans laquelle R⁹ représente indépendamment un radical choisi parmi les groupes alkyles comprenant de 1 à 6 atomes de carbone, le phényle, les groupes alkyl fluorés, et S est égal à 0, 1, 2 ou 3. De préférence, R⁹ représente indépendamment un groupe alkyle comprenant de 1 à 6 atomes de carbone. Comme groupe alkyle, on peut citer notamment le méthyle, le propyle, le butyle, l'hexyle et leurs mélanges, de préférence le méthyle ou l'éthyle. Comme groupe fluoroalkyle, on peut citer le 3, 3, 3-trifluoropropyle.

Selon un mode de réalisation particulier, les composés X et Y, identiques ou différents, sont des polyorganosiloxanes comprenant des unités de formule

(R⁹₂8iO₂)_{f} - **(V)**

dans laquelle R⁹ est tel que décrit ci-dessus, de préférence R⁹ est un radical méthyle, et f est tel que le polymère présente avantageusement une viscosité à 25°C allant de 0,5 à 3000 Pa.s, de préférence allant de 5 à 150 Pa.s. f est notamment un nombre allant de 2 à 50000, particulièrement de 3 à 3000 plus particulièrement de 5 à 1000.

Ces composés X et Y polyorganosiloxanes comprennent au moins 2 groupes trialcoxysilanes terminaux par molécule de polymère, lesdits groupes ayant la formule suivante :

-ZSiR¹ₓ(OR)₃₋ₓ, (VI)

dans laquelle
les radicaux R représentent indépendamment un groupe méthyle, éthyle, n-propyle, isopropyle, n-butyle, sec-butyle, isobutyle, de préférence un groupe méthyle ou éthyle,
R¹ est un groupe méthyle ou éthyle,
x est égal à 0 ou 1, de préférence x est égal à 0 et
Z est choisi parmi : les groupes hydrocarbonés divalents ne comportant pas d'insaturation éthylénique et comprenant de 1 à 18 atomes de carbone (groupes alkylène), préférentiellement de 2 à 18 atomes de carbones, les combinaisons de radicaux hydrocarbonés divalents et de segments siloxanes de formule **(IX)** suivante : 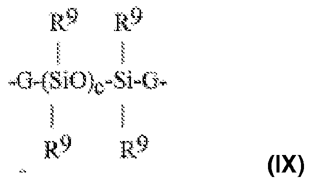 R⁹ étant tel que décrit plus haut, G est un radical hydrocarboné divalent ne comportant pas d'insaturation éthylénique et comprenant de 1 à 18 atomes de carbone, préférentiellement de 2 à 18 atome de carbone, et c est un entier allant de 1 à 6.
Z et G peuvent être notamment choisis parmi les groupements alkylènes tels que le méthylène, l'éthylène, le propylène, le butylène, le pentylène, l'hexylène, les groupements arylène tels que le phenylène.

De préférence, Z est un groupe alkylène, et mieux éthylène.

Ces polymères peuvent présenter en moyenne au moins 1,2 groupements terminaux ou chaînes terminales trialcoxysilanes par molécule, et de préférence en moyenne au moins 1,5 groupements terminaux trialcoxysilanes par molécule. Ces polymères pouvant présenter au moins 1,2 groupements terminaux trialcoxysilanes par molécule, certains peuvent comprendre d'autre types de groupes terminaux tels que des groupements terminaux de formule CH₂=CH-SiR⁹₂- ou de formule R⁶₃- Si-, dans laquelle R⁹ est tel que défini plus haut et chaque groupe R⁶ est indépendamment choisi parmi les groupes R⁹ ou vinyle. On peut citer comme exemples de tels groupements terminaux les groupes triméthoxysilane, triéthoxysilane, vinyldiméthoxysilane et vinylméthyl-oxyphénylsilane.

De tels polymères sont notamment décrits dans les documents US 3 175 993, US 4 772 675, US 4 871 827, US 4 888 380, US 4 898 910, US 4 906 719 et US 4 962 174 dont le contenu est incorporé par référence à la présente demande.

On peut citer à titre de composé X et/ou Y en particulier le polymère de formule 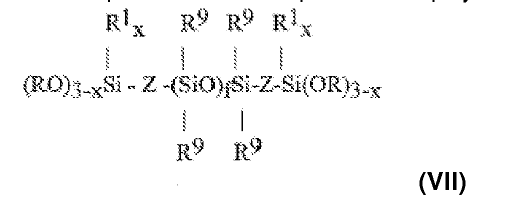 dans laquelle R, R¹, R⁹, Z, x et f sont tels que décrits plus haut.

Les composés X et/ou Y peuvent également comprendre un mélanges de polymère de formule **(VII)** ci-dessus avec des polymères de formule **(VIII)** suivante : 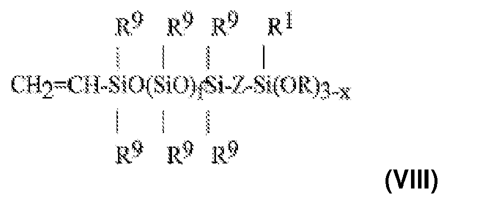 dans laquelle R, R¹, R⁹, Z, x et f sont tels que décrits plus haut.

Lorsque le composé X et/ou Y polyorganosiloxanes à groupe(s) alcoxysilane(s) comprend un tel mélange, les différents polyorganosiloxanes sont présents en des teneurs telles que les chaînes organosilyles terminales représentent moins de 40%, de préférence moins de 25% en nombre des chaînes terminales

Les composés X et/ou Y polyorganosiloxanes particulièrement préférés sont ceux de formule (VII) décrits ci-dessus. De tels composés X et/ou Y sont décrits par exemple dans le document WO 01/96450.

Comme indiqué plus haut, les composés X et Y peuvent être identiques ou différents.

Selon une variante l'un des 2 composés réactifs X ou Y, est de nature silicone et l'autre est de nature organique. Par exemple le composé X est choisi parmi les oligomères ou polymères organiques ou les oligomères ou polymères hybrides organique/silicone, lesdits polymères ou oligomères comprenant au moins deux groupements alcoxysilanes et Y est choisi parmi les composés siliconés tels que les polyorganosiloxanes décrits ci-dessus. En particulier, les oligomères ou polymères organiques sont choisis parmi les oligomères ou polymères vinyliques, (méth)acryliques, polyesters, polyamides, polyuréthanes et/ou polyurées, polyéthers, polyoléfines, perfluoropolyéthers, dendrimères et polymères hyper-ramifiés organiques, et leurs mélanges.

Les polymères organiques de nature vinylique ou (méth)acryliques, porteurs de groupes latéraux alcoxysilanes, pourront en particulier être obtenus par copolymérisation d'au moins un monomère organique vinylique ou (meth)acrylique avec un (méth)acryloxypropyltriméthoxysilane, un vinyl triméthoxysilane, un vinyltriéthoxysilane, un allyltriméthoxysilanes etc..

On peut citer par exemple les polymère (meth)acryliques décrits dans le document de KUSABE.M, Pitture e Verniei - European Coating ; 12-B, pages 43-49, 2005, et notamment les polyacrylates à groupes alcoxysilanes référencés MAX de Kaneka ou ceux décrits dans la publication de PROBSTER, M, Adhesion-Kleben & Dichten, 2004, 481 (1-2), pages 12-14.

Les polymères organiques résultant d'une polycondensation ou d'une polyaddition, tel que les polyesters, polyamides, polyuréthanes et/ou polyurées, polyéthers, et porteurs de groupes alcoysilanes latéraux et/ou terminaux, pourront résulter par exemple de la réaction d'un prépolymère oligomère tel que décrit plus haut avec l'un des co-réactifs silanes suivant porteurs d'au moins un groupe alcoxysilane : aminopropyltriméthoxysilane, aminopropyltriéthoxysilane, aminoéthyl aminopropyl triméthoxysilane, glycidoxypropyltriméthoxysilane, glycidoxypropyltriéthoxysilane, époxycyclohéxyléthyl-triméthoxysilane, mercaptopropyltriméthoxysilane.

Des exemples de polyéthers et de polyisobutylènes à groupes alcoxysilanes sont décrits dans la publication de KUSABE.M., Pitture e Verniei - European Coating ; 12-B, pages 43-49, 2005. Comme exemple de polyuréthanes à groupes alcoxysilanes terminaux , on peut citer ceux décrits dans le document PROBSTER, M., Adhesion-Kleben & Dichten, 2004, 481 (1-2), pages 12-14 ou encore ceux décrits dans le document LANDON, S., Pitture e Verniei vol. 73, N° 11 , pages 18-24, 1997 ou dans le document HUANG, Mowo, Pitture e Verniei vol. 5, 2000, pages 61-67, on peut notamment citer les polyuréthanes à groupes alcoxysilanes de OSI-WITCO-GE.

A titre de composés X et/ou Y polyorganosiloxane, on peut citer les résines de type MQ ou MT portant elle-même des extrémités alcoxysilanes et/ou silanols comme par exemple les résines poly(isobutylsilsesquioxane) fonctionnalisées par des groupes silanols proposées sous la référence SST-S7C41 (3 groupes Si-OH) par la société Gelest.

### 2a-Composé réactif additionnel

L'une des compositions utiles dans la présente invention peut comprendre en outre un composé réactif additionnel comprenant au moins deux groupes alcoxysilane ou silanol.

On peut citer par exemple :
- une ou des particules organiques ou minérales comprenant à leur surface des groupements alcoxysilanes et/ou silanols, par exemple des charges traitées en surface par de tels groupes.

### 2b-Catalyseur

La réaction de condensation peut se faire en présence d'un catalyseur à base de métal qui peut être présent dans l'une ou l'autre des compositions comprenant X et/ou Y ou dans une composition séparée. Le catalyseur utile dans ce type de réaction est de préférence un catalyseur à base de titane.

On peut citer notamment les catalyseurs à base de tetraalcoxytitane de formule

Ti(OR²)_{y}(OR³)_{4-y},

dans laquelle R² est choisi parmi les radicaux alkyle tertiaires tels que le tert butyle, le tert amyle et le 2,4-diméthyl-3-pentyl ; R³ représente un radical alkyle comprenant de 1 à 6 atomes de carbone, de préférence un groupe methyle ethyle, n-propyle, isopropyle, n-butyle, sec-butyle, hexyle et y est un nombre allant de 3 à 4, mieux de 3,4 à 4.

Le catalyseur peut être présent dans l'une ou l'autre des compositions utiles dans la présente invention en une teneur allant 0,0001 % à 20% en poids par rapport au poids total de la ou des compositions le contenant.

### 2c-Diluant

Les compositions utiles comprenant X et/ou Y peuvent comprendre en outre une huile siliconée volatile (ou diluant) destinée à faire diminuer la viscosité de la composition. Cette huile peut être choisie parmi les silicones linéaires à chaîne courte telles que l'hexamethyldisiloxane, l'octamethyltrisiloxane, les silicones cycliques telles que l'octamethylcyclotetrasiloxane, la decamethylclopentasiloxane et leurs mélanges.

Cette huile siliconée peut représenter de 5% à 95%, de préférence de 10 à 80% en poids par rapport au poids de chaque composition.

A titre d'exemple de composés X et Y porteurs de groupements alcoxysilanes et réagissant par condensation, on peut citer les mélanges fournis par la société Dow Corning A' et B' ci-après.

### Mélange A' :

| **Ingrédient (Nom INCl)** | **N°CAS** | **Teneurs** (%) | **Fonction** |
|---|---|---|---|
| Bis-Trimethoxysiloxyethyl Tetramethyldisiloxyethyl Dimethicone* | PMN87176 | 25-45 | Polymère |
| Silica Silylate | 68909-20-6 | 5-20 | Charge |
| Disiloxane | 107-46-0 | 30-70 | Solvant |

| | | | |
|---|---|---|---|
| * correspond aux composés X et Y identiques | | | |

### Mélange B' :

| **Ingrédient (Nom INCI)** | **N°CAS** | **Teneurs (%)** | **Fonction** |
|---|---|---|---|
| Disiloxane | 107-46-0 | 80-99 | Solvant |
| Tetra T Butyl Titanate | - | 1-20 | Catalyseur |

Il est d'ailleurs à noter que les composés X et Y identiques sont réunis dans le mélange A'.

### 3-Réticulation en présence de peroxyde :

Cette réaction se fait de préférence par chauffage à une température supérieure ou égale à 50°C, de préférence supérieure ou égale à 80°C, allant jusqu'à 120°C.

Les composés X et Y, identiques ou différents, comprennent dans ce cas au moins deux groupements latéraux -CH₃ et/ou au moins deux chaînes latérales portant un groupement -CH₃.

Les composés X et Y sont de préférence siliconés et peuvent être choisis par exemple parmi les polydimethylsiloxanes linéaires non volatiles de haut poids moléculaire, ayant un degré de polymérisation supérieur à 6 présentant au moins deux groupements latéraux -CH₃ reliés à l'atome de silicium et/ou au moins deux chaînes latérales portant un groupement -CH₃. On peut citer par exemple les polymères décrits dans le Catalogue « Reactive Silicones » de la société Gelest Inc., Edition 2004, page 6, et notamment les copolymères (aussi appelés gommes) de vinylmethylsiloxane-dimethylsiloxane de poids moléculaires allant de 500 000 à 900 000 et de viscosité supérieure à 2 000 000 cSt.

A titre de peroxydes utilisables dans le cadre de l'invention, on peut citer le peroxyde de benzoyle, le peroxyde de 2,4-dichlorobenzoyle et leurs mélanges.

Selon un mode de réalisation, la réaction d'hydrosilylation ou la réaction de condensation ou bien encore la réaction de réticulation en présence d'un peroxyde entre les composés X et Y est accélérée par un apport de chaleur en élevant par exemple la température du système entre 25°C et 180°C. Le système réagira notamment sur la peau.

D'une façon générale, quel que soit le type de réaction par laquelle les composés X et Y réagissent ensemble, le pourcentage molaire de X par rapport à l'ensemble des composés X et Y, c'est-à-dire le ratio X/(X+Y) x 100, peut varier de 5% à 95%, de préférence de 10% à 90%, mieux encore de 20% à 80%.

De même, le pourcentage molaire de Y par rapport à l'ensemble des composés X et Y, c'est-à-dire le ratio Y/(X+Y) x 100, peut varier de 5% à 95%, de préférence de 10% à 90%, mieux encore de 20% à 80%.

Le composé X peut présenter une masse moléculaire moyenne en poids (Mw) allant de 150 à 1 000 000, de préférence de 200 à 800 000, de préférence encore de 200 à 250 000.

Le composé Y peut présenter une masse moléculaire moyenne en poids (Mw) allant de 200 à 1 000 000, de préférence de 300 à 800 000, de préférence encore de 500 à 250 000.

Le composé X peut représenter de 0,5% à 95% en poids par rapport au poids total des compositions utiles ou par rapport au poids total de la composition lorsque X et Y sont présents au sein d'une même composition, de préférence de 1% à 90% et mieux de 5% à 80%.

Le composé Y peut représenter de 0.05 % à 95% en poids par rapport au poids total des compositions utiles ou par rapport au poids total de la composition lorsque X et Y sont présents au sein d'une même composition, de préférence de 0,1% à 90% et mieux de 0,2% à 80%.

Le ratio entre les composés X et Y peut être varié de manière à moduler la vitesse de réaction et donc la vitesse de formation du film ou encore de manière à adapter les propriétés du film formé (par exemple ses propriétés adhésives) selon l'application recherchée.

En particulier, les composés X et Y peuvent être présents en un ratio X/Y molaire allant de 0,05 à 20 et mieux de 0,1 à 10.

L'invention est illustrée plus en détails par les exemples décrits ci-après. Sauf indication contraire, les quantités indiquées sont exprimées en pourcentage massique.

### Les agents texturisants

La composition appliquée sur les fibres capillaires peut en outre comprendre un ou plusieurs agents texturisants (charges), différents des pigments, colorants fluorescents ou azurants optiques présents dans la composition.

On entend par agents texturisants, des particules minérales ou de synthèse, lamellaires ou non lamellaires, insolubles dans l'eau.

A titre d'exemple, ces agents texturisants peuvent être du carbonate de calcium colloïdal qui peut être traité ou non par de l'acide stéarique ou du stéarate, de la silice telle que les silices pyrogénées les silices précipitées, les silices traitées pour les rendre hydrophobes, du quartz moulu, de l'alumine, de l'hydroxyde d'aluminium, du dioxyde de titane, de la terre de diatomée, de l'oxyde de fer, du noir de carbone.

On peut aussi citer le talc, le mica, le kaolin, les poudres de polyamide (Nylon@) (Orgasol de chez Atochem), les poudres de polyéthylène, les poudres de polymères de tétrafluoroéthylène (TéflonB), l'amidon, , des microsphères polymériques telles que celles de chlorure de polyvinylidène/acrylonitrile comme l'Expancel (Nobel Industrie), de copolymères d'acide acrylique (PolytrapB de la société Dow Corning).

Les silices synthétiques dont la surface est modifiée par des composés siliconés pour les rendre hydrophobe en surface sont particulièrement préférées. Ces charges se différentient les unes des autres par leurs propriétés de surface, les composés de silicone utilisés pour traiter la silice, et la façon dont le traitement en surface est réalisé.

De tels agents permettent de modifier la viscosité de la formulation obtenue à partir des composés X et/ou Y et/ou de modifier les propriétés du matériau obtenu.

On préfère à titre d'agent texturisant la silice, le carbonate de calcium, les agents à base de résine.

A titre d'exemple, on peut citer les silices traitées Cab-O-Sil@TS-530, Aerosil@R8200 et Wacker HDX H2000.

Les agents texturisants peuvent être présents à raison de 0 à 48 % en poids, par rapport au poids total de la composition, de préférence 0,01 à 30 % en poids, et mieux de 0,02 % à 20 % en poids.

### II) Colorants fluorescents :

Les compositions de l'invention contiennent au moins un colorant fluorescent sous forme soluble ou sous forme de pigment. Ces compositions peuvent contenir en outre au moins un azurant optique sous forme soluble ou sous forme de pigment.

On entend par ailleurs par composé fluorescent des colorants fluorescents et lorsqu'ils sont présents des azurants optiques.

On entend par pigment tout composé fluorescent qui présente dans le milieu une solubilité dans l'eau inférieure à 0,01 % à 20 °C et à pression atmosphérique (760 mmHg)

Dans le cadre de l'invention, on entend par composé fluorescent soluble, un composé fluorescent qui présente une solubilité dans le milieu supérieure à 0,01 % à 20°C, et à pression atmosphérique préférablement supérieure à 0,05 %.

Les colorants fluorescents sont des composés fluorescents qui absorbent dans le rayonnement visible généralement entre 400 et 800 nm et qui sont capables de réémettre de la lumière dans le domaine du visible à une longueur d'onde supérieure. Par définition, ces colorants sont des espèces colorées puisqu'elles absorbent de la lumière visible.

Selon un mode de réalisation particulier, les colorants fluorescents utiles dans le cadre de l'invention réémettent de la lumière fluorescente orangée. Ils présentent notamment une longueur d'onde maximum de ré-émission comprise entre 500 et 700 nm.

A titre d'exemple de colorants fluorescents, on peut citer les composés connus de la technique, décrits par exemple dans les ouvrages suivants : Ullmann's Encyclopedia of Industrial Chemistry Release 2004, 7th edition, chapitre « Fluorescent Dyes ».

Les composés fluorescents qui peuvent être utilisés dans le cadre de l'invention sont des composés connus de la technique. Ils sont par exemple décrits dans le document FR 2 830 189.

Comme composés fluorescents solubles on peut notamment mentionner ceux appartenant aux familles suivantes naphtalimides, coumarines, xanthènes et en particulier xanthénodiquinolizines et azaxanthènes ; naphtolactames ; azlactones ; oxazines ; thiazines ; dioxazines ; azoïques ; azométhines ; méthines ; pyrazines ; stilbènes ; cétopyroles ; pyrènes.

A titre d'exemples de colorants fluorescents, on peut notamment citer :
- Les dérivés méthine de formule : 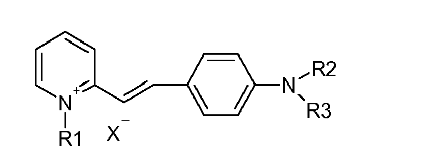
dans laquelle R₁, R₂ et R₃ indépendamment les uns des autres représentent un atome d'hydrogène ; un atome d'halogène ; un groupement aryle en C₆-C₃₀; un groupement hydroxy ; un groupement cyano ; un groupement nitro ; un groupement sulfo ; un groupement amino ; un groupement acylamino ; un groupement dialkyl(C₁-C₆)amino ; un groupement dihydroxyalkyl(C₁-C₆)amino; un groupement alkyl(C₁-C₆)hydroxyalkyl(C₁-C₆)amino ; un groupement alcoxy(C₁-C₆); un groupement alcoxy(C₁-C₆)carbonyle; un groupement carboxyalcoxy en C₁-C₆ un groupement pipéridinosulfonyle ; un groupement pyrrolidino ; un groupement alkyle(C₁-C₆)halogénoalkyle(C₁-C₆)amino ; un groupement benzoylalkyle(C₁-C₆) un groupement vinyle ; un groupement formyle ; un radical aryle en C₆-C₃₀ éventuellement substitué par un ou plusieurs groupements choisis parmi les groupements hydroxyle, alcoxy en C₁-C₆ linéaire, ramifié ou cyclique, alkyle linéaire, ramifié ou cyclique comprenant de 1 à 22 atomes de carbone lui-même étant éventuellement substitué par un ou plusieurs groupements hydroxyle,amino, alcoxy C₁-C₆ ; un radical alkyle linéaire, ramifié ou cyclique, comprenant de 1 à 22 atomes de carbone, plus particulièrement de 1 à 6 atomes de carbone, éventuellement substitué par un ou plusieurs groupements choisis parmi les groupements hydroxyle, amino, alcoxy en C₁-C₆ linéaire, ramifié ou cyclique, aryle éventuellement substitué, carboxyle, sulfo, un atome d'halogène,ce radical alkyle pouvant être interrompu par un hétéroatome, tel que l'azote, le soufre et l'oxygène ;
deux des substituants R₂ , R₃ peuvent former avec les atomes de carbone auxquels ils sont rattachés un noyau aromatique en C₆-C₃₀ ou hétérocyclique comportant au total de 5 à 30 chaînons et de 1 à 5 hétéroatomes ; ce cycle étant condensés ou non, ce cycle et l'éventuel cycle condensé étant substitués ou non par au moins un groupement choisi parmi les groupements alkyle en C₁-C₄, alcoxy(C₁-C₄)alkyl(C₁-C₄), amino, dialkyl(C₁-C₄)amino, halogène, phényle, carboxy, trialkyl(C₁-C₄)ammonioalkyl(C₁-C₄), et
X- est un anion organique ou minéral. On peut citer par exemple pour X- les ions Chlorure, Bromure, Iodure, méthosulfate, éthosulfate, mésylate, tosylate, acétate, les sels d'acide organique simple tel que les lactate, les oléate, les benzoate, les perchlorate, les triflate.

Particulièrement on peut citer les sels de 2-[2-(4-diméthylamino)phényléthényl]-1 éthyl-pyridinium : 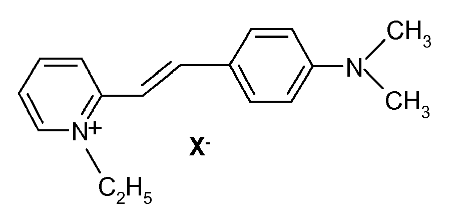 avec X- représentant un anion pouvant être choisi parmi les halogénure, les alkylsulfates tel que le méthyl sulfate et l'éthylsulfate ; les alkoxysulfates tels que le méthoxysulfate et l'éthoxysulfate ; les phosphates ; le lactate ; le citrate ; l'acétate et le tatrate.

Particulièrement le contre-ion est choisi parmi chlorure et éthosulfate.

Conviennent aussi les composés de formules ci-dessous : 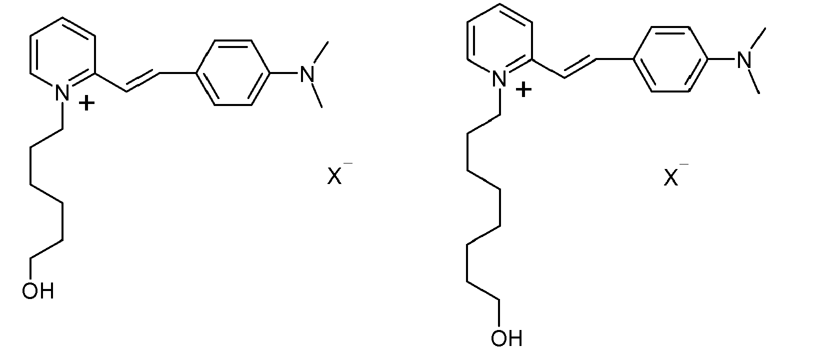 Avec X- tel que défini précédemment.
- le Jaune Brilliant B6GL commercialisé par la société SANDOZ et de structure suivante : 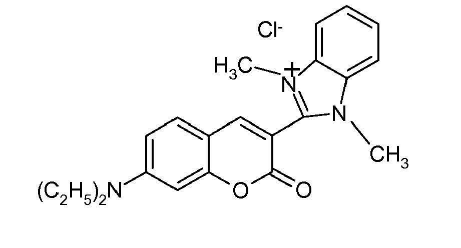
- l'hémicyanine méthinique suivante : 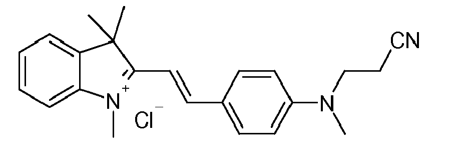
- le Basic Yellow 2, ou Auramine O commercialisé par les sociétés PROLABO, ALDRICH ou CARLO ERBA et de structure suivante : 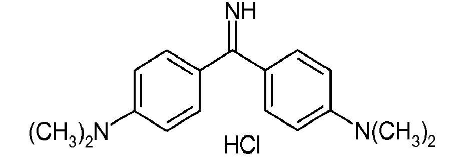 monochlorhydrate de 4,4'-(imidocarbonyl)bis(N,N-diméthylaniline)
- On peut aussi citer les composés de formule suivante : 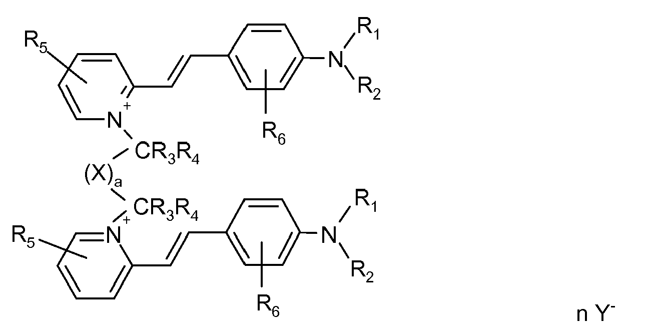 dans laquelle :
   R₁, R₂, identiques ou différents, représentent :
      ■ un atome d'hydrogène ;
      ■ un radical alkyle, linéaire ou ramifié, comprenant 1 à 10 atomes de carbone, de préférence de 1 à 4 atomes de carbone, éventuellement interrompu et/ou substitué par au moins un hétéroatome et/ou groupement comprenant au moins un hétéroatome et/ou substitué par au moins un atome d'halogène ;
      ■ un radical aryle ou arylalkyle, le groupement aryle ayant 6 atomes de carbone et le radical alkyle ayant 1 à 4 atomes de carbone ; le radical aryle étant éventuellement substitué par un ou plusieurs radicaux alkyles linéaires ou ramifiés comprenant 1 à 4 atomes de carbone éventuellement interrompus et/ou substitués par au moins un hétéroatome et/ou groupement comprenant au moins un hétéroatome et/ou substitué par au moins un atome d'halogène ;
      ■ R₁ et R₂ peuvent éventuellement être reliés de manière à former un hétérocycle avec l'atome d'azote et comprendre un ou plusieurs autres hétéroatomes, l'hétérocycle étant éventuellement substitué par au moins un radical alkyle linéaire ou ramifié, comprenant de préférence de 1 à 4 atomes de carbone et étant éventuellement interrompu et/ou substitué par au moins un hétéroatome et/ou groupement comprenant au moins un hétéroatome et/ou substitué par au moins un atome d'halogène ;
      ■ R₁ ou R₂ peut éventuellement être engagé dans un hétérocycle comprenant l'atome d'azote et l'un des atomes de carbone du groupement phényle portant ledit atome d'azote ;
      ■ R₃, R₄, identiques ou non, représentent un atome d'hydrogène, un radical alkyle comprenant 1 à 4 atomes de carbone ;
      ■ R₅, identiques ou non, représentent un atome d'hydrogène, un atome d'halogène, un radical alkyle linéaire ou ramifié comprenant 1 à 4 atomes de carbone éventuellement interrompu par au moins un hétéroatome ;
      ■ R₆, identiques ou non, représentent un atome d'hydrogène ; un atome d'halogène ; un radical alkyle linéaire ou ramifié comprenant 1 à 4 atomes de carbone, éventuellement substitué et/ou interrompu par au moins un hétéroatome et/ou groupement portant au moins un hétéroatome et/ou substitué par au moins un atome d'halogène ;
      X représente :
      ■ un radical alkyle, linéaire ou ramifié comprenant 1 à 14 atomes de carbone, ou alcényle comprenant 2 à 14 atomes de carbone, éventuellement interrompu et/ou substitué par au moins un hétéroatome et/ou groupement comprenant au moins un hétéroatome et/ou substitué par au moins un atome d'halogène ;
      ■ un radical hétérocyclique comprenant 5 ou 6 chaînons, éventuellement substitué par au moins un radical alkyle linéaire ou ramifié comprenant 1 à 14 atomes de carbone, éventuellement substitué par au moins un hétéroatome ; par au moins un radical aminoalkyle, linéaire ou ramifié, comprenant 1 à 4 atomes de carbone, éventuellement substitué par au moins un hétéroatome ; par au moins un atome d'halogène ;
      ■ un radical aromatique ou diaromatique condensé ou non, séparé ou non par un radical alkyle comprenant 1 à 4 atomes de carbone, le ou les radicaux aryles étant éventuellement substitués par au moins un atome d'halogène ou par au moins un radical alkyle comprenant 1 à 10 atomes de carbone éventuellement substitué et/ou interrompu par au moins un hétéroatome et/ou groupement portant au moins un hétéroatome;
      ■ un radical dicarbonyle ;
      ■ le groupement X pouvant porter une ou plusieurs charges cationiques ;
      ■ a étant égal à 0 ou 1 ;
      ■ Y⁻, identiques ou non, représentant un anion organique ou minéral ;
      ■ n étant un nombre entier au moins égal à 2 et au plus égal au nombre de charges cationiques présentes dans le composé fluorescent.

Rappelons que les termes hétéroatomes, représentent un atome d'oxygène ou d'azote.

Parmi les groupements porteurs de tels atomes, on peut citer entre autres les groupements hydroxyle, alcoxy, carbonyle, amino, ammonium, amido (-N-CO-), carboxyle (-O-CO- ou -CO-O-).

En ce qui concerne les groupements alcényles, ces derniers comprennent une ou plusieurs liaisons carbone-carbone insaturée (-C=C-), et de préférence une seule double liaison carbone-carbone.

Dans cette dernière formule générale, les radicaux R1 et R2, identiques ou non, représentent plus particulièrement :
- un atome d'hydrogène ;
- un radical alkyle comprenant 1 à 10 atomes de carbone, notamment 1 à 6 atomes de carbone, de préférence 1 à 4 atomes de carbone, éventuellement interrompu par un atome d'oxygène ou éventuellement substitué par au moins un radical hydroxyle, amino, ammonium, d'un atome de chlore ou de fluor ;
- un radical benzyle, phényle, éventuellement substitué par un radical alkyle ou alcoxy comprenant 1 à 4 atomes de carbone, de préférence 1 ou 2 atomes de carbone ;
- avec l'atome d'azote, un radical hétérocylique du type pyrrolo, pyrrolidino, imidazolino, imidazolo, imidazolium, pyrazolino, pipérazino, morpholino, morpholo, pyrazolo, triazolo, éventuellement substitué par au moins un radical alkyle linéaire ou ramifié comprenant 1 à 4 atomes de carbone éventuellement interrompu et/ou substitué par un atome d'azote et/ou d'oxygène et/ou groupement portant un atome d'azote et/ou d'oxygène.

En ce qui concerne les radicaux amino ou ammonium précités, les radicaux portés par l'atome d'azote peuvent ou non être identiques et représenter plus particulièrement un atome d'hydrogène, un radical alkyle en C₁-C₁₀, de préférence en C₁-C₄, un radical arylalkyle dans lequel, plus spécialement, le radical aryle comprend 6 atomes de carbone et le radical alkyle 1 à 10 atomes de carbone, de préférence 1 à 4 atomes de carbone.

Selon un mode de réalisation avantageux de l'invention, les radicaux R₁ et R₂, identiques ou non, représentent un atome d'hydrogène ; un radical alkyle linéaire ou ramifié en C₁-C₆ un radical alkyle en C₂-C₆ substitué par un radical hydroxyle ; un radical alkyle en C₂-C₆ portant un groupement amino ou ammonium ; un radical chloroalkyle en C₂-C₆ ; un radical alkyle C₂-C₆ interrompu par un atome d'oxygène ou groupement en portant un (par exemple ester) ; un radical aromatique comme les phényle, benzyle, 4-méthylphényle ; un radical hétérocyclique tel que les radicaux pyrrolo, pyrrolidino, imidazolo, imidazolino, imidazolium, pipérazino, morpholo, morphollino, pyrazolo, triazolo, éventuellement substitué par au moins un radical alkyle en C₁-C₆ ou aromatique.

De préférence, les radicaux R₁ et R₂, identiques ou différents, représentent un atome d'hydrogène, un radical alkyle linéaire ou ramifié en C₁-C₆ tels que les radicaux méthyle, éthyle, n-butyle, n-propyle ; le 2-hydroxyéthyle ; un radical alkyltriméthylammonium ou alkyltriéthylammonium, le radical alkyle étant linéaire en C₂-C₆ ; un radical (di)alkylméthylamino ou (di)alkyléthylamino, le radical alkyle étant linéaire en C₂-C₆; -CH₂CH₂Cl ; -(CH₂)ₙ-OCH₃ ou -(CH₂)ₙ-OCH₂CH₃ avec n nombre entier variant de 2 à 6 ; -CH₂CH₂-OCOCH₃; -CH₂CH₂COOCH₃.

De préférence les radicaux R₁ et R₂, identiques ou non, et de préférence identiques, représentent un radical méthyle, un radical éthyle.

Les radicaux R₁ et R₂, identiques ou différents, peuvent aussi représenter un radical hétérocyclique du type pyrrolidino, 3-amino pyrrolidino, 3-(diméthyl)amino pyrrolidino, 3-(triméthyl)amino pyrrolidino, 2,5-diméthylpyrrolo, le 1H-imidazole, 4-méthyl pipérazino, 4-benzyl pipérazino, morpholo, 3,5-(ter-butyl)-1 H-pyrazolo, 1 H-pyrazolo, 1 H-1,2,4-triazolo.

Les radicaux R₁ et R₂, identiques ou différents, peuvent aussi représenter être reliés de manière à former un hétérocycle de formules (I) et (II) suivantes : 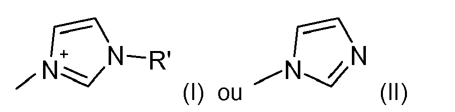 dans lesquelles R' représente un atome d'hydrogène, un radical alkyle en C₁-C₃, -CH₂CH₂OH, -CH₂CH₂OCH₃.

Conformément à un mode de réalisation plus particulier de l'invention, R₅, identiques ou non, représentent un atome d'hydrogène, un atome de fluor ou de chlore, un radical alkyle linéaire ou ramifié comprenant 1 à 4 atomes de carbone éventuellement interrompu par un atome d'oxygène ou d'azote.

Il est précisé que le substituant R₅, s'il est différent de l'hydrogène, se trouve avantageusement en position(s) 3 et/ou 5 par rapport au carbone du cycle portant l'azote substitué par les radicaux R₁ et R₂, et de préférence en position 3 par rapport à ce carbone.

Avantageusement, les radicaux R₅, identiques ou non, représentent un atome d'hydrogène ; un radical alkyle linéaire ou ramifié en C₁-C₄ ; -O-R₅₁ avec R₅₁ représentant un radical alkyle linéaire en C₁-C₄ ; -R₅₂-O-CH₃ avec R₅₂ représentant un radical alkyle linéaire en C₂-C₃ ; -R₅₃ - N (R₅₄)₂ dans laquelle R₅₃ représente un radical alkyle linéaire en C₂-C₃, R₅₄, identiques ou différents, représentent un atome d'hydrogène ou un radical méthyle.

De préférence R₅, identiques ou non, représentent l'hydrogène, un méthyle, un méthoxy, et de préférence, R₅ représente un atome d'hydrogène.

Selon un mode de réalisation particulier, les radicaux R₆, identiques ou différents, représentent un atome d'hydrogène ; un radical alkyle linéaire ou ramifié en C₁-C₄ ; -X avec X représentant un atome de chlore, de brome ou de fluor ; -R₆₁-O-R₆₂ avec R₆₁ représentant un radical alkyle linéaire en C₂-C₃ et R₆₂ représente le radical méthyle ; -R₆₃-N(R₆₄)₂ avec R₆₃ représentant un radical alkyle linéaire en C₂-C₃, R₆₄, identiques ou différents, représentent un atome d'hydrogène ou un radical méthyle, -N(R₆₅)₂ dans laquelle R₆₅, identiques ou différents, représentent un atome d'hydrogène, un radical alkyle linéaire en C₂-C₃ ; -NHCO R₆₆ avec R₆₆ représentant un radical alkyle en C₁-C₂, un radical chloroalkyle en C₁-C₂, un radical -R₆₇-NH₂ ou -R₆₇-NH(CH₃) ou -R6₇-N(CH₃)₂ ou -R₆₇-N⁺(CH₃)₃ ou -R₆₇-N⁺(CH₂CH₃)₃ avec R₆₇ représentant un radical alkyle en C₁-C₂.

II est précisé que le substituant R₆, s'il est différent de l'hydrogène, se trouve de préférence en position 2 et/ou 4 par rapport à l'atome d'azote du cycle pyridinium, et de préférence en position 4 par rapport à cet atome d'azote.

Plus particulièrement ces radicaux R₆, identiques ou non, représentent un atome d'hydrogène ou un radical méthyle ou éthyle, et de préférence, R₆ représente un atome d'hydrogène.

En ce qui concerne les radicaux R₃, R₄, ces derniers, identiques ou non, représentent avantageusement un atome d'hydrogène, un radical alkyle comprenant 1 à 4 atomes de carbone, plus spécialement un radical méthyle. De manière préférée, R₃ et R₄ représentent chacun un atome d'hydrogène.

Comme indiqué plus haut, X représente :
- un radical alkyle, linéaire ou ramifié comprenant 1 à 14 atomes de carbone, ou alcényle comprenant 2 à 14 atomes de carbone, éventuellement interrompu et/ou substitué par au moins un hétéroatome, par au moins un groupement porteur d'au moins un hétéroatome et/ou par au moins un atome d'halogène ;
- un radical hétérocyclique comprenant 5 ou 6 chaînons, éventuellement substitué par au moins un radical alkyle linéaire ou ramifié comprenant 1 à 14 atomes de carbone, par au moins un radical aminoalkyle, linéaire ou ramifié, comprenant 1 à 4 atomes de carbone, éventuellement substitué par au moins un hétéroatome ; par au moins un atome d'halogène ;
- un radical aromatique ou diaromatique condensé ou non, séparé ou non par un radical alkyle comprenant 1 à 4 atomes de carbone, le ou les radicaux aryles étant éventuellement substitués par au moins un atome d'halogène ou par au moins un radical alkyle comprenant 1 à 10 atomes de carbone éventuellement substitué et/ou interrompu par au moins un hétéroatome et/ou groupement comprenant au moins un hétéroatome ;
- un radical dicarbonyle.

En outre, il est indiqué que le groupement X peut porter une ou plusieurs charges cationiques.

Ainsi, X peut représenter un radical alkyle, linéaire ou ramifié, comprenant 1 à 14 atomes de carbone, ou alcényle comprenant 2 à 14 atomes de carbone, et peut être substitué et/ou interrompu par un ou plusieurs atomes d'oxygène et/ou d'azote, et/ou par un ou plusieurs groupements porteurs d'au moins un hétéroatome , et/ou par un atome de fluor, de chlore.

Parmi les groupements de ce type, on peut citer tout particulièrement les groupements hydroxyle, alcoxy (avec notamment un radical R de type alkyle en C₁-C₄), amino, ammonium, amido, carbonyle, carboxyle (-COO-, -O-CO-) avec notamment un radical de type alkyloxy.

Notons que l'atome d'azote, s'il est présent, peut se trouver sous une forme quaternisée ou non. Dans ce cas, le ou les deux autres radicaux portés par l'atome d'azote quaternisé ou non, sont identiques ou non et peuvent être un atome d'hydrogène, un radical alkyle en C₁-C₄, de préférence le méthyle.

Selon une autre variante, le groupement X représente un radical hétérocyclique comprenant 5 ou 6 chaînons, du type imidazolo, pyrazolo, triazino, pyridino, éventuellement substitué par au moins un radical alkyle linéaire ou ramifié comprenant 1 à 14 atomes de carbone, plus particulièrement 1 à 10 atomes de carbone, de préférence de 1 à 4 atomes de carbone ; par au moins un radical aminoalkyle, linéaire ou ramifié, comprenant 1 à 10 atomes de carbone, de préférence de 1 à 4 atomes de carbone, éventuellement substitué par un groupement comprenant au moins un hétéroatome (de préférence un radical hydroxyle), ou par un atome d'halogène. A noter que le groupement amino est de préférence lié à l'hétérocycle.

Conformément à une autre possibilité, le groupement X représente un radical aromatique (comprenant de préférence 6 atomes de carbone) ou diaromatique condensé ou non (comprenant notamment de 10 à 12 atomes de carbone), séparé ou non par un radical alkyle comprenant 1 à 4 atomes de carbone, le ou les radicaux aryles étant éventuellement substitués par au moins au moins un atome d'halogène et/ou par au moins un radical alkyle comprenant 1 à 10 atomes de carbone, de préférence 1 à 4 atomes de carbone, éventuellement interrompu par au moins un atome d'oxygène et/ou d'azote, et/ou groupement comprenant au moins un hétéroatome (comme un radical carbonyle, carboxyle, amido, amino, ammonium).

Il est à noter que le radical aromatique, de préférence un radical phényle, est relié aux groupements CR₃R₄ par l'intermédiaire de liaisons en positions 1,2 ; 1,3 ou 1,4, de préférence en positions 1,3 et 1,4. Si le radical phényle relié par l'intermédiaire de liaisons en positions 1,4, porte un ou deux substituants, ce ou ces derniers sont situés de préférence en position 1,4 par rapport à l'un des groupements CR₃R₄. Si le radical phényle relié par l'intermédiaire de liaisons en positions 1,3, porte un ou deux substituants, ce ou ces derniers sont situés de préférence en position 1 et/ou 3 par rapport à l'un des groupements CR₃R₄.

Au cas où le radical est diaromatique, il est de préférence non condensé et comprend deux radicaux phényles séparés ou non par une liaison simple (soit un carbone de chacun des deux cycles) ou par un radical alkyle, de préférence de type CH₂ ou C(CH₃)₂. De manière préférée, les radicaux aromatiques ne portent pas de substituant.

II est à noter que ledit radical diaromatique est relié aux groupements CR₃R₄ par l'intermédiaire de liaisons en positions 4,4'.

A titre d'exemples de groupements X convenables, on peut citer notamment les radicaux alkyle linéaires ou ramifiés comprenant 1 à 13 atomes de carbone tels que méthylène, éthylène, propylène, isopropylène, n-butylène, pentylène, hexylène ; le 2-hydroxypropylène, le 2-hydroxy n-butylène ; les radicaux alkylènes en C₁-C₁₃, substitués ou interrompus par un ou plusieurs atomes d'azote et/ou d'oxygène, et/ou groupements portant au moins un hétéroatome (hyroxyle, amino, ammonium, carbonyle, carboxyle, par exemple) tels que -CH₂CH₂OCH₂CH₂-, le 1,6-didéoxy-d-mannitol, -CH₂N⁺(CH₃)₂CH₂-, -CH₂CH₂N⁺(CH₃)₂-(CH₂)₆N⁺(CH₃)₂-CH₂CH₂-, CO-CO-, le 3,3-diméthylpentylène, le 2-acétoxyéthylène, le butylène1,2,3,4 tétraol ; -CH=CH- ; les radicaux aromatiques ou diaromatiques substitués par un ou plusieurs radicaux alkyle, par un ou plusieurs groupements portant au moins un hétéroatome et/ou par un ou plusieurs atomes d'halogène, tels que le 1,4-phénylène, le 1,3-phénylène, le 1,2-phénylène, le 2,6-fluorobenzène, le 4,4'-biphénylène, le 1,3-(5-méthyl benzène), le 1,2-bis(2-méthoxy)benzène, le bis(4-phényl)méthane, le 3,4 benzoate de méthyle, le 1,4-bis(amido méthyl)phényle; les radicaux de type hétérocycliques comme la pyridine, ou dérivé tel que le 2,6-bispyridine, l'imidazole, l'imidazolium, la triazine.

X représente, selon un mode de réalisation plus particulier de l'invention, un radical alkyle linéaire ou ramifié en C₁-C₁₃ ; -CH₂CH(OH)CH₂- ; -CH₂CH(Cl)CH₂- ; -CH₂CH₂-OCOCH₂- ; -CH₂CH₂COOCH₂- ; -Ra-O- Rb- avec Ra représentant un radical alkyle linéaire en C₂-C₆ et Rb représente un radical alkyle linéaire en C₁-C₂ ; - Rc-N(Rd)-Re- avec Rc représentant un radical alkyle en C₂-C₉, Rd représentant un atome d'hydrogène, un radical alkyle en C₁-C₂ et Re représentant un radical alkyle en C₁-C₆ ; -Rf-N⁺(Rg)₂-Rh- avec Rf représentant un radical alkyle linéaire en C₂-C₉, Rg, de préférence identiques, représentent un radical alkyle en C₁-C₂, Rh représente un radical alkyle linéaire en C₁-C₆ ; -CO-CO-.

X peut de plus représenter un radical imidazole, éventuellement substitué par au moins un radical alkyle comprenant 1 à 14 atomes de carbone, plus particulièrement 1 à 10 atomes de carbone, de préférence de 1 à 4, et par exemple les radicaux divalents de formule suivante : 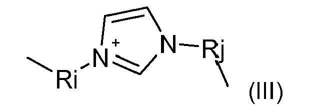 dans laquelle Ri et Rj, identiques ou non, représentent un radical alkyle linéaire en C₁-C₆; X peut de même être choisi parmi les radicaux divalents dérivés de triazine suivants :

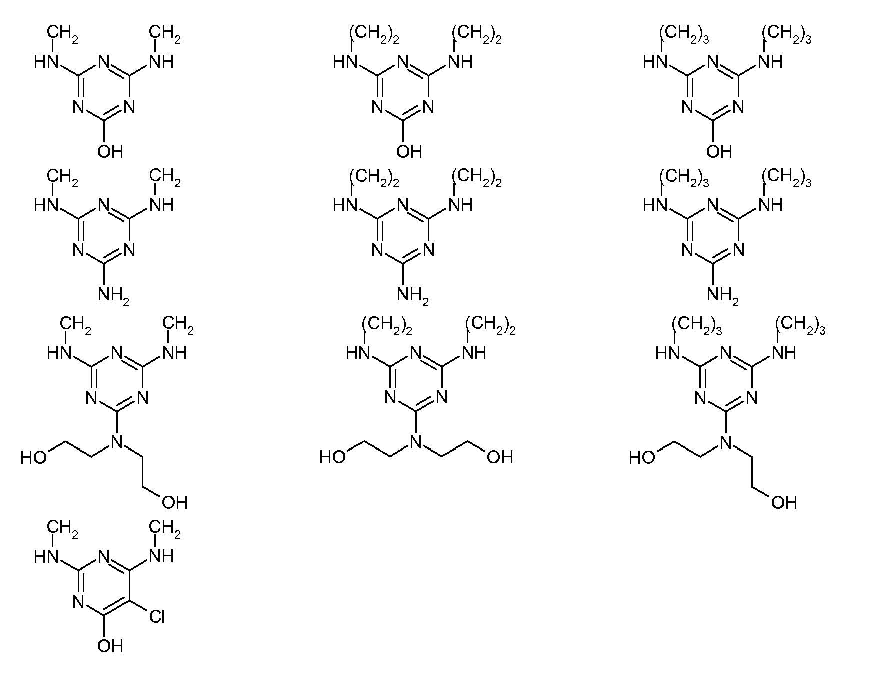

Selon une autre possibilité, X peut représenter les radicaux divalents aromatiques suivants :

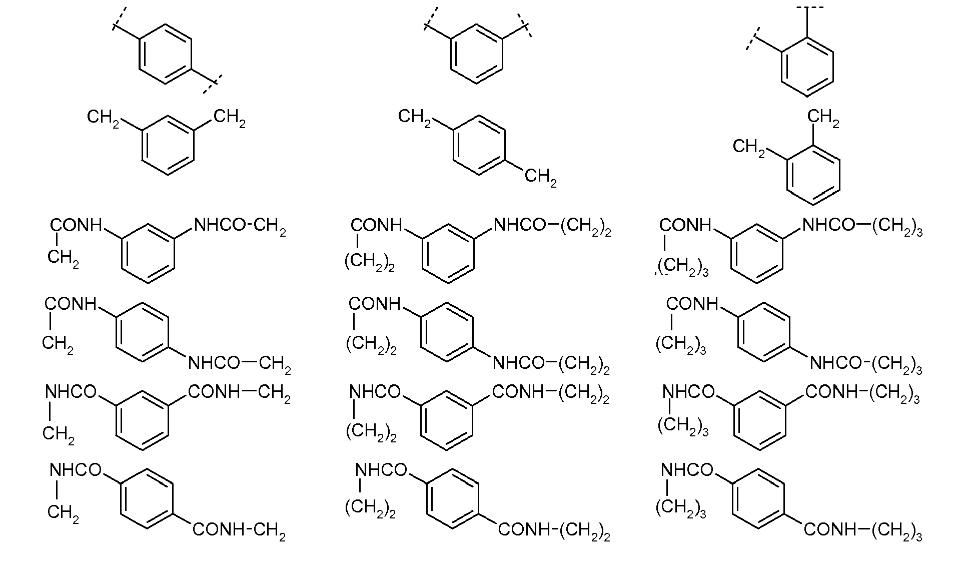

Dans la formule générale de ces composés fluorescents, Y- représente un anion organique ou minéral. S'il y a plusieurs anions Y-, ces derniers peuvent ou non être identiques.

Parmi les anions d'origine minérale, on peut citer sans intention de s'y limiter les anions provenant d'atomes d'halogène, tels que les chlorures de préférence, les iodures, les sulfates ou bisulfates, les nitrates, les phosphates, les hydrogénophosphates, les dihydrogénophosphates, les carbonate, les bicarbonates.

Parmi les anions d'origine organique, on peut citer les anions provenant des sels d'acides mono- ou polycarboxyliques, sulfoniques, sulfuriques, saturés ou non, aromatiques ou non, éventuellement substitués par au moins un radical hydroxyle, amino, ou atomes d'halogène. A titre d'exemples non limitatifs, conviennent les acétates, hydroxyactétates, aminoacétates, (tri)chloroacétates, benzoxyactétates, propionates et dérivés portant un atome de chlore, fumarates, oxalates, acrylates, malonates, succinates, lactates, tartrates, glycollates citrates, les benzoates et dérivés portant un radical méthyle ou amino, les alkylsulfates, les tosylates, les benzènesulfonates, toluènesulfonates, etc.

De préférence, le ou les anions Y, identiques ou non, sont choisis parmi le chlore, le sulfate, le méthosulfate, l'éthosulfate.

Enfin, le nombre n, entier, est au moins égal à 2 et au plus égal au nombre de charges cationiques présentes dans le composé fluorescent.

De préférence les composés fluorescents qui viennent d'être détaillés sont des composés symétriques.

Ces composés peuvent être synthétisés en mettant en faisant réagir dans une première étape de α-picoline avec un réactif comprenant deux groupes partant qui peuvent être choisis parmi les atomes d'halogène, de préférence le brome, éventuellement le chlore, ou les groupements de type tolylsulfonyle ou méthylesulfonyle.

Cette première étape peut avoir lieu en présence d'un solvant, bien qu'il ne soit pas obligatoire, comme par exemple le diméthylformamide.

Le nombre de moles d'α-picoline est en général voisin de 2 pour une mole de réactif comprenant les groupes partant.

En outre, la réaction est habituellement mise en oeuvre au reflux du réactif et/ou du solvant s'il est présent.

Le produit issu de cette première étape est ensuite contacté avec un aldéhyde correspondant de formule suivante : 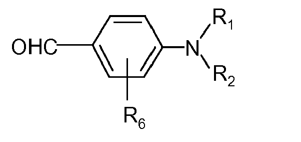 dans laquelle R₁, R₂ et R₆ ont les mêmes significations que précédemment indiquées.

Là encore, la réaction peut être effectuée en présence d'un solvant approprié, de préférence au reflux.

Il est à noter que les radicaux R₁ et R₂ de l'aldéhyde peuvent avoir la signification indiquée dans la formule générale détaillée auparavant.

Il est aussi possible de mettre en oeuvre un aldéhyde pour lequel lesdits radicaux représentent des atomes d'hydrogène et effectuer conformément à des méthodes classiques, la substitution de ces atomes d'hydrogène par des radicaux appropriés tels que décrits dans la formule générale une fois la deuxième étape terminée.

On pourra notamment se référer à des synthèses telles que décrites dans US 4256458.

On peut également citer les composés suivants :
- Les derives de thioxanthènes tels que 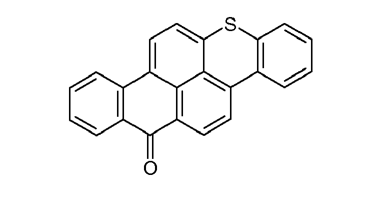 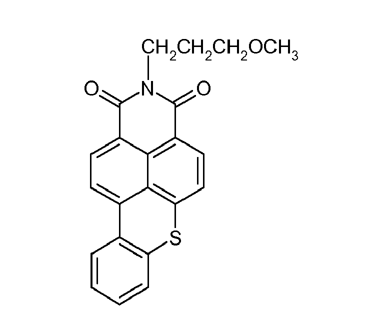 Samaron, Brilliant yellow H6GL, C.I. 56235 Disperse yellow 105
- Les rhodamines telles que 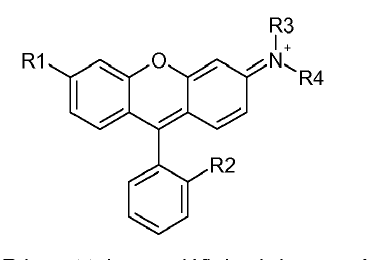 dans laquelle R1, R2, R3 et R4 sont tels que définis ci-dessus. A titre d'exemple, on peut citer 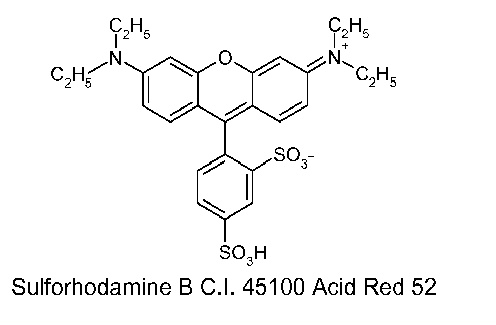
- Les Dimères de Stilbazolium de formules : 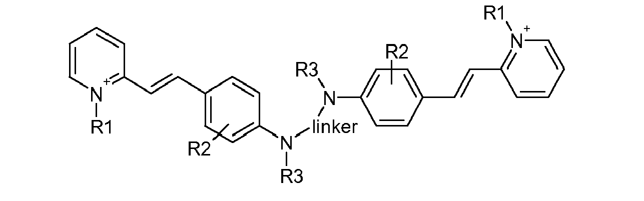 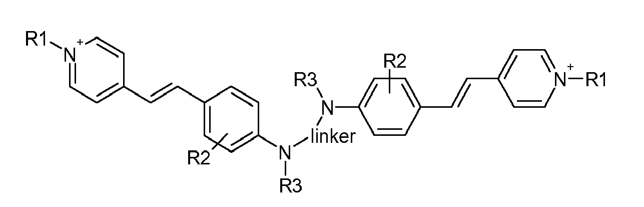 dans lesquelles :
   R1, R2, R3 indépendamment les uns des autres représentent un atome d'hydrogène ; un atome d'halogène ; un groupement aryle en C6-C30 ; un groupement hydroxy ; un groupement cyano ; un groupement nitro ; un groupement sulfo ; un groupement amino ; un groupement acylamino ; un groupement dialkyl(C1-C6)amino ; un groupement dihydroxyalkyl(C1-C6)amino ; un groupement alkyl(C1-C6)hydroxyalkyl(C1-C6)amino ; un groupement alcoxy(C1-C6) ; un groupement alcoxy(C1-C6)carbonyle ; un groupement carboxyalcoxy en C1-C6; un groupement pipéridinosulfonyle; un groupement pyrrolidino ; un groupement alkyle(C1-C6)halogénoalkyle(C1-C6)amino ; un groupement benzoylalkyle(C1-C6) ; un groupement vinyle ; un groupement formyle ; un radical aryle en C6-C30 éventuellement substitué par un ou plusieurs groupements choisis parmi les groupements hydroxyle, alcoxy en C1-C6 linéaire, ramifié ou cyclique, alkyle linéaire, ramifié ou cyclique comprenant de 1 à 22 atomes de carbone lui-même étant éventuellement substitué par un ou plusieurs groupements hydroxyle,amino, alcoxy C1-C6 ; un radical alkyle linéaire, ramifié ou cyclique, comprenant de 1 à 22 atomes de carbone, plus particulièrement de 1 à 6 atomes de carbone, éventuellement substitué par un ou plusieurs groupements choisis parmi les groupements hydroxyle, amino, alcoxy en C1-C6 linéaire, ramifié ou cyclique, aryle éventuellement substitué, carboxyle, sulfo, un atome d'halogène,ce radical alkyle pouvant être interrompu par un hétéroatome, tel que l'azote, le soufre et l'oxygène ;
   Linker représente une chaîne hydrocarbonée en C1-C12, aliphatique ou alicyclique, saturée ou insaturée, un ou plusieurs atomes de carbone de la chaîne hydrocarbonée pouvant être remplacés par un ou plusieurs atomes d'oxygène, un ou plusieurs groupements NR avec R étant un atome d'hydrogène ou un radical alkyle, la chaîne hydrocarbonée ne comportant pas de groupement diazo, nitro, nitroso ou peroxyde, et la chaîne hydrocarbonée ne pouvant pas être terminée à l'une ou l'autre de ses extrémités par un hétéroatome ; et
   X⁻ représente un contre-ion anionique tel que défini précédemment ;
- Les Trimères et tétramères méthiniques suivants 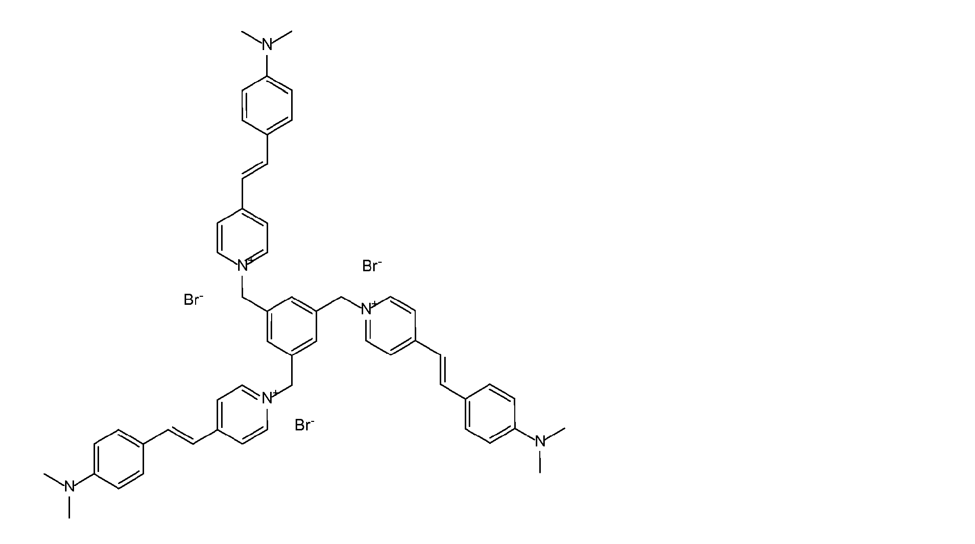 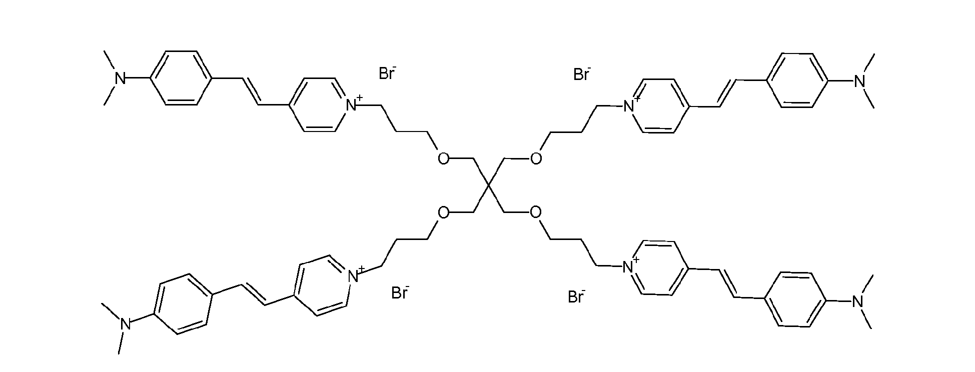
- Les dérivés de xanthènes tels que : 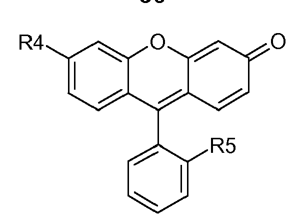
avec R4 et R5 indépendamment les uns des autres représentent un atome d'hydrogène ; un atome d'halogène ; un groupement aryle en C6-C30 ; un groupement hydroxy ; un groupement cyano ; un groupement nitro ; un groupement sulfo ; un groupement amino ; un groupement acylamino ; un groupement dialkyl(C1-C6)amino ; un groupement dihydroxyalkyl(C1-C6)amino ; un groupement alkyl(C1-C6)hydroxyalkyl(C1-C6)amino ; un groupement alcoxy(C1-C6) ; un groupement alcoxy(C1-C6)carbonyle ; un groupement carboxyalcoxy en C1-C6; un groupement pipéridinosulfonyle ; un groupement pyrrolidino ; un groupement alkyle(C1-C6)halogénoalkyle(C1-C6)amino ; un groupement benzoylalkyle(C1-C6) ; un groupement vinyle ; un groupement formyle ; un radical aryle en C6-C30 éventuellement substitué par un ou plusieurs groupements choisis parmi les groupements hydroxyle, alcoxy en C1-C6 linéaire, ramifié ou cyclique, alkyle linéaire, ramifié ou cyclique comprenant de 1 à 22 atomes de carbone lui-même étant éventuellement substitué par un ou plusieurs groupements hydroxyle,amino, alcoxy C1-C6 ; un radical alkyle linéaire, ramifié ou cyclique, comprenant de 1 à 22 atomes de carbone, plus particulièrement de 1 à 6 atomes de carbone, éventuellement substitué par un ou plusieurs groupements choisis parmi les groupements hydroxyle, amino, alcoxy en C1-C6 linéaire, ramifié ou cyclique, , aryle éventuellement substitué, carboxyle, sulfo, un atome d'halogène,ce radical alkyle pouvant être interrompu par un hétéroatome, tel que l'azote, le soufre et l'oxygène.

La composition de l'invention peut contenir en outre au moins un azurant optique. Les azurants optiques utiles dans la présente invention aussi connus sous le nom de "brighteners", ou "fluorescent brighteners", ou "fluorescent brightening agents" ou "FWA", ou "fluorescent whitening agents", ou "whiteners", ou encore "fluorescent whiteners" en terminologie anglo-saxone, sont des composés incolores car il n'absorbent pas dans la lumière visible, mais uniquement dans les Ultra Violets (longueurs d'onde allant de 200 à 400 nanomètres), et transforment l'énergie absorbée en lumière fluorescente de plus grande longueur d'onde émise dans la partie visible du spectre ; en général dans le bleu et/ou le vert, c'est-à-dire dans des longueurs d'onde allant de 400 à 550 nanomètres.

Les azurants optiques sont connus par l'homme du métier. Ils sont par exemple décrits dans Ullmann's Encyclopedia of Industrial Chemistry (2002) "Optical Brighteners" et Kirk-Othmer Encyclopedia of Chemical Technology (1995); "Fluorescent Whitening Agents".

L'azurant optique entrant éventuellement dans la composition mise en oeuvre dans l'invention, est choisi parmi les composés qui absorbent la lumière dans la partie ultraviolette du spectre, essentiellement dans l'UVA, à une longueur d'onde comprise entre 300 et 390 nm. Ces composés ré-émettent une lumière fluorescente dans le spectre du visible, comprise entre 400 et 525 nm.

Parmi les azurants optiques, conviennent tout spécialement les dérivés du stilbène, les dérivés coumariniques, les dérivés oxazole et benzoxazole, les dérivés imidazole.

A titre d'exemples, on peut citer notamment :
- le dérivé stilbénique de naphto-triazole (Tinopal GS de Ciba), le di-styryl-4,4' biphényle sulfonate di-sodique (nom CTFA : disodium distyrylbiphenyl disulfonate ; Tinopal CBS-X de Ciba = 4,4'-bis[(4,6-diamilino-1,3,5-triazin-2-yl)amino]stilbène-2,2'-disulfonate de sodium), le dérivé cationique d'aminocoumarine (Tinopal SWN Conc. de Ciba), le diéthylaminométhyl coumarine, le 4-méthyl 7-diéthyl coumarine, le 4,4'-bis[(4,6-dianilino-1,3,5-triazin-2-yl)amino]stilbène-2,2'-disulfonate de sodium (Tinopal SOP de Ciba), le 4,4'-bis-[(4-anilino-6-bis(2-hydroxyéthyl)amino-1,3,5-triazin-2-yl)amino]stilbène-2,2'-disulfonique acide (Tinopal UNPA-GX de Ciba), le 4,4'-bis-[anilino-6-morpholine-1,3,5-triazin-2-yl)amino] stilbène (Tinopal AMS-GX de Ciba), le 4,4'-bis-[(4-anilino-6-(2-hydroxy éthyl) méthylamino-1,3,5-triazin-2-yl)amino]stilbène-2,2'-disodium sulfonate (Tinopal 5BM-GX de Ciba),
- le 2,5 thiophène di-yl bis(5 ter-butyl-1,3 benzoxazole) (Uvitex OB de Ciba),
- le dérivé anionique du di-aminostilbène (dispersion dans l'eau, Leucophor BSB liquide de Clariant,
- les laques d'azurant optique (gamme Covazur de Wackherr).

A titre de colorants fluorescents et lorsqu'ils sont présent dans la composition d'azurants optiques utiles dans la présente invention on peut aussi citer :
- Les naphthalimides de formule :, 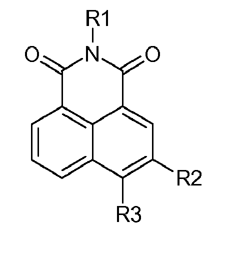 R1, R2, R3, indépendamment les uns des autres représentent un atome d'hydrogène ; un atome d'halogène ; un groupement aryle en C6-C30 ; un groupement hydroxy ; un groupement cyano ; un groupement nitro ; un groupement sulfo ; un groupement amino ; un groupement acylamino ; un groupement dialkyl(C1-C6)amino ; un groupement dihydroxyalkyl(C1-C6)amino ; un groupement alkyl(C1-C6)hydroxyalkyl(C1-C6)amino ; un groupement alcoxy(C1-C6) ; un groupement alcoxy(C1-C6)carbonyle ; un groupement carboxyalcoxy en C1-C6; un groupement pipéridinosulfonyle; un groupement pyrrolidino ; un groupement alkyle(C1-C6)halogénoalkyle(C1-C6)amino ; un groupement benzoylalkyle(C1-C6) ; un groupement vinyle ; un groupement formyle ; un radical aryle en C6-C30 éventuellement substitué par un ou plusieurs groupements choisis parmi les groupements hydroxyle, alcoxy en C1-C6 linéaire, ramifié ou cyclique, alkyle linéaire, ramifié ou cyclique comprenant de 1 à 22 atomes de carbone lui-même étant éventuellement substitué par un ou plusieurs groupements hydroxyle,amino, alcoxy C1-C6 ; un radical alkyle linéaire, ramifié ou cyclique, comprenant de 1 à 22 atomes de carbone, plus particulièrement de 1 à 6 atomes de carbone, éventuellement substitué par un ou plusieurs groupements choisis parmi les groupements hydroxyle, amino, alcoxy en C1-C6 linéaire, ramifié ou cyclique, , aryle éventuellement substitué, carboxyle, sulfo, un atome d'halogène,ce radical alkyle pouvant être interrompu par un hétéroatome, tel que N, S ou O ; les substituants R1 , R2, R3 peuvent former avec les atomes de carbone auxquels ils sont rattachés un cycle aromatique ou non, en C6-C30 ou hétérocyclique comportant au total de 5 à 30 chaînons et de 1 à 5 hétéroatomes ; ces cycles étant condensés ou non, insérant ou non un groupement carbonyle, et étant substitués ou non par un ou plusieurs groupements choisis parmi les groupements alkyle en C1-C4, alcoxy(C1-C4)alkyl(C1-C4), amino, dialkyl(C1-C4)amino, halogène, phényle, carboxy, trialkyl(C1-C4)ammonioalkyl(C1-C4).
   On peut citer par exemple le composé suivant 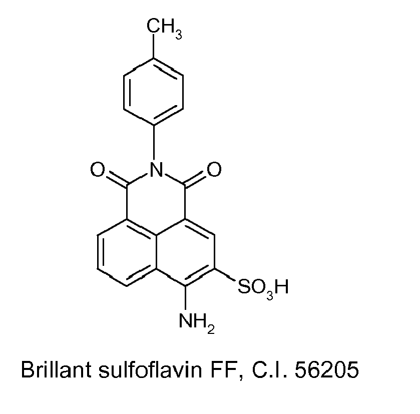
- Les DICETOPYRROLOPYRROLE de formule : 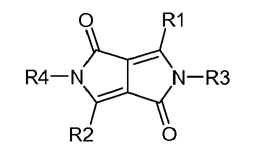 dans laquelle R1, R2, R3 et R4 indépendamment les uns des autres représentent un atome d'hydrogène ; un atome d'halogène ; un groupement aryle en C6-C30 ; un groupement hydroxy ; un groupement cyano ; un groupement nitro ; un groupement sulfo ; un groupement amino ; un groupement acylamino ; un groupement dialkyl(C1-C6)amino ; un groupement dihydroxyalkyl(C1-C6)amino ; un groupement alkyl(C1-C6)hydroxyalkyl(C1-C6)amino ; un groupement alcoxy(C1-C6) ; un groupement alcoxy(C1-C6)carbonyle ; un groupement carboxyalcoxy en C1-C6 ; un groupement pipéridinosulfonyle ; un groupement pyrrolidino ; un groupement alkyle(C1-C6)halogénoalkyle(C1-C6)amino ; un groupement benzoylalkyle(C1-C6) ; un groupement vinyle ; un groupement formyle ; un radical aryle en C6-C30 éventuellement substitué par un ou plusieurs groupements choisis parmi les groupements hydroxyle, alcoxy en C1-C6 linéaire, ramifié ou cyclique, alkyle linéaire, ramifié ou cyclique comprenant de 1 à 22 atomes de carbone lui-même étant éventuellement substitué par un ou plusieurs groupements hydroxyle, amino, alcoxy C1-C6 ; un radical alkyle linéaire, ramifié ou cyclique, comprenant de 1 à 22 atomes de carbone, plus particulièrement de 1 à 6 atomes de carbone, éventuellement substitué par un ou plusieurs groupements choisis parmi les groupements hydroxyle, amino, alcoxy en C1-C6 linéaire, ramifié ou cyclique, , aryle éventuellement substitué, carboxyle, sulfo, un atome d'halogène,ce radical alkyle pouvant être interrompu par un hétéroatome, tel que l'azote, le soufre et l'oxygène.
   On peut citer par exemple le composé suivant 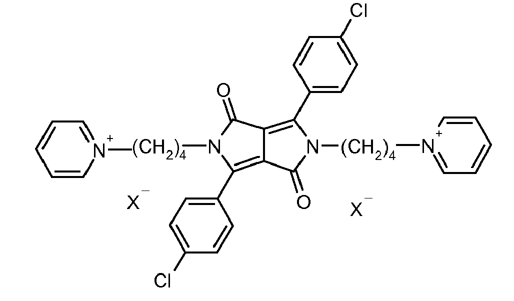 dans lequel X- est un anion défini comme précédemment.
- Les dérivés de DICYANO PYRAZINES de formules : 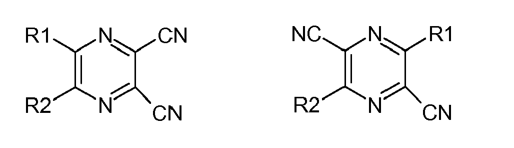 dans lesquelles R1, R2 indépendamment les uns des autres représentent un atome d'hydrogène ; un atome d'halogène ; un groupement aryle en C6-C30 ; un groupement hydroxy ; un groupement cyano ; un groupement nitro ; un groupement sulfo ; un groupement amino ; un groupement acylamino ; un groupement dialkyl(C1-C6)amino ; un groupement dihydroxyalkyl(C1-C6)amino ; un groupement alkyl(C1-C6)hydroxyalkyl(C1-C6)amino ; un groupement alcoxy(C1-C6) ; un groupement alcoxy(C1-C6)carbonyle ; un groupement carboxyalcoxy en C1-C6; un groupement pipéridinosulfonyle; un groupement pyrrolidino ; un groupement alkyle(C1-C6)halogénoalkyle(C1-C6)amino ; un groupement benzoylalkyle(C1-C6) ; un groupement vinyle ; un groupement formyle ; un radical aryle en C6-C30 éventuellement substitué par un ou plusieurs groupements choisis parmi les groupements hydroxyle, alcoxy en C1-C6 linéaire, ramifié ou cyclique, alkyle linéaire, ramifié ou cyclique comprenant de 1 à 22 atomes de carbone lui-même étant éventuellement substitué par un ou plusieurs groupements hydroxyle,amino, alcoxy C1-C6 ; un radical alkyle linéaire, ramifié ou cyclique, comprenant de 1 à 22 atomes de carbone, plus particulièrement de 1 à 6 atomes de carbone, éventuellement substitué par un ou plusieurs groupements choisis parmi les groupements hydroxyle, amino, alcoxy en C1-C6 linéaire, ramifié ou cyclique, , aryle éventuellement substitué, carboxyle, sulfo, un atome d'halogène,ce radical alkyle pouvant être interrompu par un hétéroatome, tel que l'azote, le soufre et l'oxygène ;
   deux des substituants R1 et R2, peuvent former avec les atomes de carbone auxquels ils sont rattachés un cycle aromatique ou non, en C6-C30 ou hétérocyclique comportant au total de 5 à 30 chaînons et de 1 à 5 hétéroatomes ; ces cycles étant condensés ou non,insérant ou non un groupement carbonyle, et étant substitués ou non par un ou plusieurs groupements choisis parmi les groupements alkyle en C1-C4, alcoxy(C1-C4)alkyl(C1-C4), amino, dialkyl(C1-C4)amino, halogène, phényle, carboxy, trialkyl(C1-C4)ammonioalkyl(C1-C4) ;
   deux des substituants R2 , R3 peuvent former avec les atomes de carbone auxquels ils sont rattachés un noyau aromatique en C6-C30 ou hétérocyclique comportant au total de 5 à 30 chaînons et de 1 à 5 hétéroatomes ; ce cycle étant condensés ou non,ce cycle et l'éventuel cycle condensé étant substitués ou non par au moins un groupement choisi parmi les groupements alkyle en C1-C4, alcoxy(C1-C4)alkyl(C1-C4), amino, dialkyl(C1-C4)amino, halogène, phényle, carboxy, trialkyl(C1-C4)ammonioalkyl(C1-C4).
- Les dérivés de coumarines tels que les composés correspondants aux formules suivantes 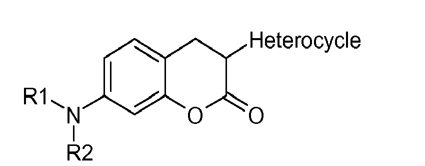 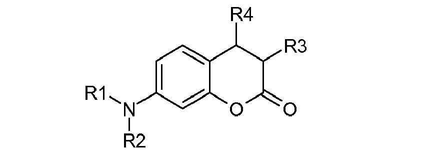 dans laquelle l'hétérocycle est choisi parmi les furane, tiophène, 2H-pyrrole, 2-pyrroline, pyrrolidine, 1,3-dioxolane, oxazole, thiazole, Imidazole, 2-imidazoline, Imidazoline, pyrazole, 2-pyrazoline, pyrazolidine, isoxazole, isothiazole, 1,2,3-oxadiazole, 1,2,3-triazole, 1,3,4-thiadiazole, 2H-pyran, 4H-pyran, pyridine, piperidine, 1,4-dioxane, morpholine, 1,4-dithiane, thiomorpholine, pyradizine, pyrimidine, pyrazine, piperazine, 1,3,5-Triazine, 1,3,5-trithiane, indolizine, indole, isoindole, 3H-indole, indoline, benzo[b]furan, benzo[b]thiophene, 1H-indazole, benzimidazole, benzothiazole, purine, 4H-quinolizine, quinoline, isoquinoline, cinnoline, phtalazine, quinazoline, quinoxaline, 1,8-naphtyridine, pteridine, quinuclidine, carbazole, acridine, phenazine, phenothiazine, phenoxazine, indene, naphtalene, azulene, fluorene, anthracene, norbornane, adamantane, et ;
   R1, R2, R3, R4 indépendamment les uns des autres représentent un atome d'hydrogène ; un atome d'halogène ; un groupement aryle en C6-C30 ; un groupement hydroxy ; un groupement cyano ; un groupement nitro ; un groupement sulfo ; un groupement amino ; un groupement acylamino ; un groupement dialkyl(C1-C6)amino ; un groupement dihydroxyalkyl(C1-C6)amino ; un groupement alkyl(C1-C6)hydroxyalkyl(C1-C6)amino ; un groupement alcoxy(C1-C6) ; un groupement alcoxy(C1-C6)carbonyle ; un groupement carboxyalcoxy en C1-C6; un groupement pipéridinosulfonyle; un groupement pyrrolidino ; un groupement alkyle(C1-C6)halogénoalkyle(C1-C6)amino ; un groupement benzoylalkyle(C1-C6) ; un groupement vinyle ; un groupement formyle ; un radical aryle en C6-C30 éventuellement substitué par un ou plusieurs groupements choisis parmi les groupements hydroxyle, alcoxy en C1-C6 linéaire, ramifié ou cyclique, alkyle linéaire, ramifié ou cyclique comprenant de 1 à 22 atomes de carbone lui-même étant éventuellement substitué par un ou plusieurs groupements hydroxyle,amino, alcoxy C1-C6 ; un radical alkyle linéaire, ramifié ou cyclique, comprenant de 1 à 22 atomes de carbone, plus particulièrement de 1 à 6 atomes de carbone, éventuellement substitué par un ou plusieurs groupements choisis parmi les groupements hydroxyle, amino, alcoxy en C1-C6 linéaire, ramifié ou cyclique, , aryle éventuellement substitué, carboxyle, sulfo, un atome d'halogène, ce radical alkyle pouvant être interrompu par un hétéroatome, tel qu'un atome d'azote, de soufre ou d'oxygène ; les substituants R1, R2, R3 et R4 peuvent former avec les atomes de carbone auxquels ils sont rattachés un cycle aromatique ou non, en C6-C30 ou hétérocyclique comportant au total de 5 à 30 chaînons et de 1 à 5 hétéroatomes ; ces cycles étant condensés ou non, insérant ou non un groupement carbonyle, et étant substitués ou non par un ou plusieurs groupements choisis parmi les groupements alkyle en C1-C4, alcoxy(C1-C4)alkyl(C1-C4), amino, dialkyl(C1-C4)amino, halogène, phényle, carboxy, trialkyl(C1-C4)ammonioalkyl(C1-C4) ; deux des substituants R3 , R4 peuvent former avec les atomes de carbone auxquels ils sont rattachés un noyau aromatique en C6-C30 ou hétérocyclique comportant au total de 5 à 30 chaînons et de 1 à 5 hétéroatomes ; ce cycle étant condensés ou non,ce cycle et l'éventuel cycle condensé étant substitués ou non par au moins un groupement choisi parmi les groupements alkyle en C1-C4, alcoxy(C1-C4)alkyl(C1-C4), amino, dialkyl(C1-C4)amino, halogène, phényle, carboxy, trialkyl(C1-C4)ammonioalkyl(C1-C4).
   A titre d'exemple, on peut citer 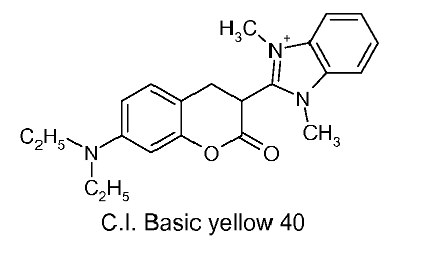
- Les dérivés de naphtolactames tels que : 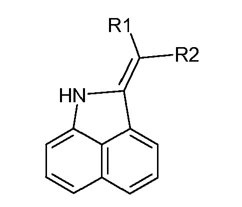 avec R1 et R2 définis comme précédemment
   On peut citer par exemple le composé suivant 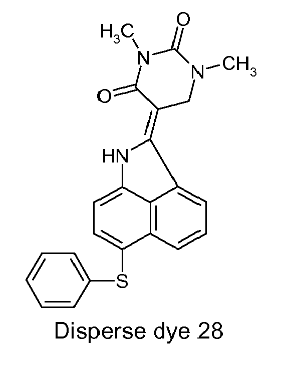
- Les dérivés AZALACTONES : 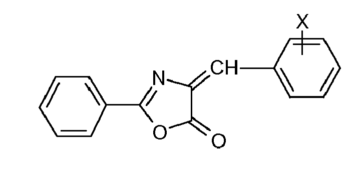 X ayant la même définition que R1 décrit précédemment.
   On peut citer par exemple le composé suivant 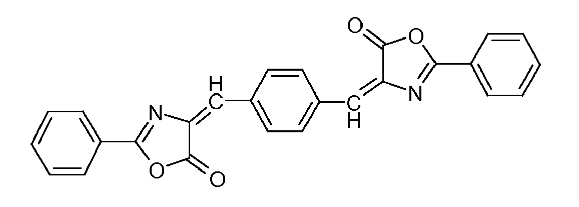
- Les dérivés méthiniques tels que 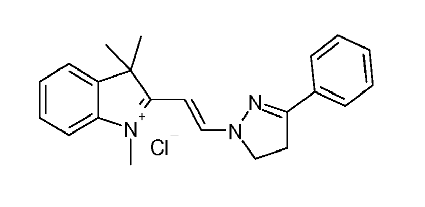
- Les dérivés oxazines et thiazines tels que 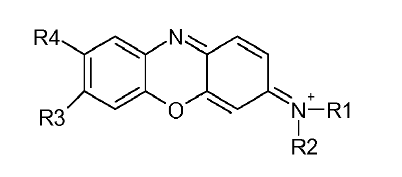 dans lesquelles R1, R2, R3 et R4 sont tels que définis ci-dessus. A titre d'exemple, on peut citer 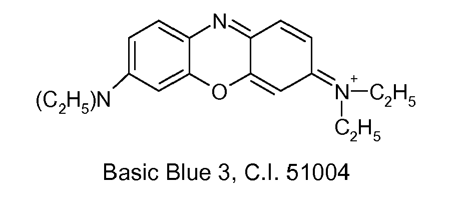
- Les dérives de 1,4-DISTYLBENZENES de formule : 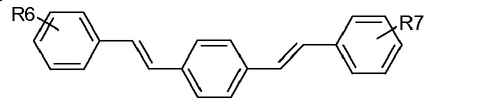 dans laquelle R6 et R7 indépendamment les uns des autres représentent un atome d'hydrogène ; un atome d'halogène ; un groupement aryle en C6-C30 ; un groupement hydroxy ; un groupement cyano ; un groupement nitro ; un groupement sulfo ; un groupement amino ; un groupement acylamino ; un groupement dialkyl(C1-C6)amino ; un groupement dihydroxyalkyl(C1-C6)amino ; un groupement alkyl(C1-C6)hydroxyalkyl(C1-C6)amino ; un groupement alcoxy(C1-C6) ; un groupement alcoxy(C1-C6)carbonyle ; un groupement carboxyalcoxy en C1-C6; un groupement pipéridinosulfonyle; un groupement pyrrolidino ; un groupement alkyle(C1-C6)halogénoalkyle(C1-C6)amino ; un groupement benzoylalkyle(C1-C6) ; un groupement vinyle ; un groupement formyle ; un radical aryle en C6-C30 éventuellement substitué par un ou plusieurs groupements choisis parmi les groupements hydroxyle, alcoxy en C1-C6 linéaire, ramifié ou cyclique, alkyle linéaire, ramifié ou cyclique comprenant de 1 à 22 atomes de carbone lui-même étant éventuellement substitué par un ou plusieurs groupements hydroxyle,amino, alcoxy C1-C6 ; un radical alkyle linéaire, ramifié ou cyclique, comprenant de 1 à 22 atomes de carbone, plus particulièrement de 1 à 6 atomes de carbone, éventuellement substitué par un ou plusieurs groupements choisis parmi les groupements hydroxyle, amino, alcoxy en C1-C6 linéaire, ramifié ou cyclique, aryle éventuellement substitué, carboxyle, sulfo, un atome d'halogène,ce radical alkyle pouvant être interrompu par un hétéroatome, tel que N, S ou O.
- Les dérivés 4,4'-DISTYRYLBIPHENYLES de formule : 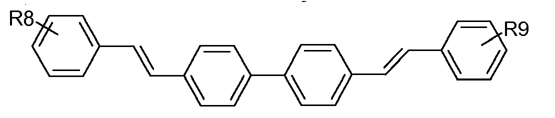 dans laquelle R8 et R9 indépendamment les uns des autres représentent un atome d'hydrogène ; un atome d'halogène ; un groupement aryle en C6-C30 ; un groupement hydroxy ; un groupement cyano ; un groupement nitro ; un groupement sulfo ; un groupement amino ; un groupement acylamino ; un groupement dialkyl(C1-C6)amino ; un groupement dihydroxyalkyl(C1-C6)amino; un groupement alkyl(Cl-C6)hydroxyalkyl(Cl-C6)amino ; un groupement alcoxy(C1-C6) ; un groupement alcoxy(C1-C6)carbonyle ; un groupement carboxyalcoxy en C1-C6; un groupement pipéridinosulfonyle; un groupement pyrrolidino ; un groupement alkyle(C1-C6)halogénoalkyle(C1-C6)amino ; un groupement benzoylalkyle(C1-C6) ; un groupement vinyle ; un groupement formyle ; un radical aryle en C6-C30 éventuellement substitué par un ou plusieurs groupements choisis parmi les groupements hydroxyle, alcoxy en C1-C6 linéaire, ramifié ou cyclique, alkyle linéaire, ramifié ou cyclique comprenant de 1 à 22 atomes de carbone lui-même étant éventuellement substitué par un ou plusieurs groupements hydroxyle,amino, alcoxy C1-C6 ; un radical alkyle linéaire, ramifié ou cyclique, comprenant de 1 à 22 atomes de carbone, plus particulièrement de 1 à 6 atomes de carbone, éventuellement substitué par un ou plusieurs groupements choisis parmi les groupements hydroxyle, amino, alcoxy en C1-C6 linéaire, ramifié ou cyclique, , aryle éventuellement substitué, carboxyle, sulfo, un atome d'halogène,ce radical alkyle pouvant être interrompu par un hétéroatome, tel que l'azote, le soufre et l'oxygène.
- Les dérivés TRIAZINYLAMINOSTILBENES de formule : 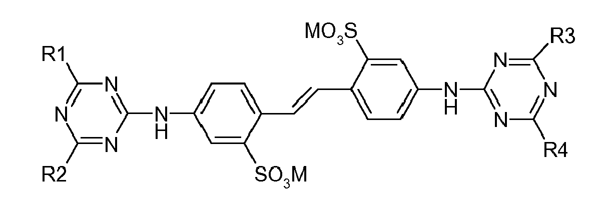 dans laquelle R1, R2, R3 et R4 indépendamment les uns des autres représentent un atome d'hydrogène ; un atome d'halogène ; un groupement aryle en C6-C30 ; un groupement hydroxy ; un groupement cyano ; un groupement nitro ; un groupement sulfo ; un groupement amino ; un groupement acylamino ; un groupement dialkyl(C1-C6)amino ; un groupement dihydroxyalkyl(C1-C6)amino ; un groupement alkyl(C1-C6)hydroxyalkyl(C1-C6)amino ; un groupement alcoxy(C1-C6) ; un groupement alcoxy(C1-C6)carbonyle ; un groupement carboxyalcoxy en C1-C6 ; un groupement pipéridinosulfonyle ; un groupement pyrrolidino ; un groupement alkyle(C1-C6)halogénoalkyle(C1-C6)amino ; un groupement benzoylalkyle(C1-C6) ; un groupement vinyle ; un groupement formyle ; un radical aryle en C6-C30 éventuellement substitué par un ou plusieurs groupements choisis parmi les groupements hydroxyle, alcoxy en C1-C6 linéaire, ramifié ou cyclique, alkyle linéaire, ramifié ou cyclique comprenant de 1 à 22 atomes de carbone lui-même étant éventuellement substitué par un ou plusieurs groupements hydroxyle,amino, alcoxy C1-C6 ; un radical alkyle linéaire, ramifié ou cyclique, comprenant de 1 à 22 atomes de carbone, plus particulièrement de 1 à 6 atomes de carbone, éventuellement substitué par un ou plusieurs groupements choisis parmi les groupements hydroxyle, amino, alcoxy en C1-C6 linéaire, ramifié ou cyclique, , aryle éventuellement substitué, carboxyle, sulfo, un atome d'halogène,ce radical alkyle pouvant être interrompu par un hétéroatome, tel que l'azote, le soufre et l'oxygène ;
   et M est un cation monovalent ou divalent issu de la famille des alcalins ou des alcalino-terreux, comme par exemple les ions sodium, potassium et calcium.
- Les dérivés Bis(BENZOXAZOLE) de formule : 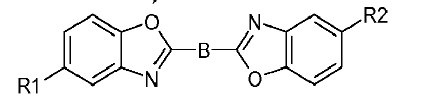 dans laquelle R1, R2 indépendamment les uns des autres représentent un atome d'hydrogène ; un atome d'halogène ; un groupement aryle en C6-C30 ; un groupement hydroxy ; un groupement cyano ; un groupement nitro ; un groupement sulfo ; un groupement amino ; un groupement acylamino ; un groupement dialkyl(C1-C6)amino ; un groupement dihydroxyalkyl(C1-C6)amino ; un groupement alkyl(C1-C6)hydroxyalkyl(C1-C6)amino ; un groupement alcoxy(C1-C6) ; un groupement alcoxy(C1-C6)carbonyle ; un groupement carboxyalcoxy en C1-C6; un groupement pipéridinosulfonyle; un groupement pyrrolidino ; un groupement alkyle(C1-C6)halogénoalkyle(C1-C6)amino ; un groupement benzoylalkyle(C1-C6) ; un groupement vinyle ; un groupement formyle ; un radical aryle en C6-C30 éventuellement substitué par un ou plusieurs groupements choisis parmi les groupements hydroxyle, alcoxy en C1-C6 linéaire, ramifié ou cyclique, alkyle linéaire, ramifié ou cyclique comprenant de 1 à 22 atomes de carbone lui-même étant éventuellement substitué par un ou plusieurs groupements hydroxyle,amino, alcoxy C1-C6 ; un radical alkyle linéaire, ramifié ou cyclique, comprenant de 1 à 22 atomes de carbone, plus particulièrement de 1 à 6 atomes de carbone, éventuellement substitué par un ou plusieurs groupements choisis parmi les groupements hydroxyle, amino, alcoxy en C1-C6 linéaire, ramifié ou cyclique, , aryle éventuellement substitué, carboxyle, sulfo, un atome d'halogène,ce radical alkyle pouvant être interrompu par un hétéroatome, tel que l'azote, le soufre et l'oxygène ; et B est choisi parmi 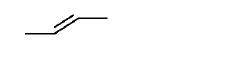 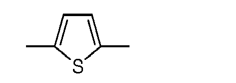 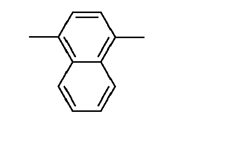 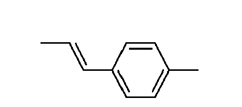
- Les Bis(BENZIMIDAZOLES) cationiques ou non
- Les 1,3-Diphenyl-2-PYRAZOLINES anioniques ou non, notamment

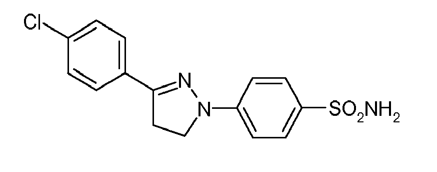

On peut citer les composés dans la publication suivante : « Selective topochemicalphotoreaction of crystallized 2,3-(-phenyletheny)-4,5-dicyanopyrazine" de Kim, Jae Hong; Matsuoka Masaru, Chem. Lett. (1999), (2), 143-144

On peut notamment citer

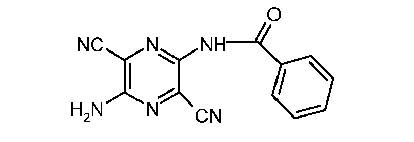

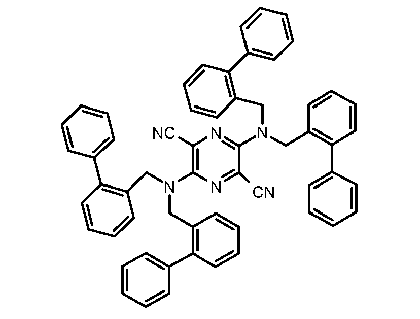

La quantité de composés fluorescents solubles utiles dans la présente invention peuvent varier dans des gammes très variées. A titre d'exemple la quantité de composés fluorescents peut être comprise entre 0,001 et 40 %, préférentiellement entre 0,01 et 20 % et plus particulièrement encore de 0,1 à 10% environ en poids du poids total de la composition.

### III) Solvants organiques

Par solvant organique, on entend une substance organique liquide à température de 25 °C et à pression atmosphérique (760 mmHg) capable de dissoudre une autre substance sans la modifier chimiquement.

Le ou les solvants organiques sont par exemple choisis parmi les alcools aromatiques tels que l'alcool benzylique, le phénoxyéthanol, l'alcool phényléthylique ; les alcools gras liquides, notamment en C₁₀-C₃₀ ; les alcanols en C₁-C₆ tels que l'éthanol, l'isopropanol, le n-propanol, le butanol, le n-pentanol, le 1,2-propanediol, le 1,3-propanediol, le 1-méthoxy 2-propanol, le 1-éthoxy 2-propanediol, les 1,3 et 1,4-butanediol, le 1,2-hexanediol ; les polyols et éthers de polyols possédant une fonction - OH libre tels que le 2-butoxyéthanol, le propylèneglycol, le monométhyléther de propylèneglycol, le monoéthyléther et le monométhyléther du diéthylèneglycol, l'éthylène glycol monoéthyléther, l'éthylène glycol mono butyl éther, le néopentyl glycol, l'isoprèneglycol, le glycérol, le glycol, le dipropylène glycol, le butylène glycol, le butyle diglycol ; les silicones volatiles telles que les silicones linéaires à chaîne courte telles que l'hexaméthyldisiloxane, l'octaméthyltrisiloxane, les silicones cycliques telles que l'octaméthylcyclotétrasiloxane, la décaméthylcyclopentasiloxane, la dodécaméthylcyclo-hexasiloxane, les polydiméthylsiloxanes modifiées ou non par des fonctions alkyle et / ou amine et / ou imine et / ou fluoroalkyl et / ou carboxylique et / ou bétaïne et / ou ammonium quaternaire ; les polydiméthylsiloxanes modifiées liquides ; les huiles minérales, organiques ou végétales ; les alcanes et plus particulièrement les alcanes de C₅ à C₁₀; les acides gras liquides ; les esters gras liquides et plus particulièrement les benzoates ou les salicylates d'alcool gras liquides.

Le ou les solvants organiques sont de préférence choisis parmi les huiles organiques ; les silicones telles que les silicones volatiles, les gommes ou huiles de silicones aminés ou non et leurs mélanges ; les huiles minérales ; les huiles végétales telles que les huiles d'olive, de ricin, de colza, de coprah, de germe de blé, d'amande douce, d'avocat, de macadamia, d'abricot, de carthame, de noix de bancoulier, de camélina, de tamanu, de citron ou encore des composés organiques tels que des alcanes en C₅-C₂₀, l'acétone, la méthyléthylcétone, les esters d'acides en C₁-C₂₀ liquides et d'alcools en C₁-C₈ tels que l'acétate de méthyle, l'acétate de butyle, l'acétate d'éthyle et le myristate d'isopropyle, le diméthoxyéthane, le diéthoxyéthane, les alcools gras liquides en C₁₀-C₃₀ tels que l'alcool oléique, les esters d'alcools gras ou d'acide gras tels que les benzoates d'alcool gras en C₁₀-C₃₀ et leurs mélanges ; l'isononanoate d'isononyle, le malate d'isostéaryle, le tétra-isostéarate de pentaérythrityle, le trimélate de tridécyle, l'huile de polybutène, le mélange cyclopentasiloxane (14,7 % en poids) / polydiméthylsiloxane dihydroxylé en positions α et w (85,3 % en poids), ou leurs mélanges.

Selon un mode de réalisation préféré, le ou les solvants organiques sont choisis parmi les silicones telles que les polydiméthylsiloxanes liquides et les polydiméthylsiloxanes modifiées liquides, leur viscosité à 25 °C étant comprise entre 0,1 cst et 1 000 000 cst, et plus préférentiellement entre 1 cst et 30 000 cst.

On citera de préférence les huiles suivantes :
- le mélange de polydiméthylsiloxane alpha-omega-dihydroxylé / cyclopentadiméthylsiloxane (14,7 / 85,3) commercialisé par Dow Corning sous le nom de DC 1501 Fluid ;
- le mélange de polydiméthylsiloxane alpha-omega-dihydroxylé / polydiméthylsiloxane commercialisé par Dow Corning sous le nom de DC 1503 Fluid ;
- le mélange de diméthicone / cyclopentadiméthylsiloxane commercialisé par Dow Corning sous le nom de DC 1411 Fluid ou celui commercialisé par Bayer sous le nom SF1214 ;
- la cyclopentadiméthylsiloxane commercialisée par Dow Corning sous le nom de DC245 Fluid ;
et les mélanges respectifs de ces huiles.

Ces solvants organiques peuvent servir de diluants pour les réactions de polycondensation.

Le ou les solvants organiques de la composition représentent généralement de 0,01 à 99 %, de préférence de 50 à 99 % en poids par rapport au poids total de la composition.

La composition de l'invention peut contenir, outre le ou les solvants organiques liquides, de l'eau. La composition de l'invention peut être anhydre, c'est-à-dire contenant moins de 1 % en poids d'eau par rapport au poids total de la composition.

La composition de l'invention peut aussi se présenter sous la forme d'une émulsion et/ou être encapsulée. Lorsque la composition est une émulsion elle est par exemple constituée par une phase dispersée ou continue qui peut être de l'eau, des alcools aliphatiques en C1-C4 ou leurs mélanges et une phase organique insoluble dans l'eau.

La composition conforme à l'invention peut également contenir outre les composés de l'invention ie les composés fluorescents solubles, les pigments et les éventuels solvants organiques ; au moins un agent utilisé habituellement en cosmétique choisi, par exemple, parmi des agents réducteurs, des corps gras, des plastifiants, des adoucissants, des agents anti-mousse, des agents hydratants, des pigments, des argiles, des charges minérales, des filtres UV, des colloïdes minéraux, des peptisants, des parfums, des conservateurs, des tensio-actifs anioniques, cationiques, non ioniques ou amphotères, des polymères fixants ou non, des polyols, des protéines, des vitamines, des colorants directs, des colorants d'oxydation, des agents nacrants, des propulseurs, et des épaississants minéraux ou organiques tels que le benzylidène-sorbitol et les N-acylaminoacides, les cires oxyéthylénées ou non, les paraffines, les acides solides : l'acide stéarique, l'acide laurique, les amides gras en C₁₀-C₃₀ tels que le diéthanolamide laurique, les esters d'alcool gras et leurs mélanges.

Cette composition peut se présenter sous des formes diverses, telles que des lotions, des sprays, des mousses et être appliquées sous forme de shampooing ou d'après-shampooing.

Dans le cas des sprays, la composition de l'invention peut contenir un propulseur. Le propulseur est constitué par les gaz comprimés ou liquéfiés usuellement employés pour la préparation de compositions aérosols. On emploiera de manière préférentielle l'air, le gaz carbonique, l'azote comprimé ou encore un gaz soluble tel que le diméthyléther, les hydrocarbures halogénés (fluorés en particuliers) ou non et leurs mélanges.

On pourra aussi le cas échéant utiliser des aérosols de poche.

La composition de l'invention peut être appliquée sur fibres kératiniques sèches ou mouillées, et peuvent être séchées à l'air libre, à l'aide d'un sèche-cheveux ou d'un casque. Les pinces plates peuvent être utilisées après pré-séchage des fibres kératiniques.

Le procédé selon l'invention consiste à appliquer sur les fibres kératiniques au moins un composé X et au moins un composé Y susceptible de réagir ensemble par réaction d'hydrosilylation éventuellement en présence d'un catalyseur d'hydrosylilation, de condensation ou de réticulation en présence d'un peroxyde, lorsqu'ils sont mis en contact l'un de l'autre ; et au moins un colorant fluorescent ou un azurant optique tels que définis précédemment.

Le ou les composés X, le ou les composés Y, peuvent être appliqués sur les fibres kératiniques à partir de plusieurs compositions contenant le ou les composés X, le ou les composés Y, le ou les colorants fluorescents ou azurants optiques, seuls ou en mélange, ou à partir d'une seule composition contenant le ou les composés X, le ou les composés Y, le ou les colorants fluorescents ou azurants optiques et le ou les éventuels solvants organiques.

Selon une première variante des procédés de coloration, le composé X, le composé Y, et les colorants fluorescents ou azurants optiques et le ou les solvants organiques sont appliqués indépendamment les uns des autres.

Selon un autre mode de réalisation particulier de l'invention, on applique sur les fibres kératiniques une composition (C) comprenant le ou les composés X, le ou les composés Y, le ou les colorants fluorescents ou azurants optiques éventuellement en présence de solvant.

Selon un autre mode de réalisation particulier de l'invention, on applique sur les fibres kératiniques une composition (D) comprenant le ou les colorants fluorescents ou azurants, et une composition (E) comprenant le ou les composés X et le ou les composés Y, un ou des solvants organiques étant éventuellement présents dans la composition (D) et / ou la composition (E), l'ordre d'application des compositions (D) et (E) étant indifférent.

Selon un autre mode de réalisation particulier de l'invention, on applique sur les fibres kératiniques une composition (D) comprenant le ou les pigments, colorants fluorescents ou azurants optiques, une composition (A) comprenant le ou les composés X et une composition (B) comprenant le ou les composés Y, un ou des solvants organiques étant éventuellement présents dans la composition (D) et / ou la composition (A) et / ou la composition (B), l'ordre d'application des compositions (A), (B) et (D) étant indifférent.

Selon un autre mode de réalisation particulier de l'invention, on applique sur les fibres kératiniques une composition (F) comprenant le ou les composés X et le ou les pigments, colorants fluorescents ou azurants optiques, et une composition (B) comprenant le ou les composés Y, un ou des solvants organiques étant éventuellement présents dans la composition (B) et / ou la composition (F), l'ordre d'application des compositions (B) et (F) étant indifférent.

Selon un autre mode de réalisation particulier de l'invention, on applique sur les fibres kératiniques une composition (A) comprenant le ou les composés X et une composition (G) comprenant le ou les composés Y et le ou les pigments, colorants fluorescents ou azurants optiques, un ou des solvants organiques étant éventuellement présents dans la composition (A) et / ou la composition (G), l'ordre d'application des compositions (A) et (G) étant indifférent.

Selon un mode de réalisation préféré de l'invention, la composition comprenant le ou les pigments, colorants fluorescents ou azurants optiques est appliquée avant la ou les compositions comprenant le ou les composés X et / ou le ou les composés Y.

Lorsque les composés X et Y sont susceptibles de réagir ensemble par une réaction de réticulation on applique sur les fibres kératiniques au moins un peroxyde tel que décrit précédemment. Le ou les peroxydes peuvent être présents dans l'une ou l'autre des compositions citées ou dans une composition supplémentaire.

Selon un autre mode de réalisation particulier de l'invention, on applique sur les fibres kératiniques au moins un catalyseur tel que défini précédemment pour activer la réaction entre le ou les composés X et le ou les composés Y.

Par exemple, le ou les catalyseurs peuvent être présents dans l'une ou l'autre ou dans plusieurs des compositions appliquées sur les fibres kératiniques ou dans une composition supplémentaire, auquel cas l'ordre d'application des différentes compositions sur les fibres kératiniques est indifférent.

Les catalyseurs avantageusement choisis sont ceux qui sont décrits précédemment.

Lorsqu'on applique au moins un catalyseur et/ou au moins un peroxyde le ou les composés X et/ou le ou les composés Y, le ou les catalyseurs et/ou le ou les peroxydes ne sont pas stockés simultanément la même composition. Ils peuvent par contre être mélangé au moment de l'emploi.

Selon un autre mode de réalisation particulier de l'invention, on applique sur les fibres kératiniques au moins un composé réactif additionnel tel que défini précédemment.

Par exemple, le ou les composés réactifs additionnels peuvent être présents dans l'une ou l'autre ou dans plusieurs des compositions appliquées sur les fibres kératiniques ou dans une composition supplémentaire, auquel cas l'ordre d'application des différentes compositions sur les fibres kératiniques est indifférent.

Les composés réactifs additionnels avantageusement choisis sont ceux qui sont décrits précédemment.

Les différentes compositions mises en oeuvre dans le procédé conforme à l'invention peuvent être appliquées sur des cheveux secs ou humides.

Un séchage intermédiaire et/ou un rinçage peut être réalisé entre chaque application.

Chaque composition utilise dans le procédé conforme à l'invention comprend un milieu cosmétiquement acceptable, véhiculant le ou les composés X et/ou le ou les composés Y, et choisi de telle sorte que les composés X et Y soient aptes à réagir l'un avec l'autre par réaction d'hydrosilylation, de condensation ou de réticulation en présence de peroxyde après l'application de la composition cosmétique sur les cheveux.

Le dépôt ainsi formé présente l'avantage d'avoir une faible solubilité attendue. En outre, il possède une bonne affinité pour la surface des fibres kératiniques, ce qui garantit une meilleure rémanence de l'ensemble du dépôt.

Lorsque les composés X et Y sont appliqués séparément, le dépôt en couches obtenu peut aussi être avantageux pour conserver les propriétés cosmétiques ou optiques du composé qui constitue la partie supérieure du dépôt.

Selon les mêmes procédés, il est possible de réaliser des superpositions multiples de couches de composés X et Y en alternance ou non pour atteindre le type de dépôt souhaité (en termes de nature chimique, résistance mécanique, épaisseur, aspect, toucher...).

L'invention concerne également un Kit de coloration comprenant au moins deux compositions (A) et (B) conditionnées séparément, le kit comprenant i) au moins un composé X, ii) au moins un composé Y, étant entendu que la composition (A) et/ou (B) comprenant au moins un colorant fluorescent ou un azurant optique ; et iii) éventuellement au moins un catalyseur ou un peroxyde ; le catalyseur lorsqu'il est présent ou le peroxyde ne sont pas présents simultanément dans la même composition ; lesdits composés X et Y étant susceptibles de réagir ensemble par une réaction d'hydrosilylation, ou par une réaction de condensation ou par une réaction de réticulation en présence d'un peroxyde, lorsqu'ils sont mis en contact les uns avec les autres.

Une variante concerne un dispositif à plusieurs compartiments avec un premier compartiment contenant une composition (A) comprenant au moins un composé X, un deuxième compartiment contenant une composition (B) comprenant au moins un composé Y, et un troisième compartiment contenant une composition (D) comprenant au moins un colorant fluorescent ou azurant optique.

Selon une mode de réalisation particulier de l'invention, le kit comporte deux compartiment, le premier compartiment comprend une composition (C) contenant au moins un composé X, au moins un composé Y, et au moins un colorant fluorescent ou azurant optique, et un second compartiment comprenant un catalyseur ou un peroxyde.

Une autre variante de kit concerne un dispositif à plusieurs compartiments comprenant un premier compartiment d'une composition (F) contenant au moins un composé X et au moins un colorant fluorescent ou azurant optique éventuellement en présence de solvant et un autre compartiment d'une composition (B) comprenant au moins un composé Y et éventuellement un troisième compartiment contenant au moins un catalyseur.

Une autre variante concerne un kit à plusieurs compartiments comprenant un premier compartiment d'une composition (G) contenant au moins un composé Y et au moins un colorant fluorescent ou azurant optique éventuellement en présence de solvant et un autre compartiment d'une composition (A) comprenant au moins un composé X et éventuellement un troisième compartiment contenant au moins un catalyseur.

Les exemples non limitatifs suivants permettent d'illustrer l'invention sans en limiter sa portée.

### EXEMPLES :

### Condensation

A titre d'exemple d'une combinaison de composés X et Y porteurs de groupements alcoxysilanes et réagissant par condensation, on peut citer les compositions suivantes comprenant des mélanges A' et B' préparés par la société Dow Corning.

### Avec un colorant fluorescent :

| **Composition 1** | en g |
|---|---|
| Cyclopentadiméthylsiloxane commercialisé par Dow Corning sous le nom de DC245 Fluid | 73,34 |
| Mélange de polydiméthylsiloxane à groupements methoxy, hexamethyldisiloxane, silice traitée, fourni par Dow Corning : *Mélange A'* | 15,15 |
| Pigments Sunbrite-SG2515 Yellow orange de SunChemical | 10 |

### où Mélange A' :

| ***Ingrédient (Nom INCI)*** | ***N°CAS*** | ***Teneurs (%)*** | ***Fonction*** |
|---|---|---|---|
| *Bis-Trimethoxysiloxyethyl Tetramethyldisiloxyethyl Dimethicone** | *PMN87176* | *25-45* | *Polymère* |
| *Silica Silylate* | *68909-20-6* | *5-20* | *Charge* |
| *Disiloxane* | *107-46-0* | *30-70* | *Solvant* |

| | | | |
|---|---|---|---|
| ** correspond aux composés X et Y identiques* | | | |

| **Composition 2** | En g |
|---|---|
| Mélange hexamethyldisiloxane et catalyseur de titane fourni par Dow Corning : *Mélange B'* | 1,51 |

### où Mélange B':

| ***Ingrédient (Nom INCI)*** | ***N°CAS*** | ***Teneurs* (%)** | ***Fonction*** |
|---|---|---|---|
| *Disiloxane* | *107-46-0* | *80-99* | *Solvant* |
| *Tetra T Butyl Titanate* | - | *1-20* | *Catalyseur* |

| | | | |
|---|---|---|---|
| On mélange de façon extemporanée les deux compositions 1 et 2 ci-dessus pour obtenir 100g d'un mélange. 0,5g de ce mélange est appliqué sur une mèche de 1g de cheveux naturels de hauteur de ton 4 propres et humides. Après une heure de pose, la mèche est séchée au sèche-cheveux pendant 2 minutes. On obtient une mèche colorée dont les cheveux sont individualisés. La couleur est rémanente. | | | |

### Avec un azurant optique :

| **Composition 3** | en g |
|---|---|
| Cyclopentadiméthylsiloxane commercialisé par Dow Corning sous le nom de DC245 Fluid | 73,34 |
| Mélange de polydiméthylsiloxane à groupements methoxy, hexamethyldisiloxane, silice traitée fournie par Dow Corning : *Mélange A'* | 15,15 |
| Diéthylamino coumarine de chez CIBA | 10 |

| **Composition 2** | En g |
|---|---|
| Mélange hexamethyldisiloxane et catalyseur de titane fourni par Dow Corning : *Mélange B'* | 1,51 |

Où les mélanges A' et B' sont tels que définis précédemment

On mélange de façon extemporanée les deux compositions ci-dessus pour obtenir 100g d'un mélange. 0,5g de ce mélange est appliqué sur une mèche de 1g de cheveux naturels de hauteur de ton 4. Après une heure de pose, la mèche est séchée au sèche-cheveux pendant 2 minutes. On obtient une mèche brillante dont les cheveux sont individualisés. La brillance est rémanente.

### Hydrosilylation

A titre d'exemple d'une combinaison de composés X et Y réagissant par hydrosilylation, on peut citer les compositions 4, 5 et 6, ainsi que les mélanges A et B suivants fournis par la société Dow Corning :

### Avec un pigment fluorescent :

| **Composition 4** | % en poids |
|---|---|
| Cyclopentadiméthylsiloxane commercialisé par Dow Corning sous le nom de DC245 Fluid | 70 |
| Mélange de polydiméthylsiloxane à groupements vinyliques terminaux, hexamethyldisiloxane, Catalyseur de Pt (II), silice traitée, fourni par Dow Corning, *Mélange A* | 10 |
| Pigments Sunbrite-SG2515 Yellow orange de SunChemical | 20 |

### Où Mélange A:

| ***Ingrédient (Nom INCl)*** | ***N°CAS*** | ***Teneurs (%)*** | ***Fonction*** |
|---|---|---|---|
| *Dimethyl Siloxane, Dimethylvinylsiloxy-terminated* | *68083-19-2* | *55-95* | *Polymère* |
| *Silica Silylate* | *68909-20-6* | *10-40* | *Charge* |
| *1,3-Diethenyl-1,1,3,3-Tetramethyldisiloxane complexes* | *68478-92-2* | *Trace* | *Catalyseur* |
| *Tetramethyldivinyldisiloxane* | *2627-95-4* | *0.1-1* | *Polymère* |

| **Composition 5** | % en poids |
|---|---|
| Cyclopentadiméthylsiloxane commercialisé par Dow Corning sous le nom de DC245 Fluid | 90 |
| Mélange de polydiméthylsiloxane à groupements vinyliques terminaux, methylhydrogenosiloxane (agent de réticulation) et silice traitée, fourni par Dow Corning : *Mélange B* | 10 |

### Où Mélange B:

| ***Ingrédient (Nom INCI)*** | ***N°CAS*** | ***Teneurs (%)*** | ***Fonction*** |
|---|---|---|---|
| *Dimethyl Siloxane, Dimethylvinylsiloxy-terminated* | *68083-19-2* | *55-95* | *Polymère* |
| *Silica Silylate* | *68909-20-6* | *10-40* | *Charge* |
| *Dimethyl, Methylhydrogen Siloxane, trimethylsiloxy-terminated* | *68037-59-2* | *1-10* | *Polymère* |

On mélange de façon extemporanée les deux compositions 4 et 5 en proportion pondérale 50/50. 0.5g de ce mélange est appliqué sur une mèche de 1 g de cheveux naturels de hauteur de ton 4 propres et humides. Après une heure de pose, la mèche est séchée au sèche-cheveux pendant 2 minutes. On obtient une mèche colorée dont les cheveux sont individualisés. La couleur est rémanente.

### Avec un azurant optique :

| **Composition 6** | % en poids |
|---|---|
| Cyclopentadiméthylsiloxane commercialisé par Dow Corning sous le nom de DC245 Fluid | 70 |
| Mélange de polydiméthylsiloxane à groupements vinyliques terminaux, hexamethyldisiloxane, Catalyseur de Pt (II), silice traitée, fourni par Dow Corning, *Mélange B* | 10 |
| Diéthylamino coumarine de chez CIBA | 20 |

| **Composition 5** | % en poids |
|---|---|
| Cyclopentadiméthylsiloxane commercialisé par Dow Corning sous le nom de DC245 Fluid | 90 |
| Mélange de polydiméthylsiloxane à groupements vinyliques terminaux, methylhydrogenosiloxane (agent de réticulation) et silice traitée, fourni par Dow Corning : *Mélange* B | 10 |

Où les mélanges A et B sont tels que définis précédemment

On mélange de façon extemporanée les deux compositions 6 et 5 en proportion pondérale 50/50. 0.5g de ce mélange est appliqué sur une mèche de 1 g de cheveux naturels de hauteur de ton 4propres et humides. Après une heure de pose, la mèche est séchée au sèche-cheveux pendant 2 minutes. On obtient une mèche brillante dont les cheveux sont individualisés. La brillance est rémanente.

## Revendications

1. Composition pour la coloration des fibres kératiniques comprenant : i) au moins un composé X ; ii) au moins un composé Y, l'un au moins des composés X ou Y étant un composé siliconé, lesdits composés X et Y étant susceptibles de réagir ensemble par réaction d'hydrosilylation éventuellement en présence d'un catalyseur d'hydrosilylation ; de condensation, ou de réticulation en présence d'un peroxyde, lorsqu'ils sont mis en contact l'un de l'autre ; et **iii)** au moins un colorant fluorescent.

2. Composition selon la revendication précédente dans laquelle les composés X et Y sont susceptibles de réagir par hydrosilylation.

3. Composition selon la revendication précédente dans laquelle le composé X est choisi parmi les composés siliconés comprenant au moins deux groupements aliphatiques insaturés pendants à la chaîne principale ou situés aux extrémités de la chaîne principale du composé.

4. Composition selon la revendication 2 ou 3 dans laquelle le composé X est choisi parmi les polyorganosiloxanes comprenant au moins deux groupements aliphatiques insaturés lié à un atome de silicium.

5. Composition selon l'une des revendications 2 à 4 dans laquelle le composé X est choisi parmi les polyorganosiloxanes comprenant des unités siloxanes de formule : 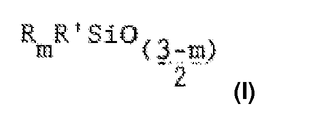 dans laquelle :
- R représente un groupe hydrocarboné monovalent, linéaire ou cyclique, comprenant de 1 à 30 atomes de carbone,
- m est égal à 1 ou 2 et
- R' représente un groupement hydrocarboné aliphatique insaturé comprenant de 2 à 10 ; ou un groupement hydrocarboné cyclique insaturé comprenant de 5 à 8 atomes de carbone.

6. Composition selon la revendication précédente dans laquelle R représente un radical alkyle comprenant de 1 à 10 atomes de carbone ou encore un groupement phényle et R' est choisi parmi les groupements vinyle, allyle, hexényle et leurs mélanges.

7. Composition selon la revendication 5 ou 6 dans laquelle les polyorganosiloxanes comprennent en outre des unités de formule : 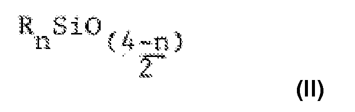 dans laquelle R est un groupe tel que défini dans la revendication 6, est n est égal à 1, 2 ou 3.

8. Composition selon la revendication 2 ou 3 dans laquelle le composé X est choisi parmi les oligomères ou polymères organiques, les oligomères ou polymères hybrides organique / silicone, lesdits oligomères ou polymères portant au moins 2 groupements aliphatiques insaturés réactifs et leurs mélanges.

9. Composition selon la revendication précédente dans laquelle le composé X est choisi parmi les polymères ou oligomères vinyliques, (méth)acryliques, les polyesters, les polyuréthanes et/ou les polyurées, les polyéthers, les perfluoropolyéthers, les polyoléfines, les dendrimères ou les polymères hyper-ramifiés organiques, lesdits polymères portant au moins 2 groupements aliphatiques insaturés réactifs et leurs mélanges.

10. Composition selon l'une des revendications 2 à 9 dans laquelle le composé Y est choisi parmi les polyorganosiloxanes comprenant au moins deux groupes Si-H libres.

11. Composition selon l'une des revendications 2 à 10 dans laquelle le composé Y est choisi parmi les polyorganosiloxanes comprenant des unités alkylhydrogènosiloxanes de formule suivante : 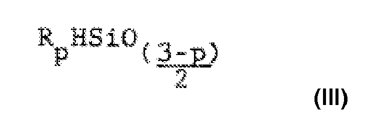 dans laquelle :
- R représente un groupe hydrocarboné monovalent, linéaire ou cyclique, comprenant de 1 à 30 atomes de carbone ; et
- p est égal à 1 ou 2.

12. Composition selon l'une des revendications 5, 7 ou 11 dans laquelle les radicaux R représentent un groupement méthyle.

13. Composition selon l'une des revendications 2 à 12 dans laquelle les polyorganosiloxanes Y comprennent des groupes terminaux de formule (CH₃)₃SiO_{1/2}.

14. Composition selon l'une des revendications 2 à 13 comprenant en outre un composé réactif additionnel comprenant au moins deux groupes aliphatiques insaturés, tel que :
- une résine de silicone comprenant au moins deux insaturations éthyléniques,
- les particules organiques ou minérales comprenant à leur surface au moins 2 groupements aliphatiques insaturés.

15. Composition selon l'une des revendications 2 à 14 comprenant au moins un catalyseur à base de platine ou d'étain.

16. Composition selon la revendication précédente dans laquelle le ou les catalyseurs représentent de 0,0001 % à 20 % en poids par rapport au poids total de la composition.

17. Composition selon une quelconque des revendications 1 à 12 dans laquelle X est un polydiméthylsiloxane à groupements vinyliques terminaux et le composé Y est le méthylhydrogénosiloxane.

18. Composition selon la revendication 1 dans laquelle les composés X et Y sont susceptibles de réagir par condensation.

19. Composition selon la revendication précédente dans laquelle les composés X et/ou Y, identiques ou différents, sont choisis parmi les composés comprenant au moins deux groupes alcoxysilanes et / ou deux groupes silanols latéraux et/ou en bout de chaîne.

20. Composition selon la revendication 18 ou 19 dans laquelle les composés X et/ou Y, identiques ou différents, comprennent de façon majoritaire des unités de formule :
R⁹₆SiO_{(4-s)/2} (IV)
dans laquelle les R⁹ représentent indépendamment un radical choisi parmi les groupes alkyles comprenant de 1 à 6 atomes de carbone, le phenyle, les groupes alkyl fluorés, et s est égal à 0, 1, 2 ou 3.

21. Composition selon la revendication précédente dans laquelle les polyorganosiloxanes comprennent des unités de formule :
(R⁹₂SiO₂)_{f} - (V)
dans laquelle R⁹ est tel que défini dans la revendication 22, et f est tel que le polymère présente une viscosité à 25 °C allant de 0,5 à 3000 Pa.s.

22. Composition selon l'une des revendications 20 à 21 dans laquelle les polyorganosiloxanes comprennent au moins 2 groupes trialcoxysilane terminaux par molécule de polymère, lesdits groupes ayant la formule suivante :
-ZSIR1ₓ(OR)₃₋ₓ, (VI)
dans laquelle :
les radicaux R représentent indépendamment un groupe méthyle, éthyle, n-propyle, isopropyle, n-butyle, sec-butyle, isobutyle ;
R¹ est un groupe méthyle ou éthyle ;
x est égal à 0 ou 1; et
Z est choisi parmi : les groupes hydrocarbonés divalents ne comportant pas d'insaturation éthylénique et comprenant de 1 à 18 atomes de carbone, les combinaisons de radicaux hydrocarbonés divalents et de segments siloxanes de formule (IX) suivante: 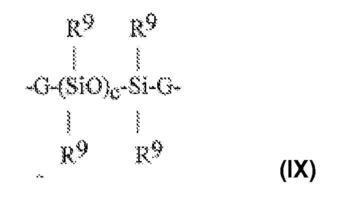
R⁹ étant tel que défini dans la revendication 22, G est un radical hydrocarboné divalent ne comportant pas d'insaturation éthylénique et comprenant de 1 à 18 atomes de carbone et c est un entier allant de 1 à 6.

23. Composition selon l'une des revendications 19 à 22 dans laquelle les polyorganosiloxanes sont choisis parmi les polymères de formule : 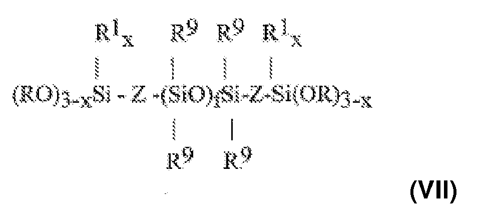 dans laquelle R, R¹, R⁹, Z, x et f sont tels que définis dans la revendication précédente.

24. Composition selon la revendication 18 dans laquelle le composé X est choisi parmi les oligomères ou polymères organiques ou les oligomères et polymères hybrides organique / silicone, lesdits polymères ou oligomères comprenant au moins deux groupements alcoxysilanes et le composé Y est choisi parmi les polyorganosiloxanes. 25.

25. Composition selon l'une des revendications 18 à 24 comprenant au moins un catalyseur à base de titane.

26. Composition selon la revendication précédent dans laquelle le ou les catalyseurs sont présents en une teneur allant de 0,0001 % à 20 % en poids par rapport au poids total de la composition.

27. Composition selon une quelconque des revendications 18 à 26 dans laquelle X et Y représentent un mélange de polydiméthylsiloxane à groupements méthoxysilanes.

28. Composition selon la revendication 1 dans laquelle les composés X et Y sont susceptibles de réagir par réticulation en présence de peroxyde.

29. Composition selon une quelconque des revendications précédentes comprenant dans au moins une des compositions une charge choisie parmi la silice ou la silice traitée en surface.

30. Composition selon l'une quelconque des revendications précédentes dans laquelle le composé X présente une masse moléculaire en poids (Mw) allant de 150 à 1 000 000.

31. Composition selon l'une quelconque des revendications précédentes dans laquelle le composé Y présente une masse moléculaire en poids (Mw) allant de 200 à 1 000 000.

32. Composition selon l'une des revendications précédentes dans laquelle le composé X représente de 0,5 à 95 % en poids par rapport au poids total de la composition.

33. Composition selon l'une des revendications précédentes dans laquelle le composé Y représente de 0,05 à 95 % en poids par rapport au poids total de la composition.

34. Composition selon l'une des revendications précédentes dans laquelle les composés X et Y sont présents dans les compositions en un ratio X / Y allant de 0,05 à 20.

35. Composition selon l'une quelconque des revendications précédentes dans laquelle le ou les colorants fluorescents absorbent dans le rayonnement visible généralement entre 400 et 800 nm réémettent de la lumière dans le domaine du visible à une longueur d'onde supérieure.

36. Composition selon la revendication précédente dans laquelle le colorant est choisi parmi :
• les dérivés méthine de formule : 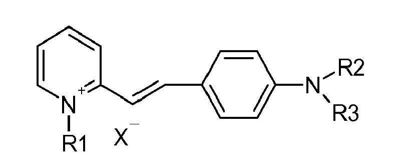 dans laquelle R₁, R₂ et R₃ indépendamment les uns des autres représentent un atome d'hydrogène ; un atome d'halogène ; un groupement aryle en C₆-C₃₀; un groupement hydroxy ; un groupement cyano ; un groupement nitro ; un groupement sulfo ; un groupement amino ; un groupement acylamino ; un groupement dialkyl(C₁-C₆)amino ; un groupement dihydroxyalkyl(C₁-C₆)amino ; un groupement alkyl(C₁-C₆)hydroxyalkyl(C₁-C₆)amino ; un groupement alcoxy(C₁-C₆) ; un groupement alcoxy(C₁-C₆)carbonyle ; un groupement carboxyalcoxy en C₁-C₆; un groupement pipéridinosulfonyle ; un groupement pyrrolidino ; un groupement alkyle(C₁-C₆)halogénoalkyle(C₁-C₆)amino; un groupement benzoylalkyle(C₁-C₆); un groupement vinyle ; un groupement formyle ; un radical aryle en C₆-C₃₀ éventuellement substitué par un ou plusieurs groupements choisis parmi les groupements hydroxyle, alcoxy en C₁-C₆ linéaire, ramifié ou cyclique, alkyle linéaire, ramifié ou cyclique comprenant de 1 à 22 atomes de carbone lui-même étant éventuellement substitué par un ou plusieurs groupements hydroxyle,amino, alcoxy C₁-C₆ un radical alkyle linéaire, ramifié ou cyclique, comprenant de 1 à 22 atomes de carbone, plus particulièrement de 1 à 6 atomes de carbone, éventuellement substitué par un ou plusieurs groupements choisis parmi les groupements hydroxyle, amino, alcoxy en C₁-C6 linéaire, ramifié ou cyclique, , aryle éventuellement substitué, carboxyle, sulfo, un atome d'halogène,ce radical alkyle pouvant être interrompu par un hétéroatome, tel que l'azote, le soufre et l'oxygène ;
deux des substituants R₂ , R₃ peuvent former avec les atomes de carbone auxquels ils sont rattachés un noyau aromatique en C₆-C₃₀ ou hétérocyclique comportant au total de 5 à 30 chaînons et de 1 à 5 hétéroatomes ; ce cycle étant condensés ou non, ce cycle et l'éventuel cycle condensé étant substitués ou non par au moins un groupement choisi parmi les groupements alkyle en C₁-C₄, alcoxy(C₁-C₄)alkyl(C₁-C₄), amino, dialkyl(C₁-C₄)amino, halogène, phényle, carboxy, trialkyl(C₁-C₄)ammonioalkyl(C₁-C₄), et
X⁻ est un anion organique ou minéral ;
• le Jaune Brilliant B6GL de structure suivante : 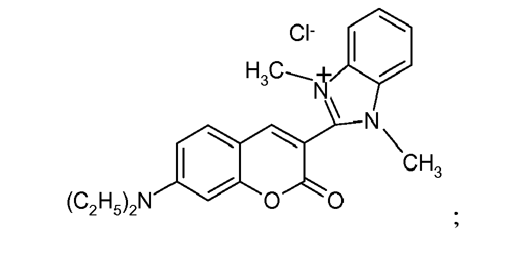
• l'hémicyanine méthinique suivante : 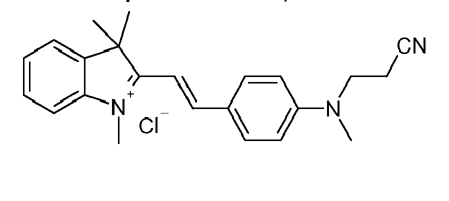
• le Basic Yellow 2, ou Auramine O commercialisé par les sociétés PROLABO, ALDRICH ou CARLO ERBA et de structure suivante : 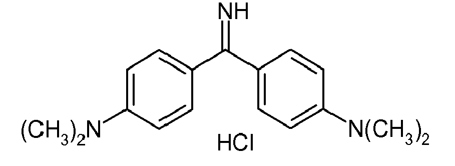 monochlorhydrate de 4,4'-(imidocarbonyl)bis(N,N-diméthylaniline)
• 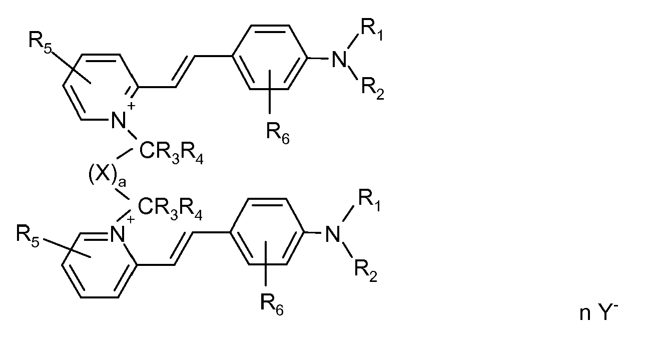 dans laquelle :
R₁, R₂, identiques ou différents, représentent :
■ un atome d'hydrogène ;
■ un radical alkyle, linéaire ou ramifié, comprenant 1 à 10 atomes de carbone, de préférence de 1 à 4 atomes de carbone, éventuellement interrompu et/ou substitué par au moins un hétéroatome et/ou groupement comprenant au moins un hétéroatome et/ou substitué par au moins un atome d'halogène ;
■ un radical aryle ou arylalkyle, le groupement aryle ayant 6 atomes de carbone et le radical alkyle ayant 1 à 4 atomes de carbone ; le radical aryle étant éventuellement substitué par un ou plusieurs radicaux alkyles linéaires ou ramifiés comprenant 1 à 4 atomes de carbone éventuellement interrompus et/ou substitués par au moins un hétéroatome et/ou groupement comprenant au moins un hétéroatome et/ou substitué par au moins un atome d'halogène ;
■ R₁ et R₂ peuvent éventuellement être reliés de manière à former un hétérocycle avec l'atome d'azote et comprendre un ou plusieurs autres hétéroatomes, l'hétérocycle étant éventuellement substitué par au moins un radical alkyle linéaire ou ramifié, comprenant de préférence de 1 à 4 atomes de carbone et étant éventuellement interrompu et/ou substitué par au moins un hétéroatome et/ou groupement comprenant au moins un hétéroatome et/ou substitué par au moins un atome d'halogène ;
■ R₁ ou R₂ peut éventuellement être engagé dans un hétérocycle comprenant l'atome d'azote et l'un des atomes de carbone du groupement phényle portant ledit atome d'azote ;
■ R₃, R₄, identiques ou non, représentent un atome d'hydrogène, un radical alkyle comprenant 1 à 4 atomes de carbone ;
■ R₅, identiques ou non, représentent un atome d'hydrogène, un atome d'halogène, un radical alkyle linéaire ou ramifié comprenant 1 à 4 atomes de carbone éventuellement interrompu par au moins un hétéroatome ;
■ R₆, identiques ou non, représentent un atome d'hydrogène ; un atome d'halogène; un radical alkyle linéaire ou ramifié comprenant 1 à 4 atomes de carbone, éventuellement substitué et/ou interrompu par au moins un hétéroatome et/ou groupement portant au moins un hétéroatome et/ou substitué par au moins un atome d'halogène ;
X représente :
■ un radical alkyle, linéaire ou ramifié comprenant 1 à 14 atomes de carbone, ou alcényle comprenant 2 à 14 atomes de carbone, éventuellement interrompu et/ou substitué par au moins un hétéroatome et/ou groupement comprenant au moins un hétéroatome et/ou substitué par au moins un atome d'halogène ;
■ un radical hétérocyclique comprenant 5 ou 6 chaînons, éventuellement substitué par au moins un radical alkyle linéaire ou ramifié comprenant 1 à 14 atomes de carbone, éventuellement substitué par au moins un hétéroatome ; par au moins un radical aminoalkyle, linéaire ou ramifié, comprenant 1 à 4 atomes de carbone, éventuellement substitué par au moins un hétéroatome ; par au moins un atome d'halogène ;
■ un radical aromatique ou diaromatique condensé ou non, séparé ou non par un radical alkyle comprenant 1 à 4 atomes de carbone, le ou les radicaux aryles étant éventuellement substitués par au moins un atome d'halogène ou par au moins un radical alkyle comprenant 1 à 10 atomes de carbone éventuellement substitué et/ou interrompu par au moins un hétéroatome et/ou groupement portant au moins un hétéroatome ;
■ un radical dicarbonyle ;
■ le groupement X pouvant porter une ou plusieurs charges cationiques ;
■ a étant égal à 0 ou 1 ;
■ Y⁻, identiques ou non, représentant un anion organique ou minéral ;
■ n étant un nombre entier au moins égal à 2 et au plus égal au nombre de charges cationiques présentes dans le composé fluorescent ;
• les dérives de thioxanthènes ;
• les rhodamines ;
• les dimères de Stilbazolium de formules : 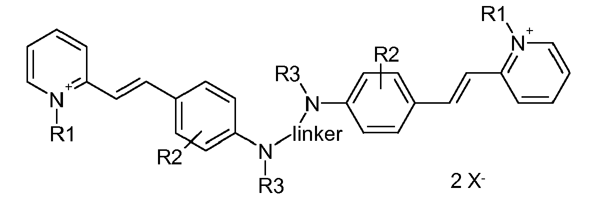 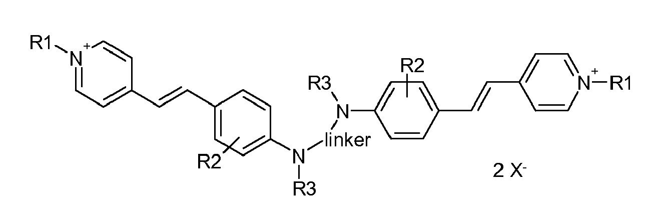 dans lesquelles :
R1, R2, R3 indépendamment les uns des autres représentent un atome d'hydrogène ; un atome d'halogène ; un groupement aryle en C6-C30 ; un groupement hydroxy; un groupement cyano; un groupement nitro; un groupement sulfo ; un groupement amino ; un groupement acylamino ; un groupement dialkyl(C1-C6)amino ; un groupement dihydroxyalkyl(C1-C6)amino ; un groupement alkyl(C1-C6)hydroxyalkyl(C1-C6)amino ; un groupement alcoxy(C1-C6) ; un groupement alcoxy(C1-C6)carbonyle ; un groupement carboxyalcoxy en C1-C6 ; un groupement pipéridinosulfonyle ; un groupement pyrrolidino ; un groupement alkyle(C1-C6)halogénoalkyle(C1-C6)amino ; un groupement benzoylalkyle(C1-C6) ; un groupement vinyle; un groupement formyle ; un radical aryle en C6-C30 éventuellement substitué par un ou plusieurs groupements choisis parmi les groupements hydroxyle, alcoxy en C1-C6 linéaire, ramifié ou cyclique, alkyle linéaire, ramifié ou cyclique comprenant de 1 à 22 atomes de carbone lui-même étant éventuellement substitué par un ou plusieurs groupements hydroxyle,amino, alcoxy C1-C6 ; un radical alkyle linéaire, ramifié ou cyclique, comprenant de 1 à 22 atomes de carbone, plus particulièrement de 1 à 6 atomes de carbone, éventuellement substitué par un ou plusieurs groupements choisis parmi les groupements hydroxyle, amino, alcoxy en C1-C6 linéaire, ramifié ou cyclique, aryle éventuellement substitué, carboxyle, sulfo, un atome d'halogène,ce radical alkyle pouvant être interrompu par un hétéroatome, tel que l'azote, le soufre et l'oxygène ;
Linker représente une chaîne hydrocarbonée en C1-C12, aliphatique ou alicyclique, saturée ou insaturée, un ou plusieurs atomes de carbone de la chaîne hydrocarbonée pouvant être remplacés par un ou plusieurs atomes d'oxygène, un ou plusieurs groupements NR avec R étant un atome d'hydrogène ou un radical alkyle, la chaîne hydrocarbonée ne comportant pas de groupement diazo, nitro, nitroso ou peroxyde, et la chaîne hydrocarbonée ne pouvant pas être terminée à l'une ou l'autre de ses extrémités par un hétéroatome ;
X- représente une contre-ion anionique ;
• les Trimères et tétramères méthiniques suivants 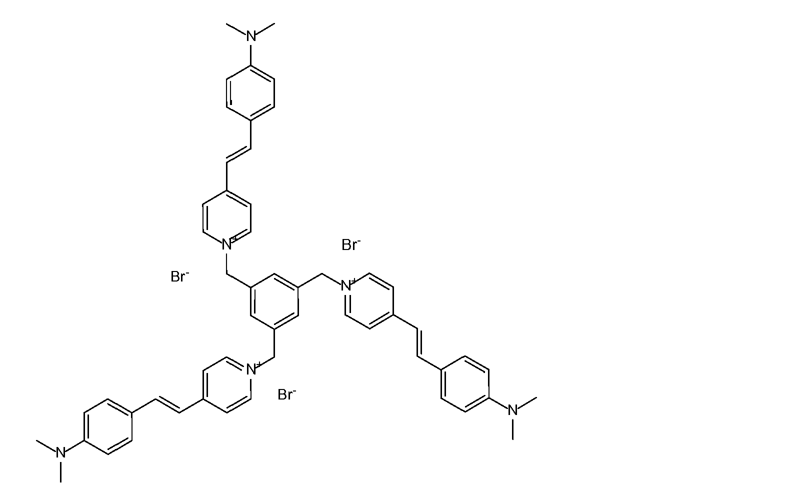 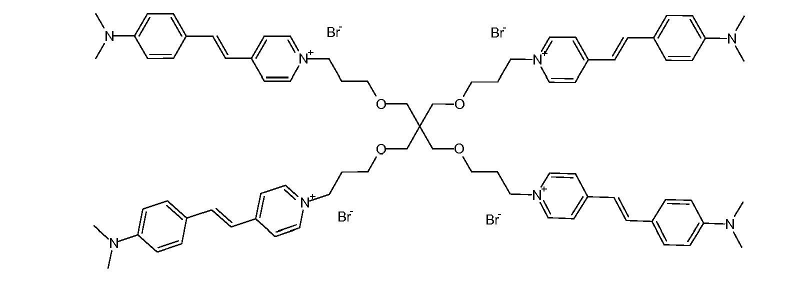
• les dérivés de xanthènes tels que : 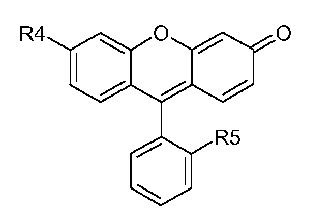 avec R4 et R5 indépendamment les uns des autres représentent un atome d'hydrogène ; un atome d'halogène ; un groupement aryle en C6-C30 ; un groupement hydroxy ; un groupement cyano ; un groupement nitro ; un groupement sulfo ; un groupement amino ; un groupement acylamino ; un groupement dialkyl(C1-C6)amino; un groupement dihydroxyalkyl(C1-C6)amino ; un groupement alkyl(C1-C6)hydroxyalkyl(C1-C6)amino ; un groupement alcoxy(C1-C6) ; un groupement alcoxy(C1-C6)carbonyle ; un groupement carboxyalcoxy en C1-C6; un groupement pipéridinosulfonyle ; un groupement pyrrolidino ; un groupement alkyle(C1-C6)halogénoalkyle(C1-C6)amino ; un groupement benzoylalkyle(C1-C6) ; un groupement vinyle; un groupement formyle ; un radical aryle en C6-C30 éventuellement substitué par un ou plusieurs groupements choisis parmi les groupements hydroxyle, alcoxy en C1-C6 linéaire, ramifié ou cyclique, alkyle linéaire, ramifié ou cyclique comprenant de 1 à 22 atomes de carbone lui-même étant éventuellement substitué par un ou plusieurs groupements hydroxyle,amino, alcoxy C1-C6 ; un radical alkyle linéaire, ramifié ou cyclique, comprenant de 1 à 22 atomes de carbone, plus particulièrement de 1 à 6 atomes de carbone, éventuellement substitué par un ou plusieurs groupements choisis parmi les groupements hydroxyle, amino, alcoxy en C1-C6 linéaire, ramifié ou cyclique, aryle éventuellement substitué, carboxyle, sulfo, un atome d'halogène,ce radical alkyle pouvant être interrompu par un hétéroatome ;
• les naphthalimides de formule : 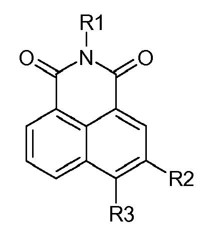 R1, R2, R3, indépendamment les uns des autres représentent un atome d'hydrogène ; un atome d'halogène ; un groupement aryle en C6-C30 ; un groupement hydroxy ; un groupement cyano ; un groupement nitro ; un groupement sulfo ; un groupement amino ; un groupement acylamino ; un groupement dialkyl(C1-C6)amino ; un groupement dihydroxyalkyl(C1-C6)amino ; un groupement alkyl(C1-C6)hydroxyalkyl(C1-C6)amino ; un groupement alcoxy(C1-C6) ; un groupement alcoxy(C1-C6)carbonyle ; un groupement carboxyalcoxy en C1-C6 ; un groupement pipéridinosulfonyle; un groupement pyrrolidino ; un groupement alkyle(C1-C6)halogénoalkyle(C1-C6)amino ; un groupement benzoylalkyle(C1-C6) ; un groupement vinyle ; un groupement formyle ; un radical aryle en C6-C30 éventuellement substitué par un ou plusieurs groupements choisis parmi les groupements hydroxyle, alcoxy en C1-C6 linéaire, ramifié ou cyclique, alkyle linéaire, ramifié ou cyclique comprenant de 1 à 22 atomes de carbone lui-même étant éventuellement substitué par un ou plusieurs groupements hydroxyle, amino, alcoxy C1-C6 ; un radical alkyle linéaire, ramifié ou cyclique, comprenant de 1 à 22 atomes de carbone, éventuellement substitué par un ou plusieurs groupements choisis parmi les groupements hydroxyle, amino, alcoxy en C1-C6 linéaire, ramifié ou cyclique, aryle éventuellement substitué, carboxyle, sulfo, un atome d'halogène, ce radical alkyle pouvant être interrompu par un hétéroatome ; les substituants R1 , R2, R3 peuvent former avec les atomes de carbone auxquels ils sont rattachés un cycle aromatique ou non, en C6-C30 ou hétérocyclique comportant au total de 5 à 30 chaînons et de 1 à 5 hétéroatomes ; ces cycles étant condensés ou non, insérant ou non un groupement carbonyle, et étant substitués ou non par un ou plusieurs groupements choisis parmi les groupements alkyle en C1-C4, alcoxy(C1-C4)alkyl(C1-C4), amino, dialkyl(C1-C4)amino, halogène, phényle, carboxy, trialkyl(C1-C4)ammonioalkyl(C1-C4) ;
• les DICETOPYRROLOPYRROLE de formule : 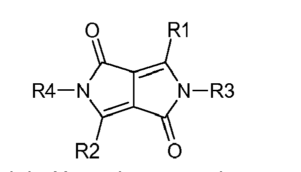 dans laquelle R1, R2, R3 et R4 indépendamment les uns des autres représentent un atome d'hydrogène ; un atome d'halogène ; un groupement aryle en C6-C30 ; un groupement hydroxy ; un groupement cyano ; un groupement nitro ; un groupement sulfo ; un groupement amino ; un groupement acylamino ; un groupement dialkyl(C1-C6)amino ; un groupement dihydroxyalkyl(C1-C6)amino ; un groupement alkyl(C1-C6)hydroxyalkyl(C1-C6)amino ; un groupement alcoxy(C1-C6) ; un groupement alcoxy(C1-C6)carbonyle ; un groupement carboxyalcoxy en C1-C6 ; un groupement pipéridinosulfonyle ; un groupement pyrrolidino ; un groupement alkyle(C1-C6)halogénoalkyle(C1-C6)amino ; un groupement benzoylalkyle(C1-C6) ; un groupement vinyle ; un groupement formyle ; un radical aryle en C6-C30 éventuellement substitué par un ou plusieurs groupements choisis parmi les groupements hydroxyle, alcoxy en C1-C6 linéaire, ramifié ou cyclique, alkyle linéaire, ramifié ou cyclique comprenant de 1 à 22 atomes de carbone lui-même étant éventuellement substitué par un ou plusieurs groupements hydroxyle, amino, alcoxy C1-C6 ; un radical alkyle linéaire, ramifié ou cyclique, comprenant de 1 à 22 atomes de carbone, éventuellement substitué par un ou plusieurs groupements choisis parmi les groupements hydroxyle, amino, alcoxy en C1-C6 linéaire, ramifié ou cyclique, aryle éventuellement substitué, carboxyle, sulfo, un atome d'halogène, ce radical alkyle pouvant être interrompu par un hétéroatome ;
• les dérivés de DICYANO PYRAZINES de formules : 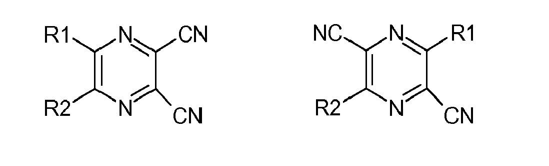 dans lesquelles R1, R2 indépendamment les uns des autres représentent un atome d'hydrogène ; un atome d'halogène ; un groupement aryle en C6-C30 ; un groupement hydroxy ; un groupement cyano ; un groupement nitro ; un groupement sulfo ; un groupement amino ; un groupement acylamino ; un groupement dialkyl(C1-C6)amino ; un groupement dihydroxyalkyl(C1-C6)amino ; un groupement alkyl(C1-C6)hydroxyalkyl(C1-C6)amino ; un groupement alcoxy(C1-C6) ; un groupement alcoxy(C1-C6)carbonyle ; un groupement carboxyalcoxy en C1-C6; un groupement pipéridinosulfonyle; un groupement pyrrolidino ; un groupement alkyle(C1-C6)halogénoalkyle(C1-C6)amino ; un groupement benzoylalkyle(C1-C6) ; un groupement vinyle ; un groupement formyle ; un radical aryle en C6-C30 éventuellement substitué par un ou plusieurs groupements choisis parmi les groupements hydroxyle, alcoxy en C1-C6 linéaire, ramifié ou cyclique, alkyle linéaire, ramifié ou cyclique comprenant de 1 à 22 atomes de carbone lui-même étant éventuellement substitué par un ou plusieurs groupements hydroxyle, amino, alcoxy C1-C6 ; un radical alkyle linéaire, ramifié ou cyclique, comprenant de 1 à 22 atomes de carbone, éventuellement substitué par un ou plusieurs groupements choisis parmi les groupements hydroxyle, amino, alcoxy en C1-C6 linéaire, ramifié ou cyclique, aryle éventuellement substitué, carboxyle, sulfo, un atome d'halogène, ce radical alkyle pouvant être interrompu par un hétéroatome, tel que l'azote, le soufre et l'oxygène ; deux des substituants R1 et R2, peuvent former avec les atomes de carbone auxquels ils sont rattachés un cycle aromatique ou non, en C6-C30 ou hétérocyclique comportant au total de 5 à 30 chaînons et de 1 à 5 hétéroatomes ; ces cycles étant condensés ou non,insérant ou non un groupement carbonyle, et étant substitués ou non par un ou plusieurs groupements choisis parmi les groupements alkyle en C1-C4, alcoxy(C1-C4)alkyl(C1-C4), amino, dialkyl(C1-C4)amino, halogène, phényle, carboxy, trialkyl(C1-C4)ammonioalkyl(C1-C4) ;
deux des substituants R2 , R3 peuvent former avec les atomes de carbone auxquels ils sont rattachés un noyau aromatique en C6-C30 ou hétérocyclique comportant au total de 5 à 30 chaînons et de 1 à 5 hétéroatomes ; ce cycle étant condensés ou non, ce cycle et l'éventuel cycle condensé étant substitués ou non par au moins un groupement choisi parmi les groupements alkyle en C1-C4, alcoxy(C1-C4)alkyl(C1-C4), amino, dialkyl(C1-C4)amino, halogène, phényle, carboxy, trialkyl(C1-C4)ammonioalkyl(C1-C4) ;
• les dérivés de coumarines de formules suivantes : 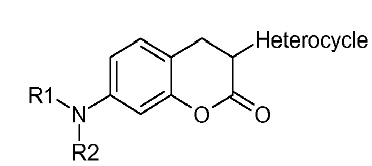 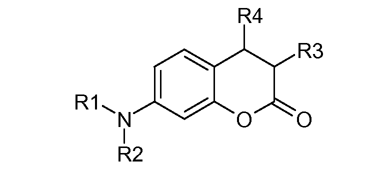 dans laquelle l'hétérocycle est choisi parmi les furane, tiophène, 2H-pyrrole, 2-pyrroline, pyrrolidine, 1,3-dioxolane, oxazole, thiazole, Imidazole, 2-imidazoline, Imidazoline, pyrazole, 2-pyrazoline, pyrazolidine, isoxazole, isothiazole, 1,2,3-oxadiazole, 1,2,3-triazole, 1,3,4-thiadiazole, 2H-pyran, 4H-pyran, pyridine, piperidine, 1,4-dioxane, morpholine, 1,4-dithiane, thiomorpholine, pyradizine, pyrimidine, pyrazine, piperazine, 1,3,5-Triazine, 1,3,5-trithiane, indolizine, indole, isoindole, 3H-indole, indoline, benzo[b]furan, benzo[b]thiophene, 1H-indazole, benzimidazole, benzothiazole, purine, 4H-quinolizine, quinoline, isoquinoline, cinnoline, phtalazine, quinazoline, quinoxaline, 1,8-naphtyridine, pteridine, quinuclidine, carbazole, acridine, phenazine, phenothiazine, phenoxazine, indene, naphtalene, azulene, fluorene, anthracene, norbornane, adamantane, et ;
R1, R2, R3, R4 indépendamment les uns des autres représentent un atome d'hydrogène ; un atome d'halogène ; un groupement aryle en C6-C30 ; un groupement hydroxy ; un groupement cyano ; un groupement nitro ; un groupement sulfo ; un groupement amino ; un groupement acylamino ; un groupement dialkyl(C1-C6)amino ; un groupement dihydroxyalkyl(C1-C6)amino ; un groupement alkyl(C1-C6)hydroxyalkyl(C1-C6)amino ; un groupement alcoxy(C1-C6) ; un groupement alcoxy(C1-C6)carbonyle ; un groupement carboxyalcoxy en C1-C6 ; un groupement pipéridinosulfonyle; un groupement pyrrolidino ; un groupement alkyle(C1-C6)halogénoalkyle(C1-C6)amino ; un groupement benzoylalkyle(C1-C6) ; un groupement vinyle ; un groupement formyle ; un radical aryle en C6-C30 éventuellement substitué par un ou plusieurs groupements choisis parmi les groupements hydroxyle, alcoxy en C1-C6 linéaire, ramifié ou cyclique, alkyle linéaire, ramifié ou cyclique comprenant de 1 à 22 atomes de carbone lui-même étant éventuellement substitué par un ou plusieurs groupements hydroxyle, amino, alcoxy C1-C6 ; un radical alkyle linéaire, ramifié ou cyclique, comprenant de 1 à 22 atomes de carbone, éventuellement substitué par un ou plusieurs groupements choisis parmi les groupements hydroxyle, amino, alcoxy en C1-C6 linéaire, ramifié ou cyclique, aryle éventuellement substitué, carboxyle, sulfo, un atome d'halogène, ce radical alkyle pouvant être interrompu par un hétéroatome, tel qu'un atome d'azote, de soufre ou d'oxygène ; les substituants R1, R2, R3 et R4 peuvent former avec les atomes de carbone auxquels ils sont rattachés un cycle aromatique ou non, en C6-C30 ou hétérocyclique comportant au total de 5 à 30 chaînons et de 1 à 5 hétéroatomes ; ces cycles étant condensés ou non, insérant ou non un groupement carbonyle, et étant substitués ou non par un ou plusieurs groupements choisis parmi les groupements alkyle en C1-C4, alcoxy(C1-C4)alkyl(C1-C4), amino, dialkyl(C1-C4)amino, halogène, phényle, carboxy, trialkyl(C1-C4)ammonioalkyl(C1-C4) ;
deux des substituants R3 , R4 peuvent former avec les atomes de carbone auxquels ils sont rattachés un noyau aromatique en C6-C30 ou hétérocyclique comportant au total de 5 à 30 chaînons et de 1 à 5 hétéroatomes ; ce cycle étant condensés ou non, ce cycle et l'éventuel cycle condensé étant substitués ou non par au moins un groupement choisi parmi les groupements alkyle en C1-C4, alcoxy(C1-C4)alkyl(C1-C4), amino, dialkyl(C1-C4)amino, halogène, phényle, carboxy, trialkyl(C1-C4)ammonioalkyl(C1-C4).
• les dérivés de naphtolactames : 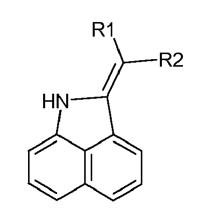 avec R1 et R2 définis comme précédemment ;
• les dérivés AZALACTONES : 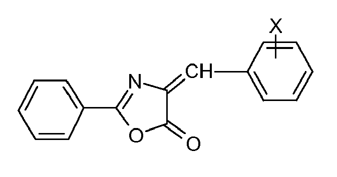 X ayant la même définition que R1 décrit précédemment ;
• les dérivés méthiniques : 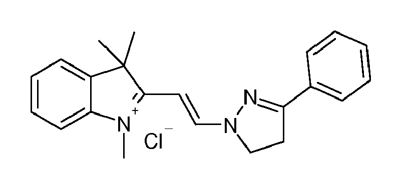
• les dérivés oxazines et thiazines : 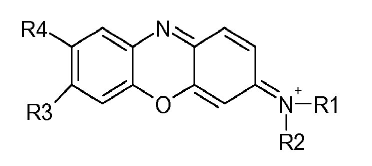
dans lesquelles R1, R2, R3 et R4 sont tels que définis ci-dessus.

37. Composition selon l'une quelconque des revendications précédentes comprenant en outre au moins un azurant optique.

38. Composition selon la revendication précédente dans laquelle l'azurant optique est choisi parmi :
• le dérivé stilbénique de naphto-triazole, le di-styryl-4,4' biphényle sulfonate di-sodique, le dérivé cationique d'aminocoumarine, le diéthylaminométhyl coumarine, le 4-méthyl 7-diéthyl coumarine, le 4,4'-bis[(4,6-dianilino-1,3,5-triazin-2-yl)amino]stilbène-2,2'-disulfonate de sodium, le 4,4'-bis-[(4-anilino-6-bis(2-hydroxyéthyl)amino-1,3,5-triazin-2-yl)amino]stilbène-2,2'-disulfonique acide, le 4,4'-bis-[anilino-6-morpholine-1,3,5-triazin-2-yl)amino] stilbène, le 4,4'-bis-[(4-anilino-6-(2-hydroxy éthyl) méthylamino-1,3,5-triazin-2-yl)amino]stilbène-2,2'-disodium sulfonate,
• le 2,5 thiophène di-yl bis(5 ter-butyl-1,3 benzoxazole),
• le dérivé anionique du di-aminostilbène ;
• les laques d'azurant optique ;
• les naphthalimides,
• les dérives de 1,4-DISTYLBENZENES de formule : 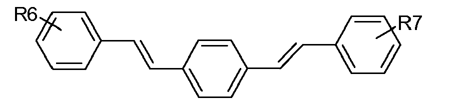 dans laquelle R6 et R7 indépendamment les uns des autres représentent un atome d'hydrogène ; un atome d'halogène ; un groupement aryle en C6-C30 ; un groupement hydroxy; un groupement cyano; un groupement nitro; un groupement sulfo ; un groupement amino ; un groupement acylamino ; un groupement dialkyl(C1-C6)amino ; un groupement dihydroxyalkyl(C1-C6)amino ; un groupement alkyl(C1-C6)hydroxyalkyl(C1-C6)amino ; un groupement alcoxy(C1-C6) ; un groupement alcoxy(C1-C6)carbonyle ; un groupement carboxyalcoxy en C1-C6; un groupement pipéridinosulfonyle ; un groupement pyrrolidino ; un groupement alkyle(C1-C6)halogénoalkyle(C1-C6)amino ; un groupement benzoylalkyle(C1-C6) ; un groupement vinyle; un groupement formyle ; un radical aryle en C6-C30 éventuellement substitué par un ou plusieurs groupements choisis parmi les groupements hydroxyle, alcoxy en C1-C6 linéaire, ramifié ou cyclique, alkyle linéaire, ramifié ou cyclique comprenant de 1 à 22 atomes de carbone lui-même étant éventuellement substitué par un ou plusieurs groupements hydroxyle,amino, alcoxy C1-C6 ; un radical alkyle linéaire, ramifié ou cyclique, comprenant de 1 à 22 atomes de carbone, éventuellement substitué par un ou plusieurs groupements choisis parmi les groupements hydroxyle, amino, alcoxy en C1-C6 linéaire, ramifié ou cyclique, aryle éventuellement substitué, carboxyle, sulfo, un atome d'halogène,ce radical alkyle pouvant être interrompu par un hétéroatome ;
• les dérivés 4,4'-DISTYRYLBIPHENYLES de formule : 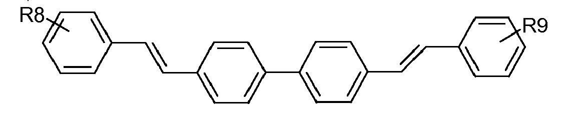 dans laquelle R8 et R9 indépendamment les uns des autres représentent un atome d'hydrogène ; un atome d'halogène ; un groupement aryle en C6-C30 ; un groupement hydroxy; un groupement cyano; un groupement nitro; un groupement sulfo ; un groupement amino ; un groupement acylamino ; un groupement dialkyl(C1-C6)amino ; un groupement dihydroxyalkyl(C1-C6)amino ; un groupement alkyl(C1-C6)hydroxyalkyl(C1-C6)amino ; un groupement alcoxy(C1-C6) ; un groupement alcoxy(C1-C6)carbonyle ; un groupement carboxyalcoxy en C1-C6 ; un groupement pipéridinosulfonyle ; un groupement pyrrolidino ; un groupement alkyle(C1-C6)halogénoalkyle(C1-C6)amino ; un groupement benzoylalkyle(C1-C6) ; un groupement vinyle; un groupement formyle ; un radical aryle en C6-C30 éventuellement substitué par un ou plusieurs groupements choisis parmi les groupements hydroxyle, alcoxy en C1-C6 linéaire, ramifié ou cyclique, alkyle linéaire, ramifié ou cyclique comprenant de 1 à 22 atomes de carbone lui-même étant éventuellement substitué par un ou plusieurs groupements hydroxyle,amino, alcoxy C1-C6 ; un radical alkyle linéaire, ramifié ou cyclique, comprenant de 1 à 22 atomes de carbone, éventuellement substitué par un ou plusieurs groupements choisis parmi les groupements hydroxyle, amino, alcoxy en C1-C6 linéaire, ramifié ou cyclique, , aryle éventuellement substitué, carboxyle, sulfo, un atome d'halogène,ce radical alkyle pouvant être interrompu par un hétéroatome ;
• les dérivés TRIAZINYLAMINOSTILBENES de formule : 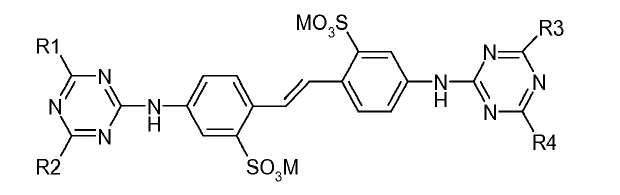 dans laquelle R1, R2, R3 et R4 indépendamment les uns des autres représentent un atome d'hydrogène ; un atome d'halogène ; un groupement aryle en C6-C30 ; un groupement hydroxy ; un groupement cyano ; un groupement nitro ; un groupement sulfo ; un groupement amino ; un groupement acylamino ; un groupement dialkyl(C1-C6)amino ; un groupement dihydroxyalkyl(C1-C6)amino ; un groupement alkyl(C1-C6)hydroxyalkyl(C1-C6)amino ; un groupement alcoxy(C1-C6) ; un groupement alcoxy(C1-C6)carbonyle ; un groupement carboxyalcoxy en C1-C6 ; un groupement pipéridinosulfonyle ; un groupement pyrrolidino ; un groupement alkyle(C1-C6)halogénoalkyle(C1-C6)amino ; un groupement benzoylalkyle(C1-C6) ; un groupement vinyle; un groupement formyle ; un radical aryle en C6-C30 éventuellement substitué par un ou plusieurs groupements choisis parmi les groupements hydroxyle, alcoxy en C1-C6 linéaire, ramifié ou cyclique, alkyle linéaire, ramifié ou cyclique comprenant de 1 à 22 atomes de carbone lui-même étant éventuellement substitué par un ou plusieurs groupements hydroxyle,amino, alcoxy C1-C6 ; un radical alkyle linéaire, ramifié ou cyclique, comprenant de 1 à 22 atomes de carbone, éventuellement substitué par un ou plusieurs groupements choisis parmi les groupements hydroxyle, amino, alcoxy en C1-C6 linéaire, ramifié ou cyclique, , aryle éventuellement substitué, carboxyle, sulfo, un atome d'halogène,ce radical alkyle pouvant être interrompu par un hétéroatome, tel que l'azote, le soufre et l'oxygène ;
et M est un cation monovalent ou divalent issu de la famille des alcalins ou des alcalino-terreux ;
• les dérivés Bis(BENZOXAZOLE) de formule : 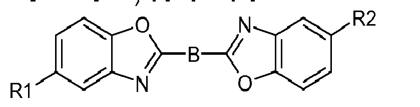 dans laquelle R1, R2 indépendamment les uns des autres représentent un atome d'hydrogène ; un atome d'halogène ; un groupement aryle en C6-C30 ; un groupement hydroxy ; un groupement cyano ; un groupement nitro ; un groupement sulfo ; un groupement amino ; un groupement acylamino ; un groupement dialkyl(C1-C6)amino ; un groupement dihydroxyalkyl(C1-C6)amino ; un groupement alkyl(C1-C6)hydroxyalkyl(C1-C6)amino ; un groupement alcoxy(C1-C6) ; un groupement alcoxy(C1-C6)carbonyle ; un groupement carboxyalcoxy en C1-C6; un groupement pipéridinosulfonyle ; un groupement pyrrolidino ; un groupement alkyle(C1-C6)halogénoalkyle(C1-C6)amino ; un groupement benzoylalkyle(C1-C6) ; un groupement vinyle; un groupement formyle ; un radical aryle en C6-C30 éventuellement substitué par un ou plusieurs groupements choisis parmi les groupements hydroxyle, alcoxy en C1-C6 linéaire, ramifié ou cyclique, alkyle linéaire, ramifié ou cyclique comprenant de 1 à 22 atomes de carbone lui-même étant éventuellement substitué par un ou plusieurs groupements hydroxyle,amino, alcoxy C1-C6 ; un radical alkyle linéaire, ramifié ou cyclique, comprenant de 1 à 22 atomes de carbone, éventuellement substitué par un ou plusieurs groupements choisis parmi les groupements hydroxyle, amino, alcoxy en C1-C6 linéaire, ramifié ou cyclique, , aryle éventuellement substitué, carboxyle, sulfo, un atome d'halogène,ce radical alkyle pouvant être interrompu par un hétéroatome ; et
B est choisi parmi 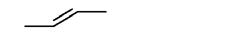 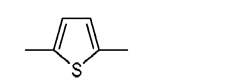 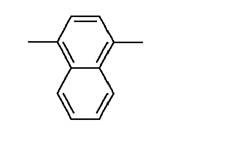 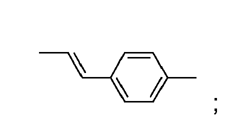
• les Bis(BENZIMIDAZOLES) cationiques ou non ; et
• les 1,3-Diphenyl-2-PYRAZOLINES anioniques ou non.

39. Composition selon l'une quelconque des revendications précédentes dans laquelle le ou les colorants fluorescents ou azurants optiques sont présents en quantité comprise entre 0,001 et 40 %, en poids du poids total de la composition.

40. Composition selon l'une quelconque des revendications précédentes dans laquelle le ou les solvants organiques sont choisis parmi les huiles organiques ; les silicones ; les huiles minérales ; les huiles végétales ; les alcanes en C₅-C₂₀ ; l'acétone ; la méthyléthylcétone ; les esters d'acides en C₁-C₂₀ liquides et d'alcools en C₁-C₈ ; le diméthoxyéthane ; le diéthoxyéthane ; les alcools gras liquides en C₁₀-C₃₀ ; les esters d'alcools gras ou d'acides gras ; l'huile de polybutène ; l'isononanoate d'isononyle ; le malate d'isostéaryle ; le tétra-isostéarate de pentaérythrityle ; le trimélate de tridécyle ; ou leurs mélanges.

41. Composition selon la revendication précédente dans laquelle le ou les solvants organiques sont choisis parmi les silicones.

42. Procédé de coloration de fibres kératiniques qui consiste à appliquer sur lesdites fibres un composé X, un composé Y et un colorant fluorescent, avec X, Y, colorant fluorescent sont tels que définis dans une quelconque des revendications précédentes.

43. Dispositif à plusieurs compartiments comprenant au moins deux compositions (A) et (B) conditionnées séparément, le kit comprenant i) au moins un composé X, ii) au moins un composé Y, étant entendu que la composition (A) et/ou (B) comprenant au moins un colorant fluorescent; et iii) éventuellement au moins un catalyseur ou un peroxyde ; le catalyseur lorsqu'il est présent ou le peroxyde ne sont pas présents simultanément dans la même composition ; lesdits composés X et Y étant susceptibles de réagir ensemble par une réaction d'hydrosilylation, ou par une réaction de condensation, ou par une réaction de réticulation en présence d'un peroxyde, lorsqu'ils sont mis en contact les uns avec les autres.

44. utilisation pour la coloration des fibres kératiniques de :
- au moins un composé X et au moins un composé Y tels que définis à l'une quelconque des revendications 1 à 34 ;
- au moins un colorant fluorescent tel que défini à l'une quelconque des revendications 1 et 35 à 36 et 39 ; et éventuellement
- au moins un solvant organique tel que défini à l'une quelconque des revendications 40 et 41.

45. utilisation pour l'éclaircissement des fibres kératiniques foncées de :
- au moins un composé X et au moins un composé Y tels que définis à l'une quelconque des revendications 1 à 34 ;
- au moins un colorant fluorescent ou un azurant optique tel que défini à l'une quelconque des revendications 1 et 36 et 39 ; et éventuellement
- au moins un solvant organique tel que défini à l'une quelconque des revendications 40 et 41.
